# EUROPEAN PATENT SPECIFICATION

(11) **EP 1 989 194 B1**
(45) Date of publication and mention of the grant of the patent: **25.08.2010**
(21) Application number: 07712220.8
(22) Date of filing: 14.02.2007
(51) Int. Cl.: C07D 335/02

(54) **CYCLIC SULFONES USEFUL AS BACE INHIBITORS**
CYCLISCHE SULFONE, DIE ZUR VERWENDUNG ALS BACE-INHIBITOREN GEEIGNET SIND
SULFONES CYCLIQUES UTILES COMME INHIBITEURS DE BAcE

(30) Priority: 14.02.2006 GB 0602951
(43) Date of publication of application: 12.11.2008
(73) Proprietor: Novartis AG, 4056 Basel (CH)
(72) Inventor: RUEEGER, Heinrich, CH-4112 Flüh (CH); MCCARTHY, Clive, CH-4056 Basel (CH); MOEBITZ, Henrik, 79100 Freiburg (DE); RONDEAU, Jean-Michel, F-68170 Rixheim (FR); TINTELNOT-BLOMLEY, Marina, 79686 Maulburg (DE)
(74) Representative: de Weerd, Petrus G.W.
(86) International application number: PCT/EP2007/051454
(87) International publication number: WO 2007/093621

(56) References cited:
- WO-A-02/057252
- WO-A-2005/047236
- JOHN VARGHESE; BECK JAMES P.; BIENKOWSKI MICHAEL J; SINHA SUKANTO; HEINRIKSON ROBERT L.: "Human ?-secretase (BACE) and BACE inhibitors" JOURNAL OF MEDICINAL CHEMISTRY, vol. 46, no. 22, 23 October 2003 (2003-10-23), pages 4625-4630, XP002435043

## Description

The present invention relates to novel heterocyclic compounds, to their preparation, to their use as medicaments and to medicaments comprising them.

More particularly the invention relates to a compound of the formula 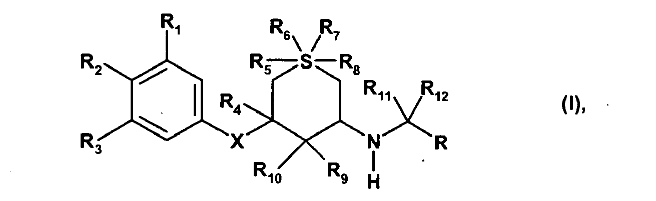 in which
R₁ is hydrogen; halogen; (C₁₋₈)alkyl; hydroxy-(C₁₋₈)alkyl; (C₁₋₈)alkoxy; (C₁₋₈)alkoxy-(C₁₋₈)alkyl; (C₁₋₈)alkoxy-(C₁₋₈)alkoxy-(C₁₋₈)alkyl; (C₆₋₁₀)aryl-(C₁₋₈)alkyl, the aryl moiety of which is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)-alkoxy, hydroxy-(C₁₋₈)alkyl and (C₁₋₈)alkoxy-(C₁₋₈)alkyl; a heteroaryl or heterocycloalkyl group, which group is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; aminocarbonyl, the amino moiety of which is unsubstituted or mono- or di-substituted by substituents selected from the group consisting of (C₁₋₈)alkyl and benzyl, the phenyl moiety of which is unsubstituted or mono-, di-or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; or (C₁₋₈)alkyl, which is mono-substituted by a pyrrolidinyl or oxazolidinyl group, which group is unsubstituted or mono-, di- or tri-substituted by substituents selected from the group consisting of oxo, (C₁₋₈)alkyl, halogen-(C₁₋₈)alkyl and (C₁₋₈)alkoxy-(C₁₋₈)alkyl;
R₂ is hydrogen; halogen; hydroxy; (C₁₋₈)alkyl; (C₁₋₈)alkoxy; (C₁₋₈)alkylamino; or an arylamino or heteroarylamino group, the aryl or heteroaryl moiety of which is unsubstituted or mono-, di- or tri-substituted by substituents selected from the group consisting of halogen, (C₁₋₈)alkyl, (C₁₋₈)alkoxy, (C₃₋₈)cycloalkyl, (C₃₋₈)cycloalkyl-(C₁₋₈)alkyl and a heteroaryl or aryl group, which heteroaryl or aryl group is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl;
R₃ is hydrogen; halogen; (C₁₋₈)alkyl; or benzyl, the phenyl moiety of which is unsubstituted or mono-, di- or tri-substituted by substituents selected from the group consisting of (C₁₋₈)alkyl;
R₄ is hydrogen; or halogen;
R₅ and R₆ together are oxo; or both are absent;
R₇ and R₈ together are oxo; or both are absent;
   either R₉ is hydroxy; and
R₁₀ is hydrogen; or hydroxy;
   or R₉ and R₁₀ together are oxo;
either R₁₁ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl; and
R₁₂ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
or R₁₁ and R₁₂ together are, together with the carbon atom, to which they are attached, (C₃₋₈)cycloalkyl, in which one -CH₂- group can be replaced with -O-;
X is O; oᵣ CH₂; and
either R is a group of the formula 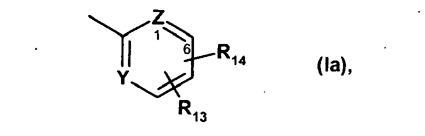 in which
R₁₃ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
R₁₄ is hydrogen; hydroxy; halogen; (C₁₋₈)alkyl; halogen-(C₁₋₈)alkyl; (C₁₋₈)alkoxy; halogen-(C₁₋₈)alkoxy; (C₁₋₈)alkoxy-(C₁₋₈)alkyl; (C₁₋₈)alkylcarbonyl; or (C₃₋₈)cycloalkyl, which is unsubstituted or mono-, di-, tri- or tetra-substituted by substituents selected from the group consisting of halogen and (C₁₋₈)alkyl;
Y is CH; or N; and
Z is CH; or N;
   or R is a group of the formula 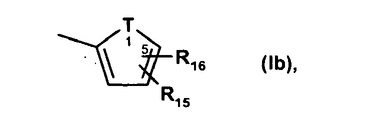 in which
   R₁₅ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
   R₁₆ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl; and
   T is O; or S,
      in free base form or in acid addition salt form.

On account of the asymmetrical carbon atoms present in the compounds of the formula I, the compounds may exist in pure optically active form or in the form of mixtures of optical isomers, e. g. in the form of racemic mixtures. All pure optical isomers and their mixtures, including the racemic mixtures, are part of the present invention.

Halogen denotes fluorine, bromine, chlorine or iodine.

Aryl is naphthyl or preferably phenyl.

Heteroaryl is an aromatic 5- or 6-membered ring, in which 1, 2 or 3 ring atoms are hetero atoms independently selected from O, N and S, such as thiazolyl, isothiazolyl, oxazolyl, isoxazolyl, pyridazinyl, pyrimidyl or pyridyl, and which ring can also be anellated with a phenyl ring, such as benzothiazolyl or benzoxazolyl. Heteroaryl is preferably isoxazolyl, pyridazinyl, pyrimidyl, pyridyl or benzothiazolyl.

Heterocycloalkyl is a non-aromatic 5- or 6-membered ring, in which 1, 2 or 3 ring atoms are hetero atoms independently selected from O, N and S, such as pyrrolidinyl, tetrahydrofuryl, tetrahydrothienyl or piperidyl.

Any non-cyclic carbon containing group or moiety with more than 1 carbon atom is straight-chain or branched.

Unless defined otherwise, carbon containing groups, moieties or molecules contain 1 to 8, preferably 1 to 6, more preferably 1 to 4, most preferably 1 or 2, carbon atoms.

In preferred embodiments, the invention relates to a compound of the formula I, in free base form or in acid addition salt form, in which
(1) R₁ is hydrogen; halogen; (C₁₋₈)alkyl; hydroxy-(C₁₋₈)alkyl; (C₁₋₈)alkoxy; (C₁₋₈alkoxy-(C₁₋₈)-alkyl; (C₁₋₈)alkoxy₋(C₁₋₈)alkoxy-(C₁₋₈)alkyl; (C₆₋₁₀)aryl-(C₁₋₈)alkyl, the aryl moiety of which is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)-alkoxy, hydroxy-(C₁₋₈)alkyl and (C₁₋₈)alkoxy-(C₁₋₈)alkyl; a heteroaryl or heterocycloalkyl group, which group is unsubstituted or mono-, di- or tri-substituted by substituents selected from the group consisting of hydroxy,halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; aminocarbonyl, the amino moiety of which is unsubstituted or mono- or di-substituted by substituents selected from the group consisting of (C₁₋₈)alkyl and benzyl, the phenyl moiety of which is unsubstituted or mono-, di-or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; or (C₁₋₈)alkyl, which is mono-substituted by a pyrrolidinyl or oxazolidinyl group, which group is unsubstituted or mono-, di- or tri-substituted by substituents selected from the group consisting of oxo, (C₁₋₈)alkyl, halogen-(C₁₋₈)alkyl and (C₁₋₈)alkoxy-(C₁₋₈)alkyl; preferably hydrogen; halogen; (C₁₋₈)alkyl; hydroxy-(C₁₋₈)alkyl; (C₁₋₈)alkoxy; (C₁₋₈)alkoxy-(C₁₋₈)-alkyl; (C₁₋₈)alkoxy-(C₁₋₈)alkoxy-(C₁₋₈)alkyl; benzyl, the phenyl moiety of which is unsubstituted or mono-, di- or tri-substituted by substituents selected from the group consisting of (C₁₋₈)-alkyl and (C₁₋₈)alkoxy-(C₁₋₈)alkyl; aminocarbonyl, the amino moiety of which is unsubstituted or mono- or di-substituted by substituents selected from the group consisting of (C₁₋₈)alkyl and benzyl, the phenyl moiety of which is unsubstituted or mono-, di- or tri-substituted by substituents selected from the group consisting of (C₁₋₈)alkyl, (C₁₋₈)alkoxy and (C₁₋₈)alkoxy-(C₁₋₈)alkyl; or (C₁₋₈)alkyl, which is mono-substituted by a pyrrolidinyl or oxazolidinyl group; which group is unsubstituted or mono-, di- or tri-substituted by substituents selected from the group consisting of oxo and (C₁₋₈)alkyl; preferably hydrogen;
(2) R₂ is hydrogen; halogen; hydroxy; (C₁₋₈)alkyl; (C₁₋₈)alkoxy; (C₁₋₈)alkylamino; or an arylamino or heteroarylamino group, the aryl or heteroaryl moiety of which is unsubstituted or mono-, di- or tri-substituted by substituents selected from the group consisting of halogen, (C₁₋₈)alkyl, (C₁₋₈)alkoxy, (C₃₋₈)cycloalkyl, (C₃₋₈)cydoalkyl-(C₁₋₈)alkyl and a heteroaryl or aryl group, which heteroaryl or aryl group is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; preferably hydrogen; halogen; hydroxy; (C₁₋₈)alkyl; (C₁₋₈)alkoxy; (C₁₋₈)alkylamino; or an arylamino or heteroarylamino group, the aryl or heteroaryl moiety of which is unsubstituted or mono-, di- or tri-substituted by substituents selected from the group consisting of halogen, (C₁₋₈)alkyl, (C₃₋₈)cycloalkyl, (C₃₋₈)cycloalkyl-(C₁₋₈)alkyl and a heteroaryl or aryl group, which heteroaryl or aryl group is unsubstituted or mono-, di- or tri-substituted by substituents selected from the group consisting of hydroxy, halogen, (C₁₋₈)alkyl, halogen-(C₁₋₈)alkyl, (C₁₋₈)-alkoxy, halogen-(C₁₋₈)alkoxy, hydroxy-(C₁₋₈)alkyl and (C₁₋₈)alkoxy-(C₁₋₈)alkyl; preferably hydrogen; hydroxy; (C₁₋₈)alkyl; arylamino, the aryl moiety of which is unsubstituted or preferably mono-substituted by a substituent selected from the group consisting of an aryl group, which aryl group is unsubstituted; or heteroarylamino, the heteroaryl moiety of which is un substituted or mono- or di-substituted by substituents selected from the group consisting of halogen, (C₁₋₈)alkyl, (C₁₋₈)alkoxy and an aryl group, which aryl group is unsubstituted or mono-, di- or tri-substituted by substituents selected from the group consisting of halogen; preferably hydrogen; hydroxy; (C₁₋₈)alkyl; or heteroarylamino, the heteroaryl moiety of which is unsubstituted or mono- or di-substituted by substituents selected from the group consisting of an aryl group, which aryl group is unsubstituted or mono-, di- or tri-substituted by substituents selected from the group consisting of halogen;
(3) R₃ is hydrogen; halogen; (C₁₋₈)alkyl; or benzyl, the phenyl moiety of which is unsubstituted or mono-, di- or tri-substituted by substituents selected from the group consisting of (C₁₋₈)alkyl;
   preferably hydrogen; halogen; or benzyl, the phenyl moiety of which is unsubstituted or
   mono- or di-substituted by substituents selected from the group consisting of (C₁₋₈)alkyl; preferably hydrogen;
(4) R₄ is hydrogen; or halogen;
   preferably hydrogen;
(5) R₅ and R₆ together are oxo; or both are absent;
   preferably R₅ and R₆ together are oxo;
(6) R₇ and R₈ together are oxo; or both are absent;
   preferably R₇ and R₈ together are oxo;
(7) either R₉ is hydroxy; and R₁₀ is hydrogen; or hydroxy; or R₉ and R₁₀ together are oxo; preferably R₉ is hydroxy; and R₁₀ is hydrogen;
(8) either R₁₁ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl; and R₁₂ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)-cycloalkyl, which is unsubstituted or mono-, di-, tri- or tetra-substituted by substituents selected from the group consisting of halogen and (C₁₋₈)alkyl; or R₁₁ and R₁₂ together are, together with the carbon atom, to which they are attached, (C₃₋₈)cycloalkyl, in which one - CH₂- group can be replaced with -O-;
   preferably either R₁₁ is hydrogen; and R₁₂ is hydrogen; or (C₁₋₈)alkyl; or R₁₁ and R₁₂ together are, together with the carbon atom, to which they are attached, (C₃₋₈)cycloalkyl; preferably R₁₁ is hydrogen; and R₁₂ is hydrogen; or (C₁₋₈)alkyl;
   preferably either R₁₁ is hydrogen; and R₁₂ is hydrogen; or R₁₁ and R₁₂ together are, together with the carbon atom, to which they are attached, (C₃₋₈)cycloalkyl;
(9) X is O; or CH₂;
   preferably CH₂;
(10) R₁₃ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
   preferably hydrogen; halogen; (C₁₋₈)alkyl; (C₁₋₈)alkoxy; or (C₃₋₈)cycloalkyl, which is unsubstituted or mono-, di-, tri- or tetra-substituted by substituents selected from the group consisting of halogen and (C₁₋₈)alkyl;
   preferably hydrogen;
(11) R₁₄ is hydrogen; hydroxy; halogen; (C₁₋₈)alkyl; halogen-(C₁₋₈)alkyl; (C₁₋₈)alkoxy; halogen-(C₁₋₈)alkoxy; (C₁₋₈)alkoxy-(C₁₋₈)alkyl; (C₁₋₈)alkylcarbonyl; or (C₃₋₈)cyc)oa!ky!, which is unsubstituted or mono-, di-, tri- or tetra-substituted by substituents selected from the group consisting of halogen and (C₁₋₈)alkyl;
   preferably hydrogen; hydroxy; halogen; (C₁₋₈)alkyl; halogen-(C₁₋₈)alkyl; (C₁₋₈)alkoxy; halogen-(C₁₋₈)alkoxy; (C₁₋₈)alkylcarbonyl; or (C₃₋₈)cycloalkyl, which is unsubstituted or mono-, di-, tri-
   or tetra-substituted by substituents selected from the group consisting of halogen and (C₁₋₈)-alkyl;
   preferably (C₁₋₈)alkyl;
(12) Y is CH; or N;
   preferably CH;
(13) Z is CH; or N;
   preferably CH;
(14) R₁₅ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
   preferably hydrogen;
(15) R₁₆ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
   preferably halogen; or (C₁₋₈)alkyl;
(16) T is O; or S.

The preferred embodiments (1) to (16) are preferred independently, collectively or in any combination or sub-combination.

In especially preferred embodiments, the invention relates to one or more than one of the compounds of the formula I mentioned in the Examples hereinafter, in free base form or in acid addition salt form.

In a further aspect, the invention relates to a process for the preparation of the compounds of the formula I and their salts, comprising the steps of
a) for the preparation of a compound of the formula I, in which R₁₁ is hydrogen, reaction of a compound of the formula 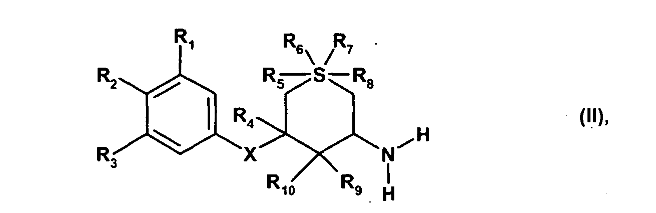 in which R₁, R₂, R₃, R₄, R₅, R₆, R₇, R₈, R₉, R₁₀ and X are as defined for the formula I, with a compound of the formula (O=)C(R₁₂)R (III), in which R₁₂ and R are as defined for the formula I, and subsequent subjection of the reaction mixture to a hydrogenating agent, such as NaBH₃CN, or
b) for the preparation of a compound of the formula I, in which R₉ is hydroxy and R₁₀ is hydrogen, reaction of a compound of the formula 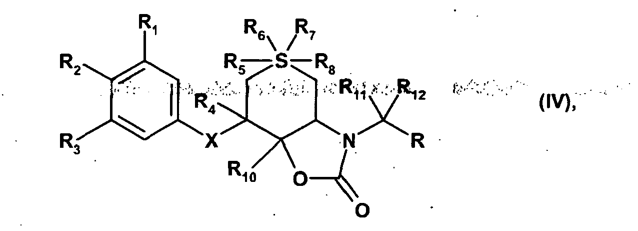 in which R₁, R₂, R₃, R₄, R₅, R₆, R₇, R₈, R₁₀, R₁₁, R₁₂, R and X are as defined for the formula I, with barium hydroxide or
c) for the preparation of a compound of the formula I, in which R₂ is a substituted amino group, conversion of a compound of the formula I, in which R₂ is an unsubstituted amino group, into a compound of the formula I, in which R₂ is a substituted amino group, for example by reaction with a corresponding halide,
   in each case optionally followed by reduction, oxidation or functionalisation of the resulting compound and/or by cleavage of protecting groups optionally present, and of recovering the so obtainable compound of the formula I in free base form or in acid addition salt form.

The reactions can be effected according to conventional methods, for example as described in the Examples.

The working-up of the reaction mixtures and the purification of the compounds thus obtainable may be carried out in accordance with known procedures.

Acid addition salts may be produced from the free bases in known manner, and vice-versa.

Compounds of the formula I can also be prepared by further conventional processes, which processes are further aspects of the invention, e. g. as described in the Examples, e. g. in Example 59.

The compounds of the formula I, in which R₂ is an unsubstituted amino group, and the starting materials of the formulae II, III and IV are known or may be prepared according to conventional procedures starting from known compounds, for example as described in the Examples.

Compounds of the formula I and their pharmaceutically acceptable acid addition salts, hereinafter referred to as "agents of the invention", exhibit valuable pharmacological properties when tested in vitro and in animals, and are therefore useful as medicaments.

The agents of the invention are inhibitors of aspartic proteases and can be used for the treatment of disorders involving processing by such enzymes. Particularly they inhibit beta-secretase and as such inhibit the generation of beta-amyloid and the subsequent aggregation into oligomers and fibrils.

### Test 1: Inhibition of human BACE

Recombinant BACE (extracellular domain, expressed in baculovirus and purified using standard methods) at 0.1-10 nM concentration is incubated with the test compound at various concentrations for 1 hour at room temperature in 10-100 mM acetate buffer, pH 4.5, containing 0.1 % CHAPS. Synthetic fluorescence-quenched peptide substrate, derived from the sequence of APP and containing a suitable fluorophore-quencher pair is added to a final concentration of 1-5 µM and the increase in fluorescence is recorded at a suitable excitation / emission wavelength in a microplate spectro-fluorimeter for 5-30 minutes in 1-minute intervals. IC₅₀ values are calculated from percentage of inhibition of BACE-activity as a function of the test compound concentration.

### Test 2: Inhibition of human BACE-2

Recombinant BACE-2 (extracellular domain, expressed in baculovirus and purified using standard methods) at 0.1-10 nM concentrations is incubated with the test compound at various concentrations for 1 hour at room temperature in 10-100 mM acetate buffer, pH 4.5, containing 0.1 % CHAPS. Synthetic peptide substrate derived from the sequence of APP and containing a suitable fluorophore-quencher pair is added to a final concentration of 1-5 µM and the increase in fluorescence is recorded at a suitable excitation / emission wavelength in a microplate spectro-fluorimeter for 5-30 minutes in 1-minute intervals. IC₅₀ values are calculated from percentage of inhibition of BACE-2-activity as a function of the test compound concentration.

### Test 3: Inhibition of human Cathepsin D

Recombinant cathepsin D (expressed as procathepsin D in baculovirus, purified using standard methods and activated by incubation in sodium formate buffer pH 3.7) is incubated with the test compound at various concentrations for 1 hour at room temperature in sodium formate or sodium acetate buffer at a suitable pH within the range of pH 3.0-5.0 Synthetic peptide substrate Mca-Gly-Lys-Pro-lle-Leu-Phe-Phe-Arg-Leu-Lys(DNP)-D-Arg-NH₂ is added to a final concentration of 1-5 µM and the increase in fluorescence is recorded at excitation of 325 nm and emission at 400 nm in a microplate spectro-fluorimeterfor 5-30 minutes in 1-minute intervals. IC₅₀ values are calculated from percentage of inhibition of cathepsin D-activity as a function of the test compound concentration.

### Test 4: Inhibition of cellular release of amyloid peptide 1-40

Chinese hamster ovary cells are transfected with the gene for amyloid precursor protein. Cells are plated at a density of 8000 cells/well in a 96- well microtiter plate and cultivated for 24 hours in DMEM cell culture medium containing 10 % FCS. The test compound is added to the cells at various concentrations, and cells are cultivated for 24 hours in the presence of the test compound. The supernatants are collected, and the concentration of amyloid peptide 1-40 is determined using sandwich ELISA. The potency of the compound is calculated from the percentage of inhibition of amyloid peptide release as a function of the test compound concentration.

In at least one of the above-indicated tests, the agents of the invention show activity at concentrations below 20 µM.

Specifically, the agent of the invention described in Example 26 shows an IC₅₀ value of 4.7 µM in Test 4.

The agents of the invention are therefore useful e. g. for the treatment and/or prevention of neurological and vascular disorders related to beta-amyloid generation and/or aggregation, such as neurodegenerative diseases like Alzheimer's disease, Down's Syndrome, memory and cognitive impairment, dementia, amyloid neuropathies, brain inflammation, nerve and brain trauma, vascular amyloidosis, or cerebral haemorrhage with amyloidosis.

Some of the agents of the invention also inhibit BACE2 (beta-site APP-cleaving enzyme 2) or Cathepsin D, close homologues of the pepsin-type aspartyl proteases and of beta-secretase. Due to the correlation of BACE2 and CathD expression with a more tumorigenic and metastatic potential of tumor cells, such inhibitors are useful for the suppression of the metastasis process associated with tumor cells.

For the above-mentioned indications, the appropriate dosage will of course vary depending upon, for example, the compound employed, the host, the mode of administration and the nature and seventy of the condition being treated. However, in general satisfactory results in animals are indicated to be obtained at a daily dosage of from about 0.1 to about 100, preferably from about 1 to about 50, mg/kg of animal body weight. In larger mammals, for example humans, an indicated daily dosage is in the range from about 10 to about 2000, preferably from about 10 to about 200, mg of an agent of the invention conveniently administered, for example, in divided doses up to four times a day or in sustained release form.

The agent of the invention may be administered by any conventional route, in particular enterally, preferably orally, for example in the form of tablets or capsules, or parenterally, for example in the form of injectable solutions or suspensions.

In accordance with the foregoing, the present invention also provides an agent of the invention, for use as a medicament, e. g. for the treatment of neurological or vascular disorders related to beta-amyloid generation and/or aggregation.

The present invention furthermore provides a pharmaceutical composition comprising an agent of the invention in association with at least one pharmaceutical carrier or diluent. Such compositions may be manufactured in conventional manner. Unit dosage forms contain, for example, from about 1 to about 1000, preferably from about 1 to about 500, mg of an agent of the invention.

The agents of the invention can be administered alone or in combination with other pharmaceutical agents effective in the treatment of conditions mentioned above.

The pharmaceutical combination may be in the form of a unit dosage form, whereby each unit dosage will comprise a predetermined amount of the two components, in admixture with suitable pharmaceutical carriers or diluents. Alternatively, the combination may be in form of a package containing the two components separately, e. g. a pack or dispenser-device adapted for the concomitant or separate administration of the two active agents, wherein these agents are separately arranged.

Moreover the present invention provides the use of an agent of the invention, for the manufacture of a medicament for the treatment of any neurological or vascular disorders related to beta-amyloid generation and/or aggregation.

In still a further aspect, the present invention provides a method for the treatment of any neurological or vascular disorders related to beta-amyloid generation and/or aggregation, in a subject in need of such treatment, which comprises administering to such subject a therapeutically effective amount of an agent of the invention.

The following Examples illustrate the invention, but do not limit it.

### Examples

### Abbreviations

- ACN: acetonitrile
- AcOH: acetic acid
- Boc: tert-butoxycarbonyl
- (Boc)₂O: di-tert-butyl dicarbonate
- DBU: 1,8-diazabicyclo[5.4.0]undec-7-ene
- Dess-Mar-:
- tin reagent: 1,1,1-tris(acetoxy)-1,1-dihydro-1,2-benziodoxol-3(1H)-one
- DIBAL: diisobutylaluminiumhydride
- DIPEA: diisopropylethylamine
- DMAP: 4-dimethylaminopyridine
- DMF: dimethylformamide
- DMSO: dimethylsulfoxide
- DPPA: diphenyl-phosphoryl azide
- EDC: 1-(3-dimethylaminopropyl)-3-ethylcarbodiimide hydrochloride
- ESIMS: electrospray ionization mass spectrometry
- EtOAc: ethyl acetate
- EtOH: ethanol
- Et₂O: diethyl ether
- h: hour(s)
- HOAt: 1-hydroxy-7-azabenzotriazole
- HPLC: high pressure liquid chromatography
- iPrOH: iso-propanol
- Lawesson re- agent: 2,4-bis(4-methoxyphenyl)-2,4-dithioxo-1,3,2,4-dithiadiphosphetane
- MeOH: methanol
- min: minute(s)
- NaHMDS: sodium hexamethyldisilazane
- NaOAc: sodium acetate
- NaOtBu: sodium tert-butoxide
- NEt₃: triethylamine
- NMR: nuclear magnetic resonance spectrometry
- Oxone: potassium monopersulfate
- Pd₂(dba)₃: tris(dibenzylideneacetone)dipalladium
- Pd(PPh₃)₄: tetrakis(triphenylphosphine)palladium
- p-TsOH: para-toluenesulfonic acid
- rt: room temperature
- Rₜ: retention time
- tBuOMe: tert-butyl methyl ether
- TEMPO: 2,2,6,6-tetramethylpiperidine 1-oxyl
- TFA: trifluoroacetic acid
- THF: tetrahydrofuran
- TLC: thin-layer chromatography
- Xantphos: 4,5-bis(diphenylphosphino)-9,9-dimethylxanthene

### General HPLC information

| | |
|---|---|
| Column dimensions: | 50 x 5 mm |
| Column type: | Nucleosil^{®} 5C₁₈, 3 micron |
| Eluent: | A) Water + 0.1 vol.-% TFA |
| | B) CAN + 0.1 vol.-% TFA |
| Gradient: | 10 - 100 % B in 3 min + 1 min 100 % B, flow = 4 ml/min |

### Example 1: (3S,4S,5R)-3-(4-Hydroxy-3-propyl-benzyl)-5-(3-isopropyl-benzylamino)-1,1-dioxo-hexahydro-1lambda*6*-thiopyran-4-ol hydrochloride

### a) 5-[1-(3-Bromo-4-methoxy-phenyl)-meth-(Z)-ylidene]-4-hydroxy-5,6-dihydro-2H-thiopyran-3-carboxylic acid methyl ester

To a solution of 3-bromo-4-methoxybenzaldehyde (81.5 g, 360 mmol), 4-oxo-tetrahydrothiopyran-3-carboxylic acid methyl ester (95.6 g, 540 mmol) and AcOH (41.0 g, 684 mmol) in EtOH (230 mL) and water (25 mL) is added pyrrolidine (45.3 mL, 540 mmol) and the resulting reaction mixture is heated at reflux for 3 days. The reaction mixture is colled to 10 °C and the precipitate is filtered. Recrystallization from THF-MeOH and once more from ACN gives the pure product as yellow crystals: m.p. 148-150 °C; TLC (hexane-tert-butylmethyl ether 3:1) Rf=0.33; ESIMS [M+H]⁺=371, 373;
¹H-NMR (400 MHz, CDCl₃): δ 12.72 (s, 1 H), 7.56 (d, 1H, J=1 Hz), 7.48 (s, 1 H), 7.28 (dd, J=7, 1 Hz, 1 H), 6.95 (d, J=7 Hz, 1 H), 3.96 (s, 3H), 3.87 (s, 3H), 3.68 (s, 2H), 3.54 (s, 2H).

### b) 5-[1-(3-Bromo-4-methoxy-phenyl)-meth-(Z)-ylidene]-4-hydroxy-1,1-dioxo-1,2,5,6-tetrahydro-1lambda*6*-thiopyran-3-carboxylic acid methyl ester

To a solution of 5-[1-(3-bromo-4-methoxy-phenyl)-meth-(Z)-ylidene]-4-hydroxy-5,6-dihydro-2H-thiopyran-3-carboxylic acid methyl ester (48.3 g, 130 mmol) in THF (900 mL) is added water (150 mL) at 40-50 °C and oxone (173 g, 273 mmol) in portions over a period of 30 min. The reaction mixture is stirred at 40-50 °C for 1 h. Excess oxone is destroyed with Na₂S₂O₅ and the reaction mixture is extracted with EtOAc.

Combined organic layers are washed with brine and dried over MgSO₄. Evaporation of the solvents and recrystallization from ACN gives the product as light yellow crystals: m.p. 190-192 °C; TLC (toluene/EtOAc 3:1) Rf=_{0.36}; ESIMS [M+H+NH₃] ⁺= 420, 422; ¹H-NMR (400 MHz, CDCl₃): δ 13.08 (s, 1 H), 7.82 (s, 1 H), 7.54 (d, J=1Hz,1H), 7.28 (dd, J=7, 1Hz, 1 H), 6.98 (d, J=7 Hz, 1 H), 4.20 (s, 2H), 4.07(s, 2H), 3.96 (s, 3H), 3.87 (s, 3H).

### c) 5-(3-Bromo-4-methoxy-benzyl)-4-hydroxy-1,1-dioxo-1,2,5,6-tetrahydro-1lambda*6*-thiopyran-3-carboxylic acid methyl ester

5-[1-(3-bromo-4-methoxy-phenyl)-meth-(Z)-ylidene]-4-hydroxy-1,1-dioxo-1,2,5,6-tetrahydro-1 lambda*6*-thiopyran-3-carboxylic acid methyl ester (96.8 g, 240 mmol) is hydrogenated (4 atm H₂) at 25°C in THF (2500 mL) with PtO₂ as catalyst (5 g) for 16 h. The catalyst is filtered off and the filtrate is evaporated. Recrystallization from THF-Et₂O 1:1 gave the product as colorless crystals: m.p. 170-172 °C; TLC (toluene/EtOAc 3:1) Rf=0.38; ESIMS [M+H+NH₃] ⁺=422, 424; ¹H-NMR (400 MHz, CDCl₃): δ 13.14 (s, 1H), 7.44 (d, J=1Hz, 1H), 7.16 (dd, J=7, 1 Hz, 1 H), 6.88 (d, J=7 Hz, 1 H), 3.94 (s, 3H), 3.84 (m, 5H), 3.2-3.4 (m, 2H), 2.9-3.1 (m, 3H).

### d) 5-(3-Bromo-4-methoxy-benzyl)-4-hydroxy-1,1-dioxo-hexahydro-1lambda*6*-thiopyran-3-carboxylic acid methyl ester

To a suspension of 5-(3-bromo-4-methoxy-benzyl)-4-hydroxy-1,1-dioxo-1,2,5,6-tetrahydro-1lambda*6*-thiopyran-3-carboxylic acid methyl ester (41.7 g, 103 mmol) in iPrOH-AcOH 4:1 (1500 mL) is added at 45-50°C NaBH₃CN(10.8g 188 mmol) in 4 portions over a period of 0.5 h. The reaction mixture is stirred for 1 h at 45-50 °C. The solvents are evaporated and the residue is redissolved in EtOAc and basified with saturated NaHCO₃-solution. After extraction with EtOAc, the combined organic layers are washed with brine and dried over MgSO₄. Evaporation of the solvents gives the product as a white solid as a mixture of diastereoisomers which is used in the next step without further purification: TLC (toluene/EtOAc 1:1) Rf=0.35, 0.28 and 0.23; ESIMS [M+H+NH₃]⁺=424, 426.

### e) 5-(3-Bromo-4-methoxy-benzyl)-4-hydroxy-1,1-dioxo-hexahydro-1lambda*6*-thiopyran-3-carboxylic acid

To a solution 5-(3-bromo-4-methoxy-benzyl)-4-hydroxy-1,1-dioxo-hexahydro-1lambda*6*-thiopyran-3-carboxylic acid methyl ester (91.6 g, 225 mmol) in dioxane (500 mL) is added 4N NaOH (450 mL) and the reaction mixture is stirred for 6 h at 25°C. The reaction mixture is acidified with 4N HCl at 15-20 °C and extracted with EtOAc. Combined organic layers are washed with brine, dried over MgSO₄, filtered and evaporated. After addition of Et₂O the product, a mixture of diastereoisomers, is filtered off and the white crystals are dried: TLC (EtOAc-AcOH 200:1) Rf=0.20; ESIMS [M+H+NH₃]⁺=410, 412.

### f) 7-(3-Bromo-4-methoxy-benzyl)-5,5-dioxo-hexahydro-1Iambda*6* -oxa-5lam bda*6*thia-3-aza-inden-2-one

To a solution of 5-(3-bromo-4-methoxy-benzyl)-4-hydroxy-1,1-dioxo-hexahydro-1lambda*6*-thiopyran-3-carboxylic acid (80.6 g, 205 mmol) in dioxane (1200 mL) is added NEt₃ (43.3 mL, 307.5 mmol) and DPPA (55.9 mL, 246 mmol) at 10-20 °C within 20 min. The reaction mixture is stirred at 25°C for 1.5 h; then heated at 60°C for 2 h and finally at reflux for 8 h. The reaction mixture is evaporated, the residue is redissolved in CH2Cl2, washed with 1N H₂SO₄-solution, saturated NaHCO₃-solution and brine and dried over MgSO₄. Evaporation of the solvents gives the product as a white solid. The crude product is suspended in EtOAc (100 mL), stirred for 0.5h, filtered and dried. The mixture of diastereoisomers is used in the next step without further purification: TLC (EtOAc) Rf=0.63 and 0.42; ESIMS [M+H+NH₃] ⁺=407, 409.

### g) 7-(3-Bromo-4-methoxy-benzyl)-2,5,5-trioxo-hexahydro-1Iambda*6*-oxa-5lambda*6*-thia-3-aza-indene-3-carboxylic acid tert-butyl ester

To a solution of 7-(3-bromo-4-methoxy-benzyl)-5,5-dioxo-hexahydro-1lambda*6*-oxa-5lambda*6*thia-3-aza-inden-2-one(77.3g 198 mmol) in ACN (1200 mL) is added NEt₃ (33.5 mL, 238 mmol), (BOC)₂O (50.1 g, 218 mmol) and a catalytic amount of DMAP (0.734 g, 6 mmol). The reaction mixture is heated at 45-50 °C for 2 h. The reaction mixture is evaporated to 1/5 of its original volume and the product is crystallized from EtOAc- Et2O. The white crystals are filtered off and the mixture of diastereoisomers is used as such in the next step: TLC (hexane-EtOAc 1:1) Rf=0.39 and 0.37; ESIMS [M+H+NH₃]⁺=507, 509.

### h) [5-(3-Bromo-4-methoxy-benzyl)-4-hydroxy-1,1-dioxo-hexahydro-1lambda*6*-thiopyran-3-yl]-carbamic acid tert-butyl ester

To a suspension of 7-(3-bromo-4-methoxy-benzyl)-2,5,5-trioxo-hexahydro-1lambda*6*-oxa-5lambda*6*-thia-3-aza-indene-3-carboxylic acid tert-butyl ester (79.9 g, 163 mmol) in MeOH (1300 mL) is added CS₂CO₃ (8.1 g, 25 mmol) and the reaction mixture is warmed to 40-50 °C. After 30 min the clear solution was evaporated to 1/4 of its volume and the crystallized product is filtered off. The filtrate is evaporated and the residue is re- dissolved in EtOAc, washed with water and brine, dried over MgSO₄ and evaporated. Crystallization from EtOAc-Et₂O gave additional amount of product as light yellow crystals. Both batches of crystals are diastereoisomeric mixtures and are used as such in the next step: TLC (hexane-EtOAc 1:1) Rf=0.21 and 0.18; ESIMS [M+H+NH₃]⁺=481, 483.

### i) [(3R*,5S*)-5-(3-Bromo-4-methoxy-benzyl)-1,1,4-trioxo-hexahydro-1lambda*6*-thiopyran-3-yl]-carbamic acid tert-butyl ester

To a solution of [5-(3-bromo-4-methoxy-benzyl)-4-hydroxy-1,1-dioxo-hexahydro-1lambda*6*-thiopyran-3-yl]-carbamic acid tert-butyl ester (39.5 g, 85 mmol) in dimethylsulfoxide (270 mL) is added NEt₃ (90.5 mL, 640 mmol) and the pyridine-SO₃ complex (35.7 g, 213.2 mmol) in portions at 12-14 °C. The reaction mixture is stirred at 25°C for 3 h, then poured onto ice-water and extracted with CH₂Cl₂. The combined organic layers are washed with cold 0.5 N H₂SO₄, saturated NaHCO₃-solution and water, dried over MgSO₄ and evaporated. Recrystallization from THF-Et₂O gave white crystals and the product is obtained as a single diastereoisomer: m.p. 197-199 °C; TLC (hexane-EtOAc 1:1) Rf=0.38; ESIMS [M+H+NH₃] ⁺=479, 481; ¹H-NMR (400 MHz, CDCl₃): δ 7.39 (d, J=1Hz, 1H), 7.09 (dd, J=7, 1 Hz, 1H), 6.87 (d, J=7 Hz, 1 H), 5.57 (bs, 1 H), 4.94 (m, 1 H), 3.98 (m, 1 H), 3.93 (s, 3H), 3.46 (m, 1 H), 3.3 (m, 3H), 3.16 (t, J=13 Hz, 1H), 2.62 (dd, J=7, 13 Hz, 1H), 1.45 (s, 9H).

### j) [(3R*,4S*,5S*)-5-(3-Bromo-4-methoxy-benzyl)-4-hydroxy-1,1-dioxo-hexahydro-1lambda*6*-thiopyran-3-yl]-carbamic acid tert-butyl ester

To a solution of [(3R*5S*)-5-(3-bromo-4-methoxy-benzyl)-1,1,4-troxo-hexahydro-1lambda*6*-thiopyran-3-yl]-carbamic acid tert-butyl ester (34.2 g, 74 mmol) in anhydrous THF (1600 mL) is added under Argon at -30 °C the LiAlH₄ (1.71 g, 44.5 mmol) in several portions. After stirring the reaction mixture for 0.5 h at -30 °C and 0.5 h at 0 °C, the reduction was carefully quenched at 0 °C with water (1.7 mL), 4N NaOH (2 mL) and with water (5.1 mL). After stirring for 1 h, the inorganic salts are filtered off. The filtrate is diluted with toluene-ACN 3:1 (500 mL) and evaporated. The product is recrystallized from hot THF-ACN-hexane. The product crystallized in white crystals, containing only the desired racemic (3R*,4S*,5S*)-diastereoisomer: m.p. 238-240 °C; TLC (hexane-EtOAc 1:1) Rf=0.18; ESIMS [M+H+NH3]⁺=481, 483; ¹H-NMR (400 MHz, CDCl₃): δ 7.39 (d, J=1Hz, 1 H), 7.09 (dd, J=7, 1 Hz, 1 H), 6.88 (d, J=7 Hz, 1 H), 5.1 (bs, 1 H), 4.06 (m, 1 H), 3.95 (s, 3H), 2.9-3.5 (m, 6H), 2.6-2.8 (m, 2H), 2.43 (m, 1 H), 1.43 (s, 9H), whereas the mother liquor contained some of the undesired (3R*,4R*,5S*)-diastereoisomer: TLC (hexane-EtOAc 1:1) Rf=0.23; ESIMS [M+H+NH₃] ⁺=481, 483.

### k) (3aR,7S,7aS)-7-(3-Bromo-4-methoxy-benzyl)-2,2-dimethyl-5,5-dioxo-hexahydro-1-oxa-5lambda*6*-thia-3-aza-indene-3-carboxylic acid tert-butyl ester and (3aS,7R,7aR)-7-(3-Bromo-4-methoxy-benzyl)-2,2-dimethyl-5,5-dioxo- hexahydro-l-oxa-5lambda*6*-thia -3-aza-indene-3-carboxylic acid tert-butyl ester

To a solution of [(3R*,4S*,5S*)-5-(3-bromo-4-methoxy-benzyl)-4-hydroxy-1,1- dioxo-hexahydro-1lambda*6*-thiopyran-3-yl]-carbamic acid tert-butyl ester (25.1 g, 54 mmol) in CH₂Cl₂ (500 mL) is added 2,2-dimethoxy-propane (67.7 mL, 540 mmol) and p-TsOH (0.31 g, 1.6 mmol) and the reaction mixture is heated at reflux for 16 h. The cold reaction mixture is washed with NaHCO₃-solution and water, dried over MgSO₄ and evaporated. The residual oil is crystallized from tBuOMe to give the racemic product as white crystals: m.p. 165-170 °C; TLC (hexane-EtOAc 1:1).Rf=0.48; ESIMS [M+H+NH3]⁺=521, 523. The racemate is separated by simulating moving bed (SMB) chromatography on a UOP-SORBEX PREP instrument with 16 columns (75x21 mm, Princton Chromatography Corporation) containing a stationary phase FA-2364/21 20 µM (equivalent to CHIRALPAK AD immobilized) with hexane-tBuOMe- THF 50:40:10 on to give enantiomerically pure (3aR,7S,7aS)-diastereoisomer as white crystals: m.p. 171-174 °C; [α]_{D} 8.3° (c 0.36, CHCl₃); ESIMS [M+H+NH₃]+=521, 523; ¹H-NMR (400 MHz, CDCl₃): δ 7.43 (d, J=1Hz, 1 H), 7.06 (dd, J=7, 1 Hz, 1 H), 6.86 (d, J=7 Hz, 1 H), 4.3 (m, 1 H), 3.93 (s, 3H), 3.7-3.9 (m, 1 H), 3.34 (t, J=13 Hz, 1H), 2.6-3.1 (m, 6H), 1.62 (bs, 6H), 1.47 (s, 9H). The undesired (3aS,7R,7aR)-diastereoisomer is obtained as white crystals: m.p. 173-176 °C; [α]_{D} -8.8° (c 0.44, CHCl₃); ESIMS [M+H+NH₃]⁺=521, 523

### l) (3aR,7S,7aS)-7-(4-Methoxy-3-vinyl-benzyl)-2,2-dimethyl-5,5-dioxo-hexahydro-1-oxa-5lambda*6*-thia-3-aza-indene-3-carboxylic acid tert-butyl ester

To a degassed solution of (3aR,7S,7aS)-7-(3-bromo-4-methoxy-benzyl)-2,2-dimethyl-5,5-dioxo-hexahydro-1-oxa-5lambda*6*-thia-3-aza-indene-3-carboxylic acid tert-butyl ester (6.46 g, 12.8 mmol), 2,4,6-trivinylcyclotriboroxane-pyridine complex (6.5 g, 25.6 mmol) and tri-tert-butylphosphine (0.529 g, 2.56 mmol) in dioxane (100 mL) is added under Argon Pd2(dba)3 (0.59 g, 0.64 mmol) and Cs₂CO₃ (8.53 g, 25.6 mmol). The resulting reaction mixture is distributed in 5 vials and heated for 30 min at 160 °C in a microwave oven. The combined reaction mixtures are diluted with EtOAc, filtered over Celite, and the filtrate is washed with NaHCO₃-solution, water and brine. Evaporation of the solvents and purification by flash-chromatography on silica gel (hexane-EtOAc 4:1 to 2: 1) gives the product as beige solid: TLC (hexane-EtOAc 2:1) Rf=0.33; ESIMS [M+H+NH₃] ⁺=469; ¹H-NMR (400 MHz, CDCl₃): δ 7.25 (d, J=1Hz, 1 H), 7.02 (m, 2H), 6.84 (d, J=7 Hz, 1 H), 5.75 (d, J=14 Hz, 1H), 5.32 (d, J=14 Hz, 1 H), 3.94 (s, 3H), 3.7-3.9 (m, 2H), 3.38 (t, J=12 Hz, 1 H), 2.5-3.1 (m, 6H), 1.64 (bs, 6H), 1.48 (s, 9H).

### m) (3aR,7S,7aS)-7-(3-Formyl-4-methoxy-benzyl)-2,2-dimethyl-5,5-dioxo-hexahydro-1-oxa-5lambda*6*-thia-3-aza-indene-3-carboxylic acid tert-butyl ester

A solution of (3aR,7S,7aS)-7-(4-methoxy-3-vinyl-benzyl)-2,2-dimethyl-5,5-dioxo-hexahydro-1-oxa-5lambda*6*-thia-3-aza-indene-3-carboxylic acid tert-butyl ester (2.53 g, 5.6 mmol) in CH₂Cl₂-MeOH 4:1 (150 mL) containing some NaHCO₃ (0.024 g, 0.28 mmol) is treated with ozone at -78 °C until the educt has disappeared. After addition of dimethylsulfide (0.85 mL, 11.3 mmol), the reaction mixture is allowed to warm to room temperature and stirred for 5 h. After evaporation of the solvents the residue is crystallized from EtOAc-hexane to give the product as white crystals: TLC (hexane/EtOAc 2:1) Rf=0.16; ESIMS [M+H+NH₃] ⁺=471; ¹H-NMR (400 MHz, CD₃OD): δ 10.41 (s, 1 H), 7.68 (d, J=1Hz, 1 H), 7.53 (dd, J=7, 1 Hz, 1 H), 7.18 (d, J=7 Hz, 1 H), 3.96 (s, 3H), 3.5-4.1 (m, 3H), 3.0-3.4 (m, 3H), 2.95 (t, J=13 Hz, 1 H), 2.8 (m, 1H), 2.5 (m, 1H), 1.64 (bs, 6H), 1.49 (s, 9H).

### n) (3aR,75,7aS)-7-[4-Methoxy-3-((Z)-propenyl)-benzyl]-2,2-dimethyl-5,5-dioxo-hexahydro-1-oxa-5lambda*6*-thia-3-aza-indene-3-carboxylic acid tert-butyl ester

To a suspension of ethyl-triphenylphosphoniumbromide (0.52 g, 1.36 mmol) in anhydrous THF (25 mL) is added under Argon a 1M NaHMDS-solution in THF(1,5 mL 1,5 mmol) and the reaction mixture is stirred for 45 min at 30-35 °C. To the resulting orange solution is added a solution of (3aR,7S,7aS)-7-(3-formyl-4-methoxy-benzyl)-2,2-dimethyl-5,5-dioxo-hexahydro-1-oxa-5lambda*6*-thia-3-aza-indene-3₋carboxylic acid tert-butyl ester (0.35 g, 0.75 mmol) in THF (5 mL) at 0-5 °C. The reaction mixture is stirred at 25 °C for 3 h. After addition of NH₄Cl-solution (10 mL) the product is extracted with EtOAc. The combined organic layers are washed with brine, dried over MgSO₄ and evaporated. The pure product is obtained after purification by flash-chromatography on silica gel (hexane-EtOAc 5:2 to 2:1) as a white foam: TLC (hexane-EtOAc 2:1) Rf=0.40; ESIMS [M+H+NH₃] ⁺=483; ¹H-NMR (400 MHz, CDCl₃): δ 7.05 (d, J=1Hz, 1H), 7.03 (d, J=7Hz, 1 H), 6.84 (d, J=7 Hz, 1H), 6.53 (d, J=10 Hz, 1 H), 5.86 (m, 1 H), 3.93 (s, 3H), 3.7-3.9 (m, 2H), 3.39 (t, J=12 Hz, 1 H), 3.0 (m, 3H), 2.71 (m, 2H), 2.58 (m, 1 H), 1.83 (d, J=7Hz, 3H), 1.64 (bs, 6H), 1.48 (s, 9H).

### o) (3aR,7S,7aS)-7-(4-Methoxy-3-propyl-benzyl)-2,2-dimethyl-5,5-dioxo-hexahydro-1-oxa-5lambda*6*-thia-3-aza-indene-3-carboxylic acid tert-butyl ester

A solution of (3aR,7S,7aS)-7-[4-methoxy-3-((Z)-propenyl)-benzyl]-2,2-dimethyl-5,5-dioxo-hexahydro-1-oxa-5lambda*6*-thia-3-aza-indene-3-carboxylic acid tert-butyl ester (0.30 g, 0.62 mmol) is hydrogenated (1 atm H₂) in MeOH (12 mL) at 25 °C over 10% Pd-C (40 mg) for 30 min. The catalyst is filtered off over Celite and after evaporation of the solvent gives the product as a white foam: TLC (hexane-EtOAc 2:1) Rf=0.39; ESIMS [M+H+NH₃] ⁺=485; ¹H-NMR (400 MHz, CDCl₃): δ 6.96 (d, J=7Hz, 1 H), 6.94 (s,1H), 6.80 (d, J=7 Hz, 1 H), 3.93 (s, 3H), 3.8 (m, 1 H), 3.38 (t, J=12 Hz, 1 H), 2.9-3.1 (m, 2H), 2.5-2.8 (m, 5H), 1.5-1.7 (m, 10H), 1.46 (s, 9H), 0.96 (t, J=7Hz, 3H).

### p) (3R,4S,5S)-3-Amino-5-(4-methoxy-3-propyl-benzyl)-1,1-dioxo-hexahydro-1lambda*6*-thiopyran-4-ol hydrochloride

(3aR,7S,7aS)-7-(4-methoxy-3-propyl-benzyl)-2,2-dimethyl-5,5-dioxo-hexahydro-1-oxa-5lambda*6*-thia-3-aza-indene-3-carboxylic acid tert-butyl ester (0.295 g, 0.62 mmol) is dissolved in 4N HCl in dioxane (5 mL) and stirred at 25°C for 2 h. The solvent is evaporated and the residue is stirred in 2N aqueous HCl (5 mL) and MeOH (20 mL) at 25 °C for 30 min. The solvents are evaporated and the residue is dried to give the hydrochloride salt as a white powder: TLC (CH₂Cl₂MeOH-AcOH-H₂O 180:20:2:1) Rf=0.19; ESIMS [M+H+NH₃] ⁺=328; ¹H-NMR (400 MHz, CD₃OD): δ 7.04 (d, J=7Hz, 1 H), 6.97 (s,1H), 6.89 (d, J=7 Hz, 1 H), 3.84 (s, 3H), 3.3-3.6 (m, 5H), 3.09 (dd, J=12, 10 Hz, 1 H), 2.82 (m, 1 H), 2.58 (t, J=7Hz, 2H), 2.44 (dd, J=9,12Hz, 1 H), 2.27 (m, 1 H), 0.93 (t, J=7Hz, 3H).

### q) (3R,4S,5S)-3-(3-lsopropyl-benzylamino)-5-(4-methoxy-3-propyl-benzyl)-1,1-dioxo-hexahydro-1lambda*6*-thiopyran-4-ol

To a solution of (3R,4S,5S)-3-amino-5-(4-methoxy-3-propyl-benzyl)-1,1-dioxo-hexahydro-1lambda*6*-thiopyran-4-ol hydrochloride (0.087 g, 0.24 mmol) in MeOH-CH₂Cl₂ 1:1 (6 mL) is added sodium acetate (0.04 g, 0.48 mmol) and 3-isopropyl-benzaldehyde (0.045 g, 0.29 mmol). The reaction mixture is stirred at 25 °C for 30 min. before NaBH₃CN (0.032 g, 0.48 mmol) is added followed by stirring for another 60 min. The reaction mixture is acidified with 1N HCl, stirred for 15 min, basified with K₂CO₃-solution and extracted with EtOAc. The combined organic layers are washed with brine, dried over MgSO₄ and evaporated. The pure product is obtained after purification by flash-chromatography on silica gel (hexane-EtOAc 2:1 to EtOAc) as a light yellow oil: TLC (hexane-EtOAc 1: 1) Rf=0.15; ESIMS [M+H]⁺=460; ¹H-NMR (400 MHz, CDCl₃): δ 7.1-7.3 (m, 4H), 6.98 (dd, J=1, 7 Hz, 1H), 6.95 (d, J=1Hz, 1H), 6.79 (d, J=7Hz, 1 H), 3.94 (d, J=14Hz, 1 H), 3.83 (s, 3H), 3.80 (d, J=14Hz, 1 H), 3.43 (m, 1 H), 2.5-3.2 (m, 13H), 2.38 (m, 1 H), 1.60 (m, 2H), 1.28 (d, J=7Hz, 6H), 0.96 (t, J=7Hz, 3H).

### r) (3S,4S,5R)-3-(4-Hydroxy-3-propyl-benzyl)-5-(3-isopropyl-benzylamino)-1,1-dioxo-hexahydro-1lambda*6*-thiopyran-4-ol hydrochloride

To a solution of (3R,4S,5S)-3-(3-isopropyl-benzylamino)-5-(4-methoxy-3-propyl-benzyl)-1,1-dioxo-hexahydro-1lambda*6*-thiopyran-4-ol (0.051 g, 0.11 mmol) in anhydrous CH₂Cl₂ (3 mL) is added under argon at 0 °C a 1M BBr₃-solution in CH₂Cl₂ (0.57 mL). After stirring at 0 °C for 1 h the reaction mixture is poured onto ice-K₂CO₃-solution (5 mL) and stirred for 30 min. The product is extracted with CHCl₃. The combined organic layers are washed with water, dried over MgSO₄ and evaporated. The free base is transferred into the hydrochloride salt with 4N HCl in Et₂O and pure product is obtained after filtration and drying as a light beige powder: TLC (CH2Cl2-MeOH 19:1) Rf=0.23; ESIMS [M+H] ⁺=446; ¹H-NMR (400 MHz, CD₃OD): δ 7.15-7.40 (m, 4H), 6.92 (s, 1 H), 6.84 (dd, J=1, 7 Hz, 1 H), 6.74 (d, J=7Hz, 1 H), 3.90 (d, J=14Hz, 1 H), 3.75 (d, J=14Hz, 1H), 3.42 (m, 1H), 2.9-3.3 (m, 4H), 2.74 (m, 1H), 2.56 (t, J=7Hz, 2H), 2.36 (dd, J=9, 14Hz, 1 H), 2.2 (m, 1H), 1.62 (m, 2H), 1.28 (d, J=7Hz, 6H), 0.96 (t, J=7Hz, 3H).

### Example 2: (3S,4S,5R)-3-(3-Butyl-4-hydroxy-benzyl)-5-(3-isopropyl-benzylamino)-1,1-dioxo-hexahydro-1lambda*6*-thiopyran-4-ol hydrochloride

The title compound is prepared in analogous manner as described for example 1, starting from (3aR,7S,7aS)-7-(3-formyl-4-methoxy-benzyl)-2,2-dimethyl-5,5-dioxo-hexahydro-1-oxa-5!ambda*6*-thta-3-aza-indene-3-carboxytic acid tert-buty ester (see example 1m) and propyl-triphenylphosphoniumbromide: TLC (CH₂Cl₂-MeOH 19:1) Rf=0.31; ESIMS [M+H] ⁺=460; ¹H-NMR (400 MHz, CD₃OD): δ 7.30-7.45 (m, 4H), 6.88 (d, J=1Hz, 1 H), 6.82 (dd, J=1, 7 Hz, 1H), 6.72 (d, J=7Hz, 1H), 4.3 (m, 2H), 3.5-3.7 (m, 4H), 2.7-3.3 (m, 4H), 2.56 (t, J=7Hz, 2H), 2.37 (dd, J=10, 14Hz, 1H), 2.2 (m, 1H), 1.58 (m, 2H), 1.36 (m, 2H), 1.30 (d, J=7Hz, 6H), 0.93 (t, J=7Hz, 3H).

### Example 3: (3S,4S,5R)-3-[4-Hydroxy-3-(2-hydroxy-ethyl)-benzyl]-5-(3-isopropyl-benzylamino)-1,1-dioxo-hexahydro-1lambda*6*-thiopyran-4-ol

### a) (3aR,7S,7aS)-7-[4-Methoxy-3-((Z)-2-methoxy-vinyl)-benzyl]-2,2-dimethyl-5,5-dioxo-hexahydro-1-oxa-5lambda*6*-thia-3-aza-indene-3-carboxylic acid tert-butyl ester and (3aR,7S,7aS)-7-[4-Methoxy-3-((E)-2-methoxy-vinyl)-benzyl]-2,2-dimethyl-5,5-dioxo-hexahydro-1-oxa-5lambda*6*-thia-3-aza-indene-3-carboxylic acid tert-butyl ester

To a suspension of methoxymethyl-triphenylphosphonium chloride (0.46 g, 1.3 mmol) in anhydrous THF (25 mL) is added under Argon a 0.5M KHMDS-solution in toluene (2.6 mL, 1.3 mmol) and the reaction mixture is stirred at 30-35 °C for 45 min. To the resulting orange solution is added a solution of (3aR,7S,7aS)7-(3-formyl-4-methoxy-benzyl)-2,2-dimethyl-5,5-dioxo-hexahydro-1-oxa-5lambda*6*-thia-3-aza-indene-3-carboxylic acid tert-butyl ester (0.3 g, 0.65 mmol) in THF (5 mL) at 0-5 °C. The reaction mixture is stirred at 25°C for 18 h. After addition of NH₄Cl-solution (10 mL) the product is extracted with EtOAc. The combined organic layers are washed with brine, dried over MgSO₄ and evaporated. The pure products are obtained after purification by flash-chromatography on silica gel (hexane-EtOAc 5:2 to 2:1) as a light yellow oil: TLC (hexane-EtOAc 2: 1) Rf=0.37; ESIMS [M+H+NH₃] ⁺=499.

### b) (3aR,7S,7aS)-7-[4-Methoxy-3-(2-methoxy-ethyl)-benzyl]-2,2-dimethyl-5,5-dioxo-hexahydro-1-oxa-5lambda*6*-thia-3-aza-indene-3-carboxylic acid tert-butyl ester

A solution of 3aR,7S,7aS)-7-[4-methoxy-3-((Z)- and 3aR,7S,7aS)-7-[4-methoxy-3-((E)- 2-methoxy-vinyl)benzyl]-2,2-dimethyl-5,5-dioxo-hexahydro-1-oxa-5lambda*6*-thia-3-aza-indene-3-carboxylic acid tert-butyl ester (0.20 g, 0.4 mmol) is hydrogenated (1 atm H₂) in MeOH (12 mL) and THF (4 mL) over 10% Pd-C (40 mg) at 25 °C for 1 h. The catalyst is filtered off over Celite and evaporation of the solvent gives the product as a colorless oil: TLC (hexane-EtOAc 2:1) Rf=0.30; ESIMS [M+H+NH₃] ⁺=501; ¹H-NMR (400 MHz, CDCl₃): δ 6.97 (m, 2H), 6.81 (d, J=7Hz, 1H), 3.84 (s, 3H), 3.8 (m, 1H), 3.58 (t, J=7Hz, 2H), 3.39 (s, 3H), 3.38 (m, 1H), 2.5-3.1 (m, 9H), 1.65 (m, 6H, 1.46 (s, 9H).

### c) (3R,4S,5S)-3-Amino-5-[4-methoxy-3-(2-methoxy-ethyl)-benzyl]-1,1-dioxo-hexahydro-1lambda*6*-thiopyran-4-ol hydrochloride

The title compound is prepared in analogous manner as described for example 1 p, starting from (3aR,7S,7aS)-7-[4-methoxy-3-(2-methoxy-ethyl)-benzyl]-2,2-dimethyl-5,5-dioxo-hexahydro-1-oxa-5lambda*6*-thia-3-aza-indene-3-carboxylic acid tert-butyl ester and 4N HCl: TLC (CH₂Cl₂-MeOH-AcOH-H₂O 180:20:2:1) Rf=0.18; ESIMS [M+H+NH₃] ⁺=344; ¹H-NMR (400 MHz, CD₃OD) δ 7.07 (d, J=7Hz, 1 H), 7.03 (s,1H), 6.92 (d, J=7 Hz, 1 H), 3.82 (s, 3H), 3.3-3.6 (m, 7H), 3.36 (s, 3H), 3.09 (dd, J=12, 10 Hz, 1H), 2.85 (m, 3H), 2.45 (dd, J=10,12Hz, 1 H), 2.28 (m, 1H).

### d) (3R,4S,5S)-3-(3-Isopropyl-benzylamino)-5-[4-methoxy-3-(2-methoxy-ethyl)-benzyl]-1,1-dioxo-hexahydro-1lambda*6*-thiopyran-4-ol

The title compound is prepared in analogous manner as described for example 1q, starting from (3R,4S,5S)-3-amino-5-[4-methoxy-3-(2-methoxy-ethyl)benzyl]-1,1-dioxo-hexahydro-1lambda*6*-thiopyran-4-ol hydrochloride and 3-isopropyl-benzaldehyde: TLC (CH₂Cl₂-MeOH 19:1) Rf=0.42; ESIMS [M+H] ⁺=476; ¹H-NMR (400 MHz, CDCl₃): δ 7.33 (t, J=7Hz, 1 H), 7.2 (m, 3H), 7.01 (dd, J=1, 7 Hz, 1 H), 6.99 (s, 1 H), 6.81 (d, J=7Hz, 1 H), 4.05 (s, 1 H), 3.91 (d, J=14Hz, 1H), 3.82 (s, 3H), 3.78 (d, J=14Hz, 1H), 3.58 (t, J=7Hz, 2H), 3.43 (m, 1H), 3.38 (s, 3H), 2.5-3.2 (m, 11H), 2.38 (m, 1H), 1.28 (d, J=7Hz, 6H).

### e) (3S,4S,5R)-3-[4-Hydroxy-3-(2-hydroxy-ethyl)-benzyl]-5-(3-isopropyl-benzylamino)-1,1-dioxo-hexahydro-1lambda*6*-thiopyran-4-ol

The title compound is prepared in analogous manner, as described for example 1r, starting from (3R,4S,5S)-3-(3-isopropyl-benzylamino)5-[4-methoxy-3-(2-methoxy-ethyl)-benzyl]-1,1-dioxo-hexahydro-1lambda*6*-thiopyran-4-ol and BBr₃: TLC (CH₂Cl₂-MeOH 19:1) Rf=0.23; ESIMS [M+H]⁺=448; ¹H-NMR (400 MHz, CDCl₃): δ 7.34 (t, J=7Hz, 1 H), 7.2 (m, 3H), 6.94 (dd, J=1, 7 Hz, 1 H), 6.87 (d, J=1Hz,1H), 6.83 (d, J=7Hz, 1 H), 3.96 (t, J=7Hz, 2H), 3.91 (d, J=14Hz, 1 H), 3.75 (m, 2H), 3.41 (m, 1 H), 3.05-3.2 (m, 3H), 2.55-3.0 (m, 9H), 2.34 (m, 1 H), 1.28 (d, J=7Hz, 6H).

### Example 4: (3S,4S,5R)-3-(3-Bromo-4-methoxy-benzyl)-5-(3-isopropyl-benzylamino)-1,1-dioxo-hexahydro-1lambda*6*-thiopyran-4-ol hydrochloride

### a) (3R,4S,5S)-3-Amino-5-(3-bromo-4-methoxy-benzyl)-1,1-dioxo-hexahydro-1lambda*6*-thiopyran-4-ol hydrochloride

(3aR,7S,7aS)7-(3-bromo-4-methoxy-benzyl)-2,2-dimethyl-5,5-dioxo-hexahydro-1-oxa-5lambda*6*-thia-3-aza-indene-3-carboxylic acid tert-butyl ester (example 1k) (11.75 g, 23.3 mmol) is dissolved in 4N HCl in dioxane (80 mL) and stirred at 25 °C for 2 h. The solvent is evaporated and the residue is stirred in 2N aqueous HCl (15 mL) and MeOH (150 mL) at 25 °C for 30 min. The solvents are evaporated and the residue crystallized from MeOH-Et₂O to give the hydrochloride salt as white crystals: mp 269-272 °C; TLC (CH₂Cl₂-MeOH-AcOH-H₂O 180:20:2:1) Rf=0.18; ESIMS [M+H]⁺=364, 366.

### b) (3S,4S,5R)-3-(3-Bromo-4-methoxy-benzyl)-5-(3-isopropyl-benzylamino)-1,1-dioxo-hexahydro-1lambda*6*-thiopyran-4-ol hydrochloride

The title compound is prepared in analogous manner as described for example 1q, starting from (3R,4S,5S)-3-amino-5-(3-bromo-4-methoxy-benzyl)-1,1-dioxo-hexahydro-1lambda*6*-thiopyran-4-ol hydrochloride and 3-isopropyl-benzaldehyde: mp 173-5 °C, TLC (CH₂Cl₂-MeOH 19:1) Rf=0.36; ESIMS [M+H] ⁺=496, 498; ¹H-NMR (400 MHz, CDCl₃): ): δ 7.40 (d, J=1Hz, 1 H), 7.15-7.35 (m, 4H), 7.09 (dd, J=1, 7 Hz, 1 H), 6.85 (d, J=1Hz, 1H), 4.08 (bs, 1 H), 3.94 (d, J=14Hz, 1H), 3.92 (s, 3H), 3.79 (d, J=14Hz, 1H), 3.43 (m, 1H), 2.9-3.2 (m, 5H), 2.7 (m, 4H), 2.40 (m, 1 H), 1.28 (d, J=7Hz, 6H).

### Example 5: (3S,4S,5R)-3-(3-Bromo-4-hydroxy-benzyl)-5-(3-isopropyl-benzylamino)-1,1-dioxo-hexahydro-1lambda*6*-thiopyran-4-ol hydrochloride

The title compound is prepared in analogous manner as described for example 1r, starting from (3S,4S,5R)-3-(3-bromo-4-methoxy-benzyl)-5-(3-isopropyl-benzylamino)-1,1-dioxo-hexahydro-1lambda*6*-thiopyran-4-ol (example 4b) and BBr₃: m.p. 181-184 °C; [α]²⁰_{D} +13° (c 0.2 MeOH); TLC (CH2Cl2-MeOH 19:1) Rf=0.22; ESIMS [M+H] ⁺= 482, 484; ¹H-NMR (400 MHz, DMSO-d₆): δ 9.0 (bs, 1 H), 7.47 (s, 1H), 7.25-7.4 (m, 4H), 7.04 (dd, J=1, 7 Hz, 1 H), 6.89 (d, J=1 Hz, 1 H), 6.1 (bd, 1 H), 4.24 (bs, 1 H), 2.75-3.85 (m, 10H), 2.44 (m, 1H), 2.05 (m, 1 H), 1.24 (d, J=7Hz, 6H).

### Example 6: (3S,4S,SR)-3-(3-Bromo-4-hydroxy-benzyl)-5-[(S)-1-(3-isopropyl-phenyl)-ethylamino]-1,1-dioxo-hexahydro-1lambda*6*-thiopyran-4-ol

### a) (3S,4S,5R)-3-(3-Bromo-4-methoxy-benzyl)-5-[(S)-1-(3-isopropyl-phenyl)-ethylamino]-1,1-dioxo-hexahydro-1lambda*6*-thiopyran-4-ol

To a suspension of (3R,4S,5S)-3-amino-5-(4-methoxy-3-propyl-benzyl)-1,1-dioxo-hexahydro-thiopyran-4-ol (0.15 g, 0.4 mmol) and 1-(3-isopropyl-phenyl)-ethanone (0.094 g, 0.57 mmol) in dichloroethane (2 mL) and iso-pentanol (0.5 mL) is added pulverized molecular sieve (0.1 g) and the reaction mixture is heated in a microwave oven at 150 °C for 0.5 h and at 160 °C for 1 h. To the reaction mixture is added NaBH₃CN (0.053 g, 0.8 mmol), AcOH (0.05 mL) and MeOH (3 mL). After stirring for 0.5 h at 25 °C, the suspension is filtered and evaporated. The residual oil is dissolved in EtOAc, washed with K₂CO₃-solution and brine, dried over MgSO₄ and concentrated. The product is obtained as a mixture of diastereoisomers after purification by flash-chromatography on silica gel (hexane-CH₂Cl₂-MeOH 50:50:5 to 0:50:5) as a light yellow solid: TLC (CH₂Cl₂-MeOH 19:1) Rf=0.54; ESIMS [M+H] ⁺=510, 512.

### b) (3S,4S,5R)-3-(3-Bromo-4-hydroxy-benzyl)-5-[(S)-1-(3-isopropyl-phenyl)-ethylamino]-1,1-dioxo-hexahydro-1lambda*6*-thiopyran-4-ol

The title compound is prepared in analogous manner as described for example 1r, starting from (35,45,5R)-3-(3-bromo-4-methoxy-benzyl)-5-[(S)-1-(3-isopropyl-phenyl)-ethylamino]-1,1-dioxo-hexahydro-1lambda*6*-thiopyran-4-ol and BBr₃. The diastereoisomers are separated by flash-chromatography on silica gel (hexane-CH₂Cl₂-MeOH 50:50:5 to 0:50:5) as beige solids: TLC (CH₂Cl₂-MeOH 19:1) Rf=0.31 and 0.21; ESIMS [M+H] ⁺=496, 498; ¹H-NMR (400 MHz, CDCl₃): δ 7.33 (m, 2H), 7.2 (d, J=7Hz, 1 H), 7.16 (s, 1H), 7.14 (d, J=7Hz, 1H), 7.02 (dd, J=7, 1 Hz, 1 H), 6.95 (d, J=7Hz, 1H), 3.84 (m, 1 H), 2.6-3.2 (m, 9H), 2.54 (dd, J=12, 14Hz, 1 H), 2.38 (m, 1 H), 1.45 (d, J=7Hz, 3H), 1.29 (d, J=7Hz, 6H).

### Example 7: (3S*,4S*,5R*)-3-(3-Bromo-4-hydroxy-benzyl)-5-[(S*)-1-(3-cyclopropyl-phenyl)-ethylamino]-1,1-dioxo-hexahydro-1lambda*6*-thiopyran-4-ol tfifluoroacetate

### a) (3S*,4S*,5R*)-3-(3-Bromo-4-methoxy-benzyl)-5-[(S*)-1-(3-cyclopropyl-phenyl)-ethylamin ]-1,1-dioxo-hexahydro-1lambda*6*-thiopyran-4-ol

A solution of (3R*,4S*,5S*)-3-amino-5-(4-methoxy-3-propyl-benzyl)1,1-dioxo-hexahydro-1lambda*6*thiopyran-4-ol (0.265 g, 0.7 mmol) and 1-(3-cyclopropyl-phenyl)-ethanone (0.232, 1.4 mmol) in iso-pentanol (5 mL) is heated at reflux for 16 h. The reaction mixture is diluted with MeOH (10 mL) and AcOH (0.3 mL) and NaBH₃CN (0.17 g, 2.8 mmol) is added at 25 °C and stirred for 30 min. The reaction mixture is evaporated, basified with K₂CO₃-solution and extracted with EtOAc. The combined organic layers are washed with brine, dried over MgSO₄ and evaporated. The pure 5-(S)- and 5-(R)-diastereoisomers are obtained after separation by flash-chromatography on silica gel (hexane-EtOAc 1:2 to EtOAc) as white solids: TLC (CH₂Cl₂-MeOH 19:1) Rf=0.58 and 0.36; ESIMS [M+H+NH₃] ⁺=508, 510; ¹H-NMR (400 MHz, CDCl₃): δ 7.38 (d, J=1Hz, 1H), 7.0-7.35 (m, 5H), 6.84 (d, J=7Hz, 1H) 3.9 (m,4H), 3.46 (m, 1H), 2.5-3.2 (m, 8H), 2.24 (m, 1H), 1.94 (m, 1H), 1.42 (d, J=7Hz, 3H), 1.02 (m, 2H), 0.75 (m, 2H).

### b) (3S*,4S*,5R*)-3-(3-Bromo-4-hydroxy-benzyl)-5-[(S*)-1-(3-cyclopropyl-phenyl)-ethylamino]-1,1-dioxo-hexahydro-1lambda*6*-thiopyran-4-ol trifluoroacetate

The title compound is prepared in analogous manner as described for example 1 r, starting from (3S*,4S*,5R*)3-(3-bromo-4-methoxy-benzyl)-5-[(S*)1-(3-cyclopropyl-phenyl)-ethylamino]-1,1-dioxo-hexahydro-1lambda*6*-thiopyran-4-ol and BBr₃. The title compound is obtained by preparative HPLC (Nucleosil 100-5 C₁₈, water-ACN 80:20 to ACN) as a colorless foam: TLC (CH₂Cl₂-MeOH 19:1) Rf=0.11; ESIMS [M+H] ⁺=494, 496; ¹H-NMR (400 MHz, CDCl₃): ): δ 6.9-7.4 (m, 5H), 4.37 (m, 1H), 3.78 (m, 1H), 3.2-3.4 (m, 4H), 2.7-3.0 (m, 2H), 2.2-2.4 (m, 2H), 1.94 (m, 1 H), 1.74 (d, J=7Hz, 3H), 1.04 (m, 2H), 0.78 (m, 2H).

### Example 8: (35,45,5R)-3-(3-Bromo-4-hydroxy-benzyl)-5-[(4-isopropyl-pyridin-2-yl-methyl)-amino]-1,1-dioxo-hexahydro-1lambda*6*-thiopyran-4-ol di-hydrochloride

### a) 4-lsopropyl-pyridine-2-carboxylic acid methyl ester

4-lsopropyl-pyridine-2-carbonitrile (CAS registry 676136-14-4) (1.62 g, 10.8 mmol) is dissolved in 10N HCl in MeOH (25 mL) and heated at 60 °C for 16h. The reaction mixture is evaporated and the residue is basified with cold NaHCO₃-solution and extracted with EtOAc. The combined organic layers are washed with brine, dried over MgSO4 and evaporated to give the product as a light yellow oil: TLC (CH₂Cl₂MeOH 19:1) Rf=0.47; ESIMS [M+H] ⁺=180; ¹H-NMR (400 MHz, CDCl₃): ): δ 8.85 (d, J=7Hz, 1H), 8.04 (d, J=1Hz, 1H), 7.36 (dd, J=1, 7Hz, 1H), 4.03 (s, 3H), 3.02 (m, 1H), 1.32 (d, J=7Hz, 1H).

### b) 4-Isopropyl-pyridine-2-carbaldehyde

To a solution of the 4-isopropyl-pyridine-2-carboxylic acid methyl ester (1.5 g, 8.4 mmol) in CH₂Cl₂ (50 mL) is added a 1 M DIBAL solution in hexane at -78 °C. After 2 h the reduction is quenched with AcOH (1.5 mL). The reaction mixture is stirred for 30 min and then diluted with water. The product is extracted EtOAc and the organic layers are washed with NaHCO₃-solution and brine, dried over MgSO₄ and evaporated to give the product as a light yellow oil: TLC (CH₂Cl₂-MeOH 19:1) Rf=0.44; ESIMS [M+H] ⁺=150; ¹H-NMR (400 MHz, CDCl₃): ): δ 9.60 (s, 1H), 8.70 (d, J=7Hz, 1H), 7.86 (d, J=1Hz, 1H), 7.42 (dd, J=1, 7Hz, 1H), 3.03 (m, 1H), 1.34 (d, J=7Hz, 1H).

### c) (3S,4S,5R)-3-(3-Bromo-4-methoxy-benzyl)-5-[(4-isopropyl-pyridin-2-ylmethyl)-amino]-1,1-dioxo-hexahydro-1lambda*6*-thiopyran-4-ol

The title compound is prepared in analogous manner as described for example 1 q, starting from (3R,4S,5S)-3-amino-5-(3-bromo-4-methoxy-benzyl)-1,1-dioxo-hexahydro-1lambda*6*-thiopyran-4-ol hydrochloride and 4-isopropyl-pyridine-2-carbaldehyde: TLC (CH₂Cl₂-MeOH 19:1) Rf=0.08; ESIMS [M+H] ⁺=497, 499.

### d) (3S,4S,5R)-3-(3-Bromo-4-hydroxy-benzyl)-5-[(4-isopropyl-pyridin-2-ylmethyl)-amino]-1,1-dioxo-hexahydro-1lambda*6*-thiopyran-4-ol di-hydrochloride

The title compound is prepared in analogous manner as described for example 1r, starting from (3S,4S,5R)-3-(3-bromo-4-methoxy-benzyl)-5-[(4-isopropyl-pyridin-2-ylmethyl)-amino]-1,1-dioxo-hexahydro-1lambda*6*-thiopyran-4-ol and BBr₃: TLC (CH₂Cl₂-MeOH 19:1) Rf=0.18; ESIMS [M+H] ⁺= 483, 485; ¹H-NMR (400 MHz, CD₃OD): δ 8.63 (d, J=7Hz, 1H), 7.67 (s, 1 H), 7.55 (d, J=7Hz, 1H), 7.39 (s, 1H), 7.06 (d, J=7Hz, 1H), 6.91 (d, J=7Hz, 1H), 4.52 (m, 2H), 3.0-3.7 (m, 7H), 2.88 (m, 1H), 2.52 (dd, J=9, 14Hz, 1H), 2.28 (m, 1H), 1.36 (d, J=7Hz, 6H).

### Example 9 (3S,4S,5R)-3-(3-Bromo-4-methoxy-benzyl)-5-[(4-cyclopropyl-pyridin-2-ylmethyl)-amino]-1,1-dioxo-hexahydro-1lambda*6*-thiopyran-4-ol

The title compound is prepared in analogous manner as described for example 7, starting from (3R,4S,5S)-3-amino-5-(3-bromo-4-methoxy-benzyl)-1,1-dioxo-hexahydro-1lambda*6*-thiopyran-4-ol hydrochloride and 4-cyclopropyl-pyridine-2-carbaldehyde: TLC (CH₂Cl₂-MeOH 19:1) Rf=0.12; ESIMS [M+H] ⁺=481, 483; ¹H-NMR (400 MHz, OD₃OD): δ 8.56 (bd, J=7Hz, 1H), 7.4-7.6 (m, 2H), 7.39 (d, J=1Hz, 1H), 7.07 (dd, J=1; 7Hz, 1H), 6.91 (d, J=7Hz, 1H), 4.48 (m, 2H), 3.0-3.7 (m, 7H), 2.87 (m, 1H), 2.54 (dd, J=9,14Hz, 1H), 2.29 (m, 1H), 2.18 (m, 1H), 1.36 (m, 2H), 1.06 (m, 2H).

### Example 10: (3S*,4S*,5R*)-3-(3-Bromo-4-hydroxy-benzyl)-5-(3-cyclopropyl-benzylamino)-1,1-dioxo-hexahydro-1lambda*6*-thiopyran-4-ol hydrochloride

### a) (3aR*,7S*,7aS*)-7-(3-Bromo-4-methoxy-benzyl)-3-(3-cyclopropyl-benzyl)-5,5-dioxo-hexahydro-1-oxa-5lambda*6*-thia-3-aza-inden-2-one

To an oil-free suspension of sodium hydride (0.72 g 50% in oil, 15 mmol) in THF-DMF 5:1 (175 mL) is added [(3R*,4S*,5S*)5-(3-bromo-4-methoxy-benzyl)-4-hydroxy-1,1-dioxo-hexahydro-1lambda*6*thiopyran-3-yl]-carbamic acid tert-butyl ester (example 1j) (2.322 g, 5.0 mmol) in portions at 25 °C. After stirring the reaction mixture at 25 °C for 2 h and at 50 °C for 1 h, the 1-chloromethyl-3-cyclopropyl-benzene (0.93 g, 5.5 mmol) dissolved in THF (5 mL) is added to the reaction mixture and stirred at 25 °C for another 16 h. The reaction is quenched with 10% aqueous NH₄Cl-solution (50 mL) and the product is extracted with EtOAc. The combined organic layers are washed with KHSO₄-solution and brine, dried over MgSO₄ and evaporated to give the product as a light yellow oil, which after crystallization from THF-Et₂O gave the title compound as white crystals: TALC (hexane-EtOAc 1:1) Rf=0.38; ESIMS [M+H+NH₃]⁺=537, 539; ¹H-NMR (400 MHz, CDCl₃): δ 7.37 (s, 1H), 7.26 (t, J=7Hz, 1 H), 7.06 (m, 4H), 6.89 (d, J=7Hz, 1 H), 4.54 (d, J=14Hz, 1 H), 4.32 (d, J=14Hz, 1 H), 3.96 (s, 3H), 3.7-3.9 (m, 2H), 2.6-3.2 (m, 7H), 1.92 (m, 1H), 1.02 (m, 2H), 0.72 (m, 2H).

### b) (3aR*,7S*,7aS*)-7-(3-Bromo-4-hydroxy-benzyl)-3-(3-cyclopropyl-benzyl)-5,5-dioxo-hexahydro-1-oxa-5lambda*6*-thia-3-aza-inden-2-one

To a solution of (3aR*,7S*,7aS*)-7-(3-bromo-4-methoxy-benzyl)-3-(3-cyclopropyl-benzyl)-5,5-dioxo-hexahydro-1-oxa-5lambda*6*-thia-3-aza-inden-2-one (0.150 g, 0.28 mmol) in CH₂Cl₂ (3 mL) is added at 0 °C a 1 M BBr₃ solution in CH₂Cl₂ (1.4 mL, 1.4 mmol) and the reaction mixture is stirred at 0-5 °C for 4 h. After hydrolysis of the excess BBr₃ with ice-water (5 mL) the product is extracted with CH₂Cl₂. The combined organic layers are washed with NaHCO₃-solution and water, dried over MgSO₄ and evaporated. Pure product is obtained after purification by flash-chromatography on silica gel (hexane-EtOAc 2:1 to EtOAc) as a light yellow foam: TLC (hexane-EtOAc 1:1) Rf=0.26; ESIMS [M+H+NH₃] ⁺=523, 525; ¹H-NMR (400 MHz, CDCl₃): δ 7.0-7.4 (m, 6H), 6.87 (d, J=7Hz, 1H), 5.7 (bs, 1H), 4.54 (d, J=14Hz, 1H), 4.30 (d, J=14Hz, 1H), 3.7-3.9 (m, 2H), 2.0-3.2 (m, 7H), 1.89 (m, 1H), 1.0 (m, 2H), 0.72 (m, 2H).

### c) (3S*,4S*,5R*)-3-(3-Bromo-4-hydroxy-benzyl)-5-(3-cyclopropyl-benzylamino)-1,1-dioxo-hexahydro-1lambda*6*-thiopyran-4-ol hydrochloride

To a suspension of (3aR*,7S*,7aS*)7-(3-bromo-4-hydroxy-benzyl)3-(3-cyclopropyl-benzyl-5,5-dioxo-hexahydro-1-oxa-5lambda*6*-thia-3-aza-inden-2-one (0.084 g, 0.166 mmol) in dioxane-water 2:1 (10 mL) is added Ba(OH)₂ 8H₂O (0.32 g, 6 mmol) and the reaction mixture is heated at reflux for 2 h. After dilution with EtOAc (30 mL), the reaction mixture is filtered and the filtrate is washed with brine, dried over MgSO₄ and evaporated. The pure title compounds is obtained after purification by flash-chromatography on silica gel (CH₂Cl₂-MeOH-hexane 50:5:50 to 50:5:0) and crystallization of the hydrochloride salt from CH₂Cl₂ as white crystals: TLC (CH₂Cl₂-MeOH-ACOH-H₂O 180:20:2:1) Rf=0.52; ESIMS [M+H+NH₃] ⁺=480, 482; ¹H-NMR (400 MHz, DMSO-d₆): δ 9.05 (bs, 1H), 7.35 (m, 4H), 7.2 (d, J=7Hz, 1H), 7.0d (dd, J=1, 7 Hz, 1H), 6.93 (d, J=7Hz, 1H), 6.18 (br, 1H), 4.22 (m, 2H), 3.0-3.9 (m, 7H), . 2.79 (m, 1H), 2.44 (dd, J=9, 14Hz, 1H), 2.05 (m, 1H), 1.95 (m, 1H), 1.01 (m, 2H), 0.75 (m, 2H).

### Example 11: (3R*,4S*,5S*)-3-(3-Cyclopropyl-benzylamino)-5-[4-hydroxy-3-(3-propyl-benzyl)-benzyl]-1,1-dioxo-hexahydro-1lambda*6*-thiopyran-4-ol hydrochloride

### a) (3aR*,75*,7aS*)-3-(3-Cyclopropyl-benzyl)-7-[4-methoxy-3-(4,4,5,5-tetramethyl-[1,3,2]dioxaborolan-2-yl)-benzyl]-5,5-dioxo-hexahydro-1-oxa-5lambda*6*-thia-3-aza-inden-2-one

To a solution of (3aR*,7S*,7aS*)-7-(3-bromo-4-methoxy-benzyl)-3-(3-cyclopropyl-benzyl)-5,5-dioxo-hexahydro-1-oxa-5lambda*6*-thia-3-aza-inden-2-one (example 10 a) (0.60 g, 1.14 mmol) in dioxane (10 mL) is added under Argon [1,1'-bis(diphenylphosphino)ferrocene]-dichloropalladium(II) (0.049 g, 0.057 mmol), 4,4,5,5-tetramethyl-1,3,2-dioxaborolane as a 1 M solution in THF (3.4 mL, 3.4 mmol) and NEt₃ (0.96 mL, 6.8 mmol) and the degassed reaction mixture is heated in the microwave oven at 200 °C for 20 min. The reaction mixture is diluted with EtOAc and washed with saturated NaHCO₃-solution and brine. The organic layer is dried over MgSO₄ and evaporated. The product is obtained after purification by flash-chromatography on silica gel (hexane-EtOAc 3:1 to 1:2) as a light yellow foam: TLC (hexane-EtOAc 1:1) Rf=0.26; ESIMS [M+H+NH₃] ⁺=585; ¹H-NMR (400 MHz, CDCl₃): δ 7.39 (d, J=1Hz, 1H), 7.0-7.3 (m, 5H), 6.84 (d, J=7Hz, 1H), 4.54 (d, J=14Hz, 1H), 4.28 (d, J=14Hz, 1H), 3.84 (s, 3H), 3.7-3.9 (m, 2H), 2.1-2.9 (m, 7H), 1.89 (m, 1H), 1.38 (s, 12H), 1.02 (m, 2H), 0.72 (m, 2H).

### b) (3aR*,75*,7aS*)-3-(3-Cyclopropyl-benzyl)-7-[4-methoxy-3-(3-propyl-benzyl)-benzyl]-5,5-dioxo-hexahydro-1-oxa-5lambda*6*-thia-3-aza-inden-2-one

To a solution of (3aR*,7S*,7aS*)-3-(3-cyclopropyl-benzyl)-7-[4-methoxy-3-(4,4,5,5-tetramethyl-[1,3,2]dioxaborolan-2-yl)benzyl]-5,5-dioxo-hexahydro-1-oxa-5lambda*6*-thia-3-aza-inden-2-one (0.35 g, 0.2 mmol) in EtOH (5 mL) is added under Argon 1-bromomethyl-3-propyl-benzene (CAS reg. no. 216658-92-3) (0.097 g, 0.45 mmol), K₂CO₃ (0.096 g, 0.7
mmol) and [1,1'-bis(diphenylphosphino)ferrocene]dichloropalladium(II) (0.015 g, 0.017 mmol) and the degassed reaction mixture is heated in the microwave oven at 120 °C for 20 min. The reaction mixture is diluted with EtOAc and washed with saturated NaHCO₃-solution and brine. The organic layer is dried over MgSO₄ and evaporated. The product is obtained after purification by flash-chromatography on silica gel (hexane-EtOAc 10:1 to EtOAc) as a light yellow foam: TLC (hexane-EtOAc 1:1) Rf=0.46; ESIMS [M+H+NH₃] ⁺=591; ¹H-NMR (400 MHz, CDCl₃): δ 6.8-7.3 (m, 7H), 4.52 (d, J=14Hz, 1H), 4.27 (d, J=14Hz, 1 H), 3.83 (s, 3H), 3.7-4.0 (m, 4H), 2.5-3.2 (m, 9H), 1.91 (m, 1 H), 1.64 (m, 2H), 1.02 (m, 2H), 0.96 (t, J=7Hz, 3H), 0.72 (m, 2H).

### c) (3aR*,7S*,7aS*)-3-(3-Cyclopropyl-benzyl)-7-[4-hydroxy-3-(3-propyl-benzyl)-benzyl]-5,5-dioxo-hexahydro-1-oxa-5lambda*6*-thia-3-aza-inden-2-one

To a solution of (3aR*,7S*,7aS*)-3-(3-cyclopropyl-benzyl)-7-[4-methoxy-3-(3-propyl-benzyl)-benzyl]-5,5-dioxo-hexahydro-1-oxa-5lambda*6*-thia-3-aza-inden-2-one (0.160 g, 0.275
mmol) in 1,2-dichlorethane (10 mL) is added under Argon BBr₃-Me₂S (0.55 g, 1.66 mmol) and the reaction mixture is heated at reflux for 16 h. The reaction mixture is added to an ice-K₂CO₃-solution and stirred for 0.5 h. The product is extracted with CHCl₃. Combined extracts are washed with water, dried over MgSO₄ and evaporated to yield the title compound as a beige foam: TLC (hexane-EtOAc 1:1) Rf=0.37; ESIMS [M+H+NH₃] ⁺=577.

### d) (3R*,4S*,5S*)-3-(3-Cyclopropyl-benzylamino)-5-[4-hydroxy-3-(3-propyl-benzyl)-benzyl]-1,1-dioxo-hexahydro-1lambda*6*-thiopyran-4-ol hydrochloride

To a suspension of (3aR*,7S*,7aS*)3-(3-cyclopropyl-benzyl)7-[4-hydroxy-3-(3-propyl-benzyl)-benzyl]-5,5-dioxo-hexahydro-1-oxa-5lambda*6*-thia-3-aza-inden-2-one (0.15 g, 0.265 mmol) in dioxane-water 2:1 (10 mL) is added Ba(OH)₂ 8H₂O (0.507 g, 1.59 mmol) and the reaction mixture is heated in a microwave oven at 150 °C for 0.5 h. After dilution with EtOAc (30 mL), the reaction mixture is filtered and the filtrate is washed with brine, dried over MgSO₄ and evaporated. The pure title compounds is obtained after purification by flash-chromatography on silica gel (CH₂Cl₂-MeOH-hexane 50:5:50 to 50:5:0) and crystallization of the hydrochloride salt from CH₂Cl₂-Et₂O as beige crystals: TLC (CH₂Cl₂-MeOH 19:1) Rf=0.26; ESIMS [M+H+NH₃] ⁺=534; ¹H-NMR (400 MHz, CDCl₃, free base): δ 7.0-7.3 (m, 7H), 6.92 (s, 1H), 6.90 (d, J=8Hz, 1H), 6.72 (d, J=8Hz, 1H), 3.97 (m, 2H), 3.85 (d, J=13Hz, 1H), 3.71 (d, J=13Hz, 1H), 3.39 (dt, J=14, 4Hz, 1H), 3.12 (m, 2H), 3.04 (t, J=10Hz, 1H), 2.92 (dt, J=14, 4Hz, 1H), 2.67 (dd, J=10, 12Hz, 1H), 2.61 (m, 1H), 2.57 (t, J=8Hz, 2H), 2.35 (m, 1H), 1.93 (m, 1H), 1.65 (m, 2H), 1.31 (m, 1H), 1.02 (m, 2H), 0.96 (t, J=7Hz, 3H), 0.75 (m, 2H).

### Example 12: (3R*,4S*,5S*)-3-(3-Cyclopropyl-benzylamino)-5-[4-hydroxy-3-(3-methyl-benzyl)-benzyl]-1,1-dioxo-hexahydro-1lambda*6*-thiopyran-4-ol

The title compound is prepared in analogous manner as described in example 11, starting from (3aR*,7S*,7aS*)-3-(3-cyclopropyl-benzyl)-7-[4-methoxy-3-(4,4,5,5-tetramethyl-[1,3,2]dioxaborolan-2-yl)-benzyl]-5,5-dioxo-hexahydro-1-oxa-5lambda*6*-thia-3-aza-inden-2-one and 3-methyl-benzyl bromide: TLC (CH₂Cl₂-MeOH 19:1) Rf=0.10; ESIMS [M+H] ⁺=506; ¹H-NMR (400 MHz, CDCl₃, free base): δ 6.9-7.3 (m, 12H), 6.77 (d, J=7Hz, 1H), 4.52 (d, J=14Hz, 1H), 4.27 (d, J=14Hz, 1H), 3.83 (s, 3H), 3.7-4.0 (m, 5H), 3.42 (m; 1H), 2.9-3.2 (m, 4H),2.65 (m, 3H), 2.36 (m, 1H), 2.34 (s, 3H), 1.93 (m, 1H), 1.03 (m, 2H), 0.75 (m, 2H).

### Example 13: (3S,4S,5R)-3-(4-Hydroxy-benzyl)-5-(3-isopropyl-benzylamino)-1,1-dioxo-hexahydro-1lambda*6*-thiopyran-4-ol hydrochloride

### a) (3aR,7S,7aS)-7-(3-Bromo-4-methoxy-benzyl)-3-(3-isopropyl-benzyl)-5,5-dioxo-hexahydro-1-oxa-5lambda*6*-thia-3-aza-inden-2-one

To a solution of (35,45,5R)-3-(3-bromo-4-methoxy-benzyl)-5-(3-isopropyl-benzylamino)1,1-dioxo-hexahydro-1lambda*6*-thiopyran-4-ol (9.18 g, 18.5 mmol) (example 5) in 1,2-dichlorethane (150 mL) is added DIPEA (6.7 mL, 37 mmol), carbonyl-diimidazole (7.68 g, 46.4 mmol) and a catalytic amount of DMAP (0.116 g, 0.93 mmol). The reaction mixture is heated at reflux for 1.5 h. After dilution with CH₂Cl₂, the organic layer is extracted with K₂CO₃-solution and water, dried over MgSO₄ and evaporated. Crystallization from CH₂Cl₂-Et₂O gives the product as light yellow crystals: m.p. 188-190 °C; TLC (hexane/EtOAc 1:1) Rf=0.32; ESIMS [M+H+NH₃] ⁺=539, 541; ¹H-NMR (400 MHz, CDCl₃): δ 7.1-7.4 (m, 6H), 6.89 (d, J=7Hz, 1H), 4.55 (d, J=14Hz, 1H), 4.36 (d, J=14Hz, 1Hz), 3.94 (s, 3H), 3.91 (m, 1H), 3.81 (dd, J=12, 14Hz, 1H), 2.6-3.2 (m, 8H), 1.25 (d, J=7Hz, 6H).

### b) (3aR,7S,7aS)-3-(3-Isopropyl-benzyl)-7-(4-methoxy-benzyl)-5,5-dioxo-hexahydro-1-oxa-5lambda*6*-thia-3-aza-inden-2-one

(3aR,7S,7aS)-7-(3-Bromo-4-methoxy-benzyl)-3-(3-isopropyl-benzyl)-5,5-dioxo-hexahydro-1-oxa-5lambda*6*-thia-3-aza-inden-2-one (0.732 g, 1.4 mmol) is hydrogenated (1 atm H₂) at 40 °C in THF-MeOH 1:1 (50 mL) with 10% Pd-C as catalyst (0.2 g) for 16 h. The catalyst is filtered off and the filtrate is evaporated. Recrystallisation from EtOAc-hexane gave the product as white crystals: TLC (hexane/EtOAc 1:1) Rf=0.38; ESIMS [M+H+NH₃] ⁺=461; ¹H-NMR (400 MHz, CDCl₃): δ 7.05-7.40 (m, 6H), 6.89 (d, J=7Hz, 2H), 4.58 (d, J=14Hz, 1H), 4.35 (d, J=14Hz), 3.90 (dt, J=4, 12Hz, 1H), 3.84 (s, 3H), 3.80 (dd, J=12, 14Hz, 1H), 2.6-3.2 (m, 8H), 1.25 (d, J=7Hz; 6H).

### c) (3aR,7S,7aS)-7-(4-Hydroxy-benzyl)-3-(3-isopropyl-benzyl)-5,5-dioxo-hexahydro-1-oxa-5lambda*6*-thia-3-aza-inden-2-one

To a solution of (3aR,7S,7aS)-3-(3-isopropyl-benzyl)-7-(4-methoxy-benzyl)-5,5-dioxo-hexahydro-1-oxa-5lambda*6*-thia-3-aza-inden-2-one (0.560 g, 1.23 mmol) in anhydrous CH₂Cl₂ (20 mL) is added under Argon at 0 °C a 1M BBr₃-solution in CH₂Cl₂ (6.3 mL). After stirring at 0 °C for 1 h the reaction mixture is poured onto ice-K₂CO₃-solution (5 mL) and stirred for 30 min. The product is extracted with CHCl₃. The combined organic layers are washed with water, dried over MgSO₄ and evaporated. The product is obtained after filtration and drying as a white solid: TLC (hexane-EtOAc 1:1) Rf=0.24; ESIMS [M+H+NH₃] ⁺=447; ¹H-NMR (400 MHz, CDCl₃): δ 7.1-7.4 (m, 4H), 7.04 (d, J=7Hz, 2H), 6.83 (d, J=7Hz, 2H), 4.56 (d, J=14Hz, 1 H), 4.35 (d, J=14Hz, 1 Hz), 3.89 (dt, J=4, 12Hz, 1 H), 3.79 (t, J=12Hz, 1 H), 2.6-3.2 (m, 8H), 1.25 (d, J=7Hz, 6H).

### d) (3S,4S,5R)-3-(4-Hydroxy-benzyl)-5-(3-isopropyl-benzylamino)-1,1-dioxo-hexahydro-1lambda*6*-thiopyran-4-ol hydrochloride

To a suspension of (3aR,75,7aS)-7-(4-hydroxy-benzyl)-3-(3-isopropyl-benzyl)-5,5-dioxo-hexahydro-1-oxa-5lambda*6*-thia-3-aza-inden-2-one (0.05 g, 0.115 mmol) in dioxane-water 1:1 (2 mL) is added Ba(OH)₂ 8H₂O (0.185 g, 0.575 mmol) and the resulting suspension was heated in a microwave oven at 160 °C for 10 min. After dilution with EtOAc (30 mL), the reaction mixture is filtered and the filtrate is washed with saturated NaHCO₃-solution and brine, dried over MgSO₄ and evaporated. The pure title compounds is obtained after crystallization of the hydrochloride salt from ACN-Et₂O as white crystals: m.p. 196-198 °C. TLC (CH₂Cl₂-MeOH-AcOH-H₂O 180:20:2:1) Rf=0.40; ESIMS [M+H] ⁺=404; ¹H-NMR (400 MHz, CD₃OD): δ 7.3-7.5 (m, 4H), 7.04 (d, J=7Hz, 2H), 6.79 (d, J=7Hz, 2H), 4.34 (m, 2H), 3.2-3.8 (m, 5H), 3.09 (dd, J=12, 14Hz, 1H), 3.02 (m, 1H), 2.84 (m, 1H), 2.44 (dd, J=12, 14Hz,1H), 2.24 (m, 1H), 2.6-3.2, 1.33 (d, J=7Hz, 6H).

### Example 14: (3S,4S,5R)-3-Benzyl-5-(3-isopropyl-benzylamino)-1,1-dioxo-hexahydro-1lambda*6*-thiopyran-4-ol hydrochloride

### a) (3aR,75,7aS)-3-(3-Isopropyl-benzyl)-7-(4-trifluoromethansulfonyl-benzyl)-5,5-dioxo-hexahydro-1-oxa-5lambda*6*-thia-3-aza-inden-2-one

To a solution of (3aR,7S,7aS)-7-(4-hydroxy-benzyl)-3-(3-isopropyl-benzyl)-5,5-dioxo-hexahydro-1-oxa-5lambda*6*-thia-3-aza-inden-2-one (example 13c) (0.122 g, 0.285 mmol) in CH₂Cl₂ (5 mL) is added 2,6-lutidine (0.062 mL, 0.51 mmol) and trifluoromethanesulfonic acid anhydride (0.082 mL, 0.5 mmol) at 0-5 °C and the reaction mixture is stirred at 0-5 °C for 3 h. The reaction mixture is poured onto cold 1 N HCl and is extracted with CH₂Cl₂. The combined organic layers are washed with water, dried over MgSO₄ and evaporated. The product is obtained after purification by flash-chromatography on silica gel (hexane-EtOAc 3:1) as a white solid: TLC (toluene-EtOAc 3:1) Rf=0.29; ESIMS [M+H+NH₃] ⁺=579; ¹H-NMR (400 MHz, CDCl₃): δ 7.1-7.35 (m, 8H), 4.55 (d, J=14Hz, 1H), 4.35 (d, J=14Hz, 1Hz), 3.90 (dt, J=4, 12Hz, 1H), 3.81 (t, J=12Hz, 1H), 2.7-3.3 (m, 8H), 1.25 (d, J=7Hz, 6H).

### b) (3aR,7S,7aS)-7-Benzyl-3-(3-isopropyl-benzyl)-5,5-dioxo-hexahydro-1-oxa-5lambda*6*-thia-3-aza-inden-2-one

To a solution of (3aR,7S,7aS)-3-(3-isopropyl-benzyl)-7-(4-trifluoromethansulfonyl-benzyl)-5,5-dioxo-hexahydro-1-oxa-5lambda*6*-thia-3-aza-inden-2-one (0.075 g, 0.13 mmol) in DMF (2 mL) is added under Argon triethylsilane (0.055 mL, 0.33 mmol), 1,3-bis-(phenylphosphino)-propane (0.001 g, 0.003 mmol) and Pd(OAc)₂ (0.001 g, 0.003 mmol) and the reaction mixture is heated in a microwave oven at 130°C for 15 min. The reaction mixture is evaporated and the residue is re-dissolved in EtOAc and washed with water and brine, dried over MgSO₄ and evaporated. The crude product is crystallized from EtOAc-Et₂O to give the product as beige crystals: TLC (hexane-EtOAc 1:1) Rf=0.48; ESIMS [M+H+NH₃] ⁺=431; ¹H-NMR (400 MHz, CDCl₃): δ 7.1-7.4 (m, 9H), 4.56 (d, J=14Hz, 1H), 4.34 (d, J=14Hz, 1Hz), 3.92 (dt, J=4, 12Hz, 1 H), 3.81 (t, J=12Hz, 1 H), 2.7-3.2 (m, 8H), 1.26 (d, J=7Hz, 6H).

### c) (3S,4S,5R)-3-Benzyl-5-(3-isopropyl-benzylamino)-1,1-dioxo-hexahydro-1lambda*6*-thiopyran-4-ol hydrochloride

The title compound is prepared in analogous manner as described for example 13d, starting from (3aR,75,7aS)-7-benzyl-3-(3-isopropyl-benzyl)-5,5-dioxo-hexahydro-1-oxa-slambda*6*-thia-3-aza-inden-2-one and Ba(OH)₂ 8H₂O: m.p. 239-242 °C; TLC (CH₂Cl₂-MeOH 19:1) Rf=0.30; ESIMS [M+H] ⁺=388; ¹H-NMR (400 MHz, CD₃OD): δ 7.2-7.5 (m, 9H), 4.34 (m, 2H), 3.3-3.8 (m, 5H), 3.14 (dd, J=12, 14Hz, 1H), 3.01 (m, 1H), 2.78 (m, 1H), 2.5 (dd, J=12, 14Hz, 1H), 2.29 (m, 1H), 1.33 (d, J=7Hz, 6H).

### Example 15: (3S,4S,5R)-3-[4-Hydroxy-3-(3-methoxymethyl-5-methyl-benzyl)-benzyl]-5-(3-isopropy)-benzylamino)-1,1-dioxo-hexahydro-1lambda*6*-thiopyran-4-ol hydrachloride

### a) (3aR,7S,7aS)-3-(3-Isopropyl-benzyl)-7-[4-methoxy-3-(4,4,5,5-tetramethyl-[1,3,2]dioxaborolan-2-yl)-benzyl]-5,5-dioxo-hexahydro-1-oxa-5lambda*6*-thia-3-aza-inden-2-one

To a solution of (3aR,7S,7aS)-7-(3-bromo-4-methoxy-benzyl)-3-(3-isopropyl-benzyl)-5,5-dioxo-hexahydro-1-oxa-5lambda*6*-thia-3-aza-inden-2-one (example XX a) (1.20 g, 2.27 mmol) in dioxane (20 mL) is added under Argon [1,1'-bis(diphenylphosphino)ferrocene]-dichloropalladium(II) (0.098 g, 0.114 mmol), 4,4,5,5-tetramethyl-1,3,2-dioxaborolane as a 1 M solution in THF (6.8 mL, 6.8 mmol) and NEt₃ (1.92 mL, 13.6 mmol) and the degassed reaction mixture was heated in the microwave oven at 200 °C for 20 min. The reaction mixture is diluted with EtOAc and washed with 10% citric acid and brine. The organic layer is dried over MgSO₄ and evaporated. The product is obtained after purification by flash-chromatography on silica gel (hexane-EtOAc 8:1 to EtOAc) as a yellow solid: TLC (hexane-EtOAc 1:1) Rf=0.34; ESIMS [M+H+NH₃]⁺= 587; ¹H-NMR (400 MHz, CDCl₃): δ 7.41 (d, J=1Hz, 1H), 7.1-7.3 (m, 5H), 6.85 (d, J=7Hz, 1H), 4.54 (d, J=14Hz, 1H), 4.34 (d, J=14Hz, 1H), 3.90 (dt, J=4, 12Hz, 1H), 3.86 (s, 3H), 3.82, dd, J=12, 14Hz, 1H), 2.6-3.2 (m, 8H), 1.38 (s, 12H), 1.27 (d, J=7Hz, 6H).

### b) 5-[(3aR,7S,7aS)-3-(3-Isopropyl-benzyl)-2,5,5-trioxo-octahydro-l-oxa-5lambda*6*-thia-3-aza-inden-7-ylmethyl]-2-methoxy-boronic acid

To a solution of (3aR,7S,7aS)-3-(3-isopropyl-benzyl)-7-[4-methoxy-3-(4,4,5,5-tetramethyl-[1,3,2]dioxaborolan-2-yl)-benzyl]-5,5-dioxo-hexahydro-1-oxa-5lambda*6*-thia-3-aza-inden-2-one (0.322 g, 1.9 mmol) in THF-water 4:1 (40 mL) is added NalO₄ (1.24 g, 5.7 mmol) and the reaction mixture is stirred for 0.5 h at 25 °C. After addition of 2N HCl (1.9 mL) the reaction mixture was stirred at 25 °C for 3 h before it is diluted with EtOAc and separated. The organic layer is washed with brine, dried over MgSO₄ and evaporated. The product is obtained after purification by flash-chromatography on silica gel (hexane-EtOAc 4:1 to EtOAc containing 0.1 % AcOH) as a light yellow foam: TLC (hexane-EtOAc 1:1) Rf=0.12; ESIMS [M+H+NH₃] ⁺=505; ¹H-NMR (400 MHz, CDCl₃): δ 7.59 (d, J=1Hz, 1H), 7.1-7.4 (m, 5H), 6.85 (d, J=7Hz, 1H), 4.55 (d, J=14Hz, 1 H), 4.34 (d, J=14Hz, 1H), 3.93 (s, 3H), 3.90 (dt, J=4, 12Hz, 1H), 3.81, dd, J=12, 14Hz, 1H), 2.6-3.2 (m, 8H), 1.28 (d, J=7Hz, 6H).

### c) 2-Hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-boronic acid

To a solution of 5-[(3aR,7S,7aS)-3-(3-isopropyl-benzyl)-2,5,5-trioxo-octahydro-1-oxa-5lambda*6*-thia-3-aza-inden-7-ylmethyl]-2-methoxy-boronic acid (0.39 g, 0.8 mmol) in anhydrous CH₂Cl₂ (15 mL) is added under Argon at -30 °C a 1 M BBr₃-solution in CH₂Cl₂ (4.0 mL) and the reaction mixture is allowed to warm to room temperature. After stirring at 25 °C for 3 h the reaction mixture is poured onto ice-K₂CO₃-solution (5 mL) and stirred for 30 min. The aqueous layer is acidified with citric acid to pH 4 and the product is extracted with CH₂Cl₂. The combined organic layers are washed with water, dried over MgSO₄ and evaporated. The product is obtained after purification by flash-chromatography on silica gel (CH₂Cl₂-MeOH 19:1) as a light yellow foam: TLC (CH₂Cl₂MeOH 19:1) Rf=0.44; ESIMS [M-H] =472; ¹H-NMR (400 MHz, CDCl₃): δ 8.16 (bs, 1H), 7.1-7.6 (m, 7H), 4.55 (d, J=14Hz, 1H), 4.34 (d, J=14Hz, 1H), 3.90 (m, 2H), 2.6-3.2 (m, 8H), 1.26 (d, J=7Hz, 6H).

### d) (3aR,7S,7aS)-7-[4-Hydroxy-3-(3-methoxymethyl-5-methyl-benzyl)-benzyl]-3-(3-isopropyl-benzyl)-5,5-dioxo-hexahydro-1-oxa-5lambda*6*-thia-3-aza-inden-2-one

To a solution 2-hydroxy-5-[(3aR,75,7aS)-3-(3-isopropyl-benzyl)-2,5,5-trioxo-octahydro-1-oxa-5lambda*6*-thia-3-aza-inden-7-ylmethyl]-boronic acid (0.06 g, 0.124 mmol) in EtOH-dioxane 4:1 (5 mL) is added under Argon 1-bromomethyl-3-methoxymethyl-5-methyl-benzene (0.037 g, 0.16 mmol), [1,1'-bis(diphenylphosphino)ferrocene]dichloropalladium(II) (0.005 g, 0.006 mmol) and K₂CO₃ (0.27 g, 0.19 mmol) and the degassed reaction mixture was heated in the microwave oven at 120 °C for 20 min. The reaction mixture is diluted with EtOAc, filtered over Celite and evaporated. The product is obtained after purification by flash-chromatography on silica gel (toluene-EtOAc 8:1 to 1:2) as a colorless foam: TLC (toluene-EtOAc 3:1) Rf=0.22; ESIMS [M+H+NH₃] ⁺=595.

### e) (3S,4S,5R)-3-[4-Hydroxy-3-(3-methoxymethyl-5-methyl-benzyl)-benzyl]-5-(3-isopropyl-benzylamino)-1,1-dioxo-hexahydro-1lambda*6*-thiopyran-4-ol

To a suspension of (3aR,7S,7aS)-7-[4-hydroxy-3-(3-methoxymethyl-5-methyl-benzyl)-benzyl]-3-(3-isopropyl-benzyl)-5,5-dioxo-hexahydro-1-oxa-5lambda*6*-thia-3-aza-inden-2-one (0.028 g, 0.048 mmol) in dioxane-water 1:1 (2 mL) is added Ba(OH)₂ 8H₂O (0.076 g, 0.24 mmol) and the resulting suspension was heated in a microwave oven at 160 °C for 15 min. After dilution with EtOAc (30 mL) and NaHCO₃-solution (3 mL), The product is extracted with EtOAc. The combined layers are washed with brine, dried over MgSO₄ and evaporated. The pure title compound is obtained after crystallization of the hydrochloride salt from ACN-Et2O as a white solid: TLC (CH₂Cl₂-MeOH-AcOH-H₂O 180:20:2:1) Rf=0.54; ESIMS [M+H] ⁺=552; ¹H-NMR (400 MHz, CD₃OD): δ 7.94 (s, 1H), 7.3-7.5 (m, 4H), 6.9-7.1 (m, 3H), 6.89 (dd, J=1, 7Hz, 1H), 6.85 (s, 1H), 6.79 (d, J=7Hz, 1H), 4.36 (s, 2H), 4.32 (m, 4H), 3.93 (s, 3H), 2.1-3.8 (m, 9H), 2.33 (s, 3H), 1.31 (d, J=7Hz, 6H).

### f) (3-Methoxymethyl-5-methyl-phenyl)-methanol

To a solution of (3-hydroxymethyl-5-methyl-phenyl)-methanol (CAS reg. no. 27711-63-3) (3.1 g, 20.1 mmol) in DMF (60 mL) is added under Argon NaH (1.13 g 60% in oil, 28.2 mmol) in two portions and the reaction mixture is stirred at 25 °C for 0.5 h followed by 0.5 h at 50 °C. After cooling to 10 °C methyl iodide (2.55 mL, 40.3 mmol) is added within 15 min and the reaction mixture is stirred at 25°C for 3 h. The reaction is quenched with 10% aqueous NH₄Cl-solution. After removal of the solvents, the residual solid is extracted from saturated NaHCO₃-solution with EtOAc. Combined extracts are washed with brine, dried over MgSO₄ and evaporated. The crude product is obtained after flash-chromatography on silica gel (toluene-EtOAc 10:1 to EtOAc) as a colorless oil: TLC (EtOAc) Rf=0.56; ESIMS [M+H+NH₃] ⁺=184.

### g) 1-Bromomethyl-3-methoxymethyl-5-methyl-benzene

To a solution of (3-methoxymethyl-5-methyl-phenyl)-methanol (1.7 g, 10.1 mmol) in anhydrous THF (40 mL) is added phosphorous tribromide (0.49 mL, 5.1 mmol) at 0-5 °C and the resulting reaction mixture is stirred at 25 °C for 3 h and 2 h at 40-50 °C. After addition of water at 5-10°C the product is extracted with EtOAc. Combined extracts are washed with brine, dried over MgSO₄ and evaporated. The crude product is purified by flash-chromatography on silica gel (hexane-EtOAc 2:1 to 1:1) as a light yellow oil: TLC (hexane-EtOAc 1:1) Rf=0.60; ESIMS [M+H+NH₃] ⁺=246, 248; ¹H-NMR (400 MHz, CDCl₃): δ 7.20 (s, 1H), 7.16 (s, 1H), 7.12 (s, 1H), 4.50 (s, 2H), 4.45 (s, 2H), 3.42 (s, 3H), 3.38 (s, 3H).

### Example 16: (3S,4S,5R)-3-(3-Bromo-benzyl)-5-(3-isopropyl-benzylamino)-1,1-dioxo-hexahydro-1lambda*6*-thiopyran-4-ol

### a) (3aR,7S,7aS)-7-(3-Bromo-4-hydroxy-benzyl)-3-(3-isopropyl-benzyl)-5,5-dioxo-hexahydro-1-oxa-5lambda*6*-thia-3-aza-inden-2-one

To a solution of (3aR,7S,7aS)-7-(3-bromo-4-methoxy-benzyl)-3-(3-isopropyl-benzyl)-5,5-dioxo-hexahydro-1-oxa-5lambda*6*-thia-3-aza-inden-2-one (0.400 g, 0.75 mmol) in anhydrous CH₂Cl₂ (15 mL) is added under Argon at 0 °C a 1 M BBr₃-solution in CH₂Cl₂ (3.75 mL). After stirring at 0 °C for 1 h the reaction mixture is poured onto ice-K₂CO₃-solution (5 mL) and stirred for 30 min. The product is extracted with CHCl₃. The combined organic layers are washed with water, dried over MgSO₄ and evaporated. The product is obtained after filtration and drying as a white solid: TLC (hexane-EtOAc 1:1) Rf=0.31; ESIMS [M+H+NH₃] ⁺=525, 527; ¹H-NMR (400 MHz, CDCl₃): δ 7.1-7.4 (m, 3H), 7.1 (s, 1H), 7.06 (d, J=7Hz, 1H), 6.91 (dd, J=7, 1Hz, 1H), 6.86 (d, J=7Hz), 4.46 (d, J=14Hz, 1H), 4.31 (d, J=14Hz, 1Hz), 3.82 (m, 2H), 2.5-3.4 (m, 8H), 1.22 (d, J=7Hz, 6H).

### b) Trifluoro-methanesulfonic acid 2-bromo-4-[(3aR,7S,7aS)-3-(3-isopropyl-benzyl)-2,5,5-trioxo-octahydro-1-oxa-5lambda*6*-thia-3-aza-inden-7-ylmethyl]-phenyl ester

To a solution of (3aR,7S,7aS)-7-(3-bromo-4-hydroxy-benzyl)-3-(3-isopropyl-benzyl)-5,5-dioxo-hexahydro-1-oxa-5lambda*6*-thia-3-aza-inden-2-one (0.362 g, 0.71 mmol) in CH₂Cl₂ (15 mL) is added 2,6-lutidine (0.16 mL, 1.30 mmol) and trifluoromethanesulfonic acid anhydride (0.20 mL, 1.2 mmol) at 0-5 °C and the reaction mixture is stirred at 0-5 °C for 16 h. The reaction mixture is poured onto cold 1 N HCl and is extracted with CH₂Cl_{2:} The combined organic layers are washed with water, dried over MgSO₄ and evaporated. The product is obtained after purification by flash-chromatography on silica gel (hexane-EtOAc 2:1) as a beige solid: TLC (hexane-EtOAc 1:1) Rf=0.41; ESIMS [M+H+NH₃] ⁺=657, 659; ¹H-NMR (400 MHz, CDCl₃): δ 7.1-7.35 (m, 7H), 4.56 (d, J=14Hz, 1H), 4.37 (d, J=14Hz, 1Hz), 3.90 (dt, J=4, 12Hz, 1H), 3.82 (t, J=12Hz, 1H), 2.7-3.3 (m, 8H), 1.25 (d, J=7Hz, 6H).

### c) (3aR,7S,7aS)-7-(3-Bromo-benzyl)-3-(3-isopropyl-benzyl)-5,5-dioxo-hexahydro-1-oxa-5lambda*6*-thia-3-aza-inden-2-one

To a solution of trifluoro-methanesulfonic acid 2-bromo-4-[(3aR,7S,7aS)-3-(3-isopropylbenzyl)-2,5,5-trioxo-octahydro-1-oxa-5lambda*6*-thia-3-aza-inden-7-ylmethyl]-phenyl ester (0.050 g, 0.075 mmol) in DMF (1.5 mL) is added under Argon triethylsilane (0.035 mL, 0.2 mmol), DPPP (0.001 g, 0.002 mmol) and Pd(OAc)₂ (0.002 g, 0.01 mmol) and the reaction mixture is heated in a microwave oven at 100 °C for 2x15 min. The reaction mixture is evaporated and the product is obtained after purification by flash-chromatography on silica gel (hexane-EtOAc 2:1) as a white solid: TLC (hexane-EtOAc 1:1) Rf=0.40; ESIMS [M+H+NH₃]⁺=509, 511; ¹H-NMR (400 MHz, CDCl₃): δ 7.1-7.4 (m, 8H), 4.56 (d, J=14Hz, 1H), 4.34 (d, J=14Hz, 1Hz), 3.89 (dt, J=4, 12Hz, 1H), 3.81 (t, J=12Hz, 1H), 2.6-3.2 (m, 8H), 1.26 (d, J=7Hz, 6H).

### d) (3S,4S,5R)-3-(3-Bromo-benzyl)-5-(3-isopropyl-benzylamino)-1,1-dioxo-hexahydro-1lambda*6*-thiopyran-4-ol

The title compound is prepared in analogous manner as described for example 15e, starting from (3S,4S,5R)-3-(3-bromo-benzyl)-5-(3-isopropyl-benzylamino)-1,1-dioxo-hexahydro-1lambda*6*-thiopyran-4-ol and Ba(OH)₂ 8H₂O: TLC (CH₂Cl₂-MeOH 19:1) Rf=0.57; ESIMS [M+H] ⁺=466, 468; ¹H-NMR (400 MHz, CD₃OD): δ 7.2-7.5 (m, 8H), 4.35 (m, 2H), 3.3-3.8 (m, 5H), 3.15 (dd, J=12, 14Hz, 1H), 3.01 (m, 1H), 2.84 (m, 1H), 2.54 (dd, J=12, 14Hz, 1H), 2.29 (m, 1H), 1.33 (d, J=7Hz, 6H).

### Example 17: (3R,4S,5S)-3-(3-Isopropyl-benzylamino)-1,1-dioxo-5-(3-propoxy-benzyl)-hexahydro-1lambda*6*-thiopyran-4-ol

### a) (3aR,7S,7aS)-3-(3-Isopropyl-benzyl)-5,5-dioxo-7-(3-propoxy-benzyl)-hexahydro-1-oxa-5lambda*6*-thia-3-aza-inden-2-one

To a solution of (3aR,7S,7aS)-7-(3-bromo-benzyl)-3-(3-isopropyl-benzyl)-5,5-dioxo-hexahydro-1-oxa-5lambda*6*-thia-3-aza-inden-2-one (0.236 g, 0.48 mmol) in toluene (2 mL) is added under Argon Cs₂CO₃ (0.240 g, 0.72 mmol), 2-(di-butylphosphino)-1,1'-binaphtyl (0.016 g, 0.039 mmol), 1-propanol (0.074 mL, 0.96 mmol) and Pd(OAc)₂ (0.006 g, 0.024 mmol). The reaction mixture is heated in a microwave oven at 130°C for 2x 30 min. The reaction mixture is diluted with ACN, filtered and evaporated. The product is obtained after purification by flash-chromatography on silica gel (hexane-EtOAc 2:1) as a light yellow solid: TLC (toluene-EtOAc 3:1) Rf=0.44; ESIMS [M+H+NH₃]⁺=489; ¹H-NMR (400 MHz, CDCl₃): δ 7.2-7.4 (m, 4H), 7.16 (s, 1H), 7.11 (d, J=7hz, 1H), 6.84 (dd, J=7, 1Hz,1H), 6.69 (s, 1H), 4.57 (d, J=14Hz, 1H), 4.36 (d, J=14Hz, 1Hz), 3.8-4.0 (m, 4H), 2.6-3.2 (m, 8H), 1.83 (m, 2H), 1.26 (d, J=7Hz, 6H), 1.09 (t, 7Hz, 3H).

### b) (3R,4S,5S)-3-(3-Isopropyl-benzylamino)-1,1-dioxo-5-(3-propoxy-benzyl)-hexahydro-1lambda*6*-thiopyran-4-ol

The title compound is prepared in analogous manner as described for example 15e, starting from (3aR,75,7aS)-3-(3-isopropyl-benzyl)-5,5-dioxo-7-(3-propoxy-benzyl)-hexahydro-1-oxa-5lambda*6*-thia-3-aza-inden-2-one and Ba(OH)₂ 8H₂O: TLC (CH₂Cl₂-MeOH 19:1) Rf=0.41; ESIMS [M+H] ⁺=446; ¹H-NMR (400 MHz, CD₃OD): δ 6.8-7.5 (m, 8H), 4.34 (m, 2H), 3.96 (t, J=7Hz, 2H), 3.3-3.8 (m, 5H), 3.11 (dd, J=12, 14Hz, 1H), 3.06 (m, 1H), 2.92 (m, 1H), 2.48 (dd, . J=12, 14Hz, 1H), 2.31 (m, 1H), 1.82 (m, 2H), 1.33 (d, J=7Hz, 6H), 1.07 (t, J=7Hz, 3H).

### Example 18: (3S,4S,5R)-3-(3-Butoxy-benzyl)-5-(3-isopropyl-benzylamino)-1,1-dioxo-hexahydro-1lambda*6*-thiopyran-4-ol

The title compound is obtained by an analogous reaction sequence as for example 17, starting from (3S,4S,5R)-3-(3-bromo-benzyl)-5-(3-isopropyl-benzylamino)1,1-dioxo-hexahydro-1lambda*6*-thiopyran-4-ol and n-butanol: TLC (CH₂Cl₂-MeOH 19: 1) Rf=0.49; ESIMS [M+H] ⁺=460; ¹H-NMR (400 MHz, CD₃OD): δ 6.8-7.5 (m, 8H), 4.33 (m, 2H), 4.0 (t, J=7Hz, 2H), 3.3-3.8 (m, 5H), 3.15 (dd, J=12, 14Hz, 1H), 3.02 (m, 1H), 2.91 (m, 1H), 2.48 (dd, J=12, 14Hz, 1H), 2.32 (m, 1H), 1.79 (m, 2H), 1.53 (m, 2H), 1.33 (d, J=7Hz, 6H), 1.03 (t, J=7Hz, 3H).

### Example 19: 3R,4S,5S)-3-(3-Isopropyl-benzylamino)-1,1-dioxo-5-(3-pentyloxy-benzyl)-hexahydro-1lambda*6*-thiopyran-4-ol

The title compound is obtained by an analogous reaction sequence as for example 17, starting from (3S,4S,5R)-3-(3-bromo-benzyl)-5-(3-isopropyl-benzylamino)-1,1-dioxo-hexahydro-1lambda*6*-thiopyran-4-ol and n-pentanol: TLC (CH₂Cl₂-MeOH 19:1) Rf=0.56; ESIMS [M+H] ⁺=474; ¹H-NMR (400 MHz, CD₃OD): δ 6.7-7.5 (m, 8H), 4.34 (m, 2H), 3.97 (t, J=7Hz, 2H), 3.3-3.8 (m, 5H), 3.14 (dd, J=12, 14Hz, 1H), 3.03 (m, 1H), 2.85 (m, 1H), 2.48 (dd, J=12, 14Hz, 1H), 2.32 (m, 1H), 1.82 (m, 2H), 1.46 (m, 4H), 1.33 (d, J=7Hz, 6H), 0.98 (t, J=7Hz, 3H).

### Example 20: (3S,4S,5R)-3-(3-Ethyl-4-hydroxy-benzyl)-5-(3-isopropyl-benzylamino)-1,1-dioxo-hexahydro-1lambda*6*-thiopyran-4-ol hydrochloride

### a) (3aR,7S,7aS)-3-(3-Isopropyl-benzyl)-7-(4-methoxy-3-vinyl-benzyl)-5,5-dioxo-hexahydro-1-oxa-5lambda*6*-thia-3-aza-inden-2-one

To a solution of (3aR,7S,7aS)-7-(3-bromo-4-methoxy-benzyl)-3-(3-isopropyl-benzyl)-5,5-dioxo-hexahydro-1-oxa-5lambda*6*-thia-3-aza-inden-2-one (example 13a) (2.44 g, 4.67 mmol) in dioxane (60 mL) is added under Argon Cs₂CO₃ (3.12 g, 9.38 mmol), 2,4,6-trivinylcyclotriboroxane-pyridine complex (1.78 g, 7.03 mmol), tri-tert-butylphosphine (0.145 g, 0.70 mmol) and Pd₂(dba)₃ (0.216 g, 0.234 mmol). The reaction mixture is heated at reflux for 0.5 h, filtered over Celite and evaporated. The residue is dissolved in EtOAc and washed with K₂CO₃-solution and brine. The organic layer is dried over MgSO₄ and evaporated. The product is obtained after crystallization from EtOAc-Et₂O as white crystals: mp 174-176°C; TLC (hexane-EtOAc 1:1) Rf=0.52; ESIMS [M+H] ⁺=487.

### b) (3aR,7S,7aS)-7-(3-Ethyl-4-methoxy-benzyl)-3-(3-isopropyl-benzyl)-5,5-dioxo-hexahydro-1-oxa-5lambda*6*-thia-3-aza-inden-2-one

A solution of (3aR,7S,7aS)-3-(3-isopropyl-benzyl)-7-(4-methoxy-3-vinyl-benzyl)-5,5-dioxo-hexahydro-1-oxa-5lambda*6*-thia-3-aza-inden-2-one (0.10 g, 0.2 mmol) is hydrogenated (1 atm H₂) in MeOH (10 mL) over 10% Pd-C (20 mg) at 25°C for 1.5 h. The catalyst is filtered off over Celite and after evaporation of the solvent the product is obtained as a white foam: TLC (hexane-EtOAc 2:1) Rf=0.22; ESIMS [M+H+NH₃] ⁺=489; ¹H-NMR (400 MHz, CDCl₃): δ 7.32 (t, J=7Hz, 1H), 7.24 (d, J=7Hz, 1H), 7.16 (s, 1H), 7.13 (d, J=7Hz, 1H). 6.94 (dd, J=7, 1Hz, 1 H), 6.92 (s, 1H), 6.81 (d, J=7Hz, 1H), 4.56 (d, J=14Hz, 1H), 4.33 (d, J=14Hz, 1H), 3.92 (dt, J=3, 12Hz, 1H), 3.84 (s, 3H), 3.81 (t, J=12 Hz, 1H), 2.6-3.2 (m, 10H), 1.29 (d, J=7Hz, 6H), 1.02 (t, J=7Hz, 3H).

### c) (3aR,7S,7aS)-3-Benzyl-7-(3-ethyl-4-hydroxy-benzyl)-5,5-dioxo-hexahydro-1-oxa-5lambda*6*-thia-3-aza-inden-2-one

The title compound is prepared in analogous manner as described for example 10b from (3aR,7S,7aS)-3-benzyl-7-(3-ethyl-4-methoxy-benzyl)-5,5-dioxo-hexahydro-1-oxa-5lambda*6*-thia-3-aza-inden-2-one and BBr₃: TLC (hexane-EtOAc 1:1) Rf=0.36; ESIMS [M+H+NH₃] ⁺=475; ¹H-NMR (400 MHz, CDCl₃): δ 7.32 (t, J=7Hz, 1H), 7.23 (d, J=7Hz, 1H), 7.16 (s, 1H), 7.13 (d, J=7Hz, 1H). 6.91 (d, J=1Hz, 1H), 6.88 (dd, J=7, 1 Hz, 1H), 6.75 (d, J=7Hz, 1H), 4.86 (s, 1H), 4.57 (d, J=14Hz, 1H), 4.34 (d, J=14Hz, 1H), 3.91 (dt, J=3, 12, 4Hz, 1H), 3.80 (t, J=12 Hz, 1H), 2.6-3.2 (m, 10H), 1.27 (d, J=7Hz, 6H), 1.24 (t, J=7Hz, 3H).

### d) (3S,4S,5R)-3-(3-Ethyl-4-hydroxy-benzyl)-5-(3-isopropyl-benzylamino)-1,1-dioxo-hexahydro-1lambda*6*-thiopyran-4-ol hydrochloride

The title compound is prepared in analogous manner as described for example 15e, starting from (3aR,7S,7aS)-3-benzyl-7-(3-ethyl-4-hydroxy-benzyl)-5,5-dioxo-hexahydro-1-oxa-5lambda*6*-thia-3-aza-inden-2-one and Ba(OH)₂ 8H₂O: TLC (CH₂Cl₂-MeOH 19:1) Rf=0.27; ESIMS [M+H]⁺=432; ¹H-NMR (400 MHz, CD₃OD): δ 7.3-7.6 (m, 4H), 6.95 (d, J=1 Hz, 1 H), 6.87 (dd, J=7, 1 Hz, 1 H), 6.74 (d, J=7Hz, 1 H), 4.35 (m, 2H), 3.5-3.8 (m, 4H), 3.28 (dd, J=12, 4Hz, 1 H), 3.09 (dd, J=12, 14Hz, 1 H), 3.0 (m, 1 H), 2.82 (m, 1 H), 2.62 (q, J=7Hz, 2H), 2.41 (dd, J=12, 14Hz, 1 H), 2.22 (m, 1H), 1.34 (d, J=7Hz, 6H), 1.21 (t, 7Hz, 3H).

### Example 21: (3S,4S,5R)-3-(4-Hydroxy-3-hydroxymethyl-benzyl)-5-(3-isopropyl-benzy)amino)-1,1-dioxo-hexahydro-1lambda*6*-thiopyran-4-ol

### a) 5-[(3aR,7S,7aS)-3-(3-Isopropyl-benzyl)-2,5,5-trioxo-octahydro-1-oxa-5lambda*6*-thia-3-aza-inden-7-ylmethyl]-2-methoxy-benzaldehyde

A solution of (3aR,7S,7aS)-3-(3-isopropyl-benzyl)-7-(4-methoxy-3-vinyl-benzyl)-5,5-dioxo-hexahydro-1-oxa-5lambda*6*-thia-3-aza-inden-2-one (5.15 g, 10.5 mmol) in CH₂Cl₂MeOH 4:1 (200 mL) is ozonized at -78°C until the blue color persisted. The ozonide is destroyed by stirring the reaction mixture overnight at 25°C with di-methylsulfide (1.6 mL), 21.4 mmol). After evaporation the product is crystallized from Et₂O to give the title compound as white crystals: mp 198-200 °C; TLC (hexane-EtOAc 1:1) Rf=0.23; ESIMS [M+H]=489; ¹H-NMR (400 MHz, CDCl₃): δ 10.46 (s, 1H), 7.58 (s, 1 H), 7.42 (dd, J=7, 1 Hz, 1 H), 7.31 (t, J=7Hz, 1H), 7.24 (d, J=7Hz, 1H), 7.16 (s, 1 H), 7.13 (d, J=7Hz, 1 H). 7.01 (d, J=1Hz, 1 H), 4.57 (d, J=14Hz, 1 H), 4.34 (d, J=14Hz, 1 H), 3.96 (s, 3H), 3.91 (dt, J=12, 4Hz, 1 H), 3.82 (t, J=12 Hz, 1H), 2.6-3.2 (m, 10H), 1.26 (d, J=7Hz, 6H).

### b) 2-Hydroxy-5-[(3aR,7S,7aS)-3-(3-isopropyl-benzyl)-2,5,5-trioxo-octahydro-oxa-5lambda*6*-thia-3-aza-inden-7-ylmethyl]-benzaldehyde

The title compound is prepared in analogous manner as described for example 1r, starting from 5-[(3aR,7S,7aS)-3-(3-isopropyl-benzyl)2-,5,5-trioxo-octahydro-1-oxa-5lambda*6*-thia-3-aza-inden-7-ylmethyl]-2-methoxy-benzaldehyde and BBr₃: TLC (toluene-EtOH-NH₃ conc. 90:20:1) Rf=0.45; ESIMS [M+H]⁺= 475.

### c) (3aR,7S,7aS)-7-(4-Hydroxy-3-hydroxymethyl-benzyl)-3-(3-isopropyl-benzyl)-5,5-dioxo-hexahydro-1-oxa-5lambda*6*-thia-3-aza-inden-2-one

To a solution of 2-hydroxy-5-[(3aR,7S,7aS)-3-(3-isopropyl-benzyl)2,5,5-trioxo-octahydro-1-oxa-5lambda*6*-thia-3-aza-inden-7-ylmethyl]-benzaldehyde (0.16 g, 0.35 mmol) in THF (10 mL) is added under Argon LiBH₄ (0.028 g, 1.2 mmol) at 25°C. After stirring for 15 min.at 25 °C the reaction mixture is diluted with a 20% KHSO₄-solution and extracted with EtOAc. Combined layers are washed with K₂CO₃-solution and brine, dried over MgSO₄ and evaporated. The title compound is obtained as a yellow foam: TLC (CH₂Cl₂-MeOH-AcOH-H₂O 180:20:2:1) Rf=0.36; ESIMS [M+H+NH₃]⁺=477.

### d) (3S,4S,5R)-3-(4-Hydroxy-3-hydroxymethyl-benzyl)-5-(3-isopropyl-benzylamino)-1,1-dioxo-hexahydro-1lambda*6*-thiopyran-4-ol

The title compound is prepared in analogous manner as described for example 15e, starting from (3aR,7S,7aS)-7-(4-hydroxy-3-hydroxymethyl-benzyl)-3-(3-isopropyl-benzyl)-5,5-dioxo-hexahydro-1-oxa-5lambda*6*-thia-3-aza-inden-2-one and Ba(OH)₂ 8H₂O: TLC (CH₂Cl₂-MeOH-AcOH-H₂O 180:20:2:1) Rf=0.31; ESIMS [M+H+NH₃]⁺=434; ¹H-NMR (400 MHz, CDCl₃):δ 7.34 (t, J=7Hz,1H), 7.2 (m, 3H), 7.02 (d J=7Hz, 1H), 6.85 (s, 1H), 6.83(d, J=7Hz, 1H), 4.84 (s, 2H), 3.92 (d, J=14Hz, 1H), 3.78 (d, J=14Hz, 1H), 3.74 (m, 1H), 3.41 (dt, J=12, 4Hz, 1 H), 2.6-3.2 (m, 8H), 2.35 (m, 1H), 1.29 (d, J=7Hz, 6H).

### Example 22: N-Benzyl-N-ethyl-2-hydroxy-5-[(3S,4S,5R)-4-hydroxy-5-(3-isopropyl-benzylamino)-1,1-dioxo-hexahydro-1lambda*6*-thiopyran-3-ylmethyl]-benzamide hydrochloride

### a) 5-[(3aR,7S,7aS)-3-(3-Isopropyl-benzyl)-2,5,5-trioxo-octahydro-1-oxa-5lambda*6*-thia-3-aza-inden-7-ylmethyl]-2-methoxy-benzoic acid

A mixture of 5-[(3aR,7S,7aS)-3-(3-Isopropyl-benzyl)-2,5,5-trioxo-octahydro-1-oxa-5lambda*6*-thia-3-aza-inden-7-ylmethyl]-2-methoxy-benzaldehyde (2.3 g, 4.8 mmol), TEMPO (0.073 g, 0.34 mmol), ACN (100 mL) and sodium phosphate buffer (10 mL, 0.67 M, pH 6.7) is heated to 35°C. Then NaClO₂ (0.1.52 g, 13.2 mmol) in H₂O (4 mL) and dilute bleach (5 mL 2% in H₂O) is added simutaneously at 40°C over a period of 2 h. After 5 h the reaction mixture is poured onto cold NaSO₃ solution and after stirring for 0.5 h, the solution is basified with 2N NaOH to pH 10. After extraction with Et₂O, the aqueous layer is acidified to pH 2 and extracted with EtOAc. Combined organic layers are washed with brine, dried over MgSO₄ and evaporated. The product is obtained after crystallization from EtOAc-Et₂O as white solid: TLC (CH₂Cl₂MeOH 19:1) Rf=0.16; ESIMS [M-H]⁺=486; ¹H-NMR (400 MHz, CDCl₃): δ 7.95 (s, 1H), 7.44 (dd, J=7, 1 Hz, 1H), 7.1-7.4 (m, 6H), 4.56 (d, J=14Hz, 1H), 4.34 (d, J=14Hz, 1 H), 3.9 (m, 2H), 2.6-3.4 (m, 11H). 1.27 (d, J=7Hz, 6H).

### b) 2-Hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzoic acid

To a solution of 5-[(3aR,7S,7aS)-3-(3-isopropyl-benzyl)-2,5,5-trioxo-octahydro-1-oxa-5lambda*6*-thia-3-aza-inden-7-ylmethyl]-2-methoxy-benzoic acid (2.0 g, 4.1 mmol) in CH₂Cl₂ (70 mL) is added under Argon a 1 M solution of BBr₃ in CH₂Cl₂ (25 mL) at 0-5 °C and the reaction mixture is stirred at 0-5 °C for 1 h. The mixture is poured onto ice-water, MeOH (20 mL) added and after 10 min the layers are separated. After extraction with CH₂Cl₂, the combined layers are washed with water, dried over MgSO₄ and evaporated. The product is obtained after crystallization from CH₂Cl₂-Et₂O as light yellow crystals: TLC (CH₂Cl₂-MeOH-AcOH-H₂O 180:20:2:1) Rf=0.31; ESIMS [M-H]⁺=472; ¹H-NMR (400 MHz, DMSO-d₆): δ 7.58 (s, 1 H), 7.1-7.3 (m, 5H), 6.8 (d, J=7Hz, 1 H), 4.39 (d, J=14Hz, 1 H), 2.4-4.3 (m, 11 H), 1.22 (d, J=7H, 6H).

### c) N-Benzyl-N-ethyl-2-hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzamide

To a solution of 2-hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzoic acid (0.076 g, 0.16 mmol) in DMF (3 mL) is added N-ethyl-benzylamine (0.05 mL, 0.32 mmol), EDC (0.063 g, 0.32 mmol), HOAt (0.044 g, 0.32 mmol) and DIPEA (0.11 mL, 0.64 mmol) and the reaction mixture is heated at 50 °C for 16 h. After evaporation of the solvent, the residual oil is diluted in K₂CO₃-solution and extracted with EtOAc. Combined layers are washed with 1 N HCL and brine, dried over MgSO₄ and evaporated. The product is obtained after purification by flash-chromatography on silica gel (CH₂Cl₂-MeOH 97:3) as a colorless foam: TLC (CH₂Cl₂-MeOH 19:1) Rf=0.50; ESIMS [M+H]⁺=591; ¹H-NMR (400 MHz, CDCl₃)_{:} δ 7.43 (t, J=7Hz, 1 H), 7.41 (d, J=1Hz, 1H), 7.1-7.3 (m, 8H), 7.09 (s, 1 H), 7.02 (d, J=7Hz, 1 H), 4.75 (m, 2H), 4.53 (d, J=14Hz, 1 H), 4.33 (d, J=14Hz, 1 H), 3.80 (dt, J=3, 12Hz, 1 H), 3.5-3.7 (m, 3H), 2.4-3.15 (m, 8H), 1.31 (t, J=7Hz, 3H), 1.29 (d, J=7Hz, 6H).

### d) (3aR,7S,7aS)-7-{3-[(Benzyl-ethyl-amino)-methyl]-4-hydroxy-benzyl}-3-(3-isopropyl-benzyl)-5,5-dioxo-hexahydro-1-oxa-5lambda*6*-thia-3-aza-inden-2-one

To a solution of N-benzyl-N-ethyl-2-hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzamide (0.05 g, 0.085 mmol) in anhydrous THF (5 mL) is added under Argon a 1 M BH₃-solution in THF (0.3 mL) and the reaction mixture is stirred at 0-5°C for 3 h. Excess BH₃-THF is destroyed with MeOH and the reaction mixture is evaporated serveral times with MeOH. The product is obtained after purification by flash-chromatography on silica gel (CH₂Cl₂-MeOH 95:5) as a colorless foam: TLC (CH₂Cl₂MeOH 10:1) Rf=0.80; ESIMS [M+H]⁺=577.

### e) N-Benzyl-N-ethyl-2-hydroxy-5-[(3S,4S,5R)-4-hydroxy-5-(3-isopropyl-benzylamino)-1,1-dioxo-hexahydro-liambda*6*-thiopyran-3-ylmethyl]-benzamide hydrochloride

The title compound is prepared in analogous manner as described for example 15e, starting from (3aR,7S,7aS)-7-{3-[(benzyl-ethyl-amino)-methyl]-4-hydroxy-benzyl}-3-(3-isopropylbenzyl)-5,5-dioxo-hexahydro-1-oxa-5lambda*6*-thia-3-aza-inden-2-one and Ba(OH)₂ 8H₂O: TLC (CH₂Cl₂-MeOH 19:1) Rf=0.28; ESIMS [M+H]⁺=551.

### Example 23: N-Benzyl-N-butyl-2-hydroxy-5-[(3S,4S,5R)-4-hydroxy-5-(3-isopropyl-benzylamino)-1,1-dioxo-hexahydro-1lambda*6*-thiopyran-3-ylmethyl]-benzamide hydrochloride

The title compound is obtained by an analogous reaction sequence as for example 22, starting from 5-[(3aR,7S,7aS)-3-(3-isopropyl-benzyl)-2,5,5-trioxo-octahydro-1-oxa-5lambda*6*-thia-3-aza-inden-7-ylmethyl]-2-methoxy-benzoic acid and N-butyl-benzylamine: TLC (CH₂Cl₂-MeOH 19:1) Rf=0.42; ESIMS [M+H]⁺=579.

### Example 24: (3S,4S,5R)-3-(3-Hydroxymethyl-benzyl)-5-(3-isopropyl-benzylamino)-1,1-dioxo-hexahydro-1lambda*6*-thiopyran-4-ol hydrochloride

### a) 2-Hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzoic acid methyl ester

To a solution of 2-hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzoic acid (1.8 g, 3.8 mmol) in CH₂Cl₂-MeOH 4:1 (50 mL) is added a 2M solution of trimethylsilyl-diazomethane in hexane (3 mL) at 0-5 °C over a period of 1 h. After addition of a few drops of AcOH, the reaction mixture is evaporated. The product is dissolved in EtOAc, washed with NaHCO₃-solution and brine, dried over MgSO₄ and evaporated. The product is obtained as a yellow solid: TLC (hexane-EtOAc 1:1) Rf=0.32; ESIMS [M-H]⁺=486; ¹H-NMR (400 MHz, CDCl₃): δ 10.75 (s, 1H), 7.62 (s, 1H), 7.32 (t, J=7Hz, 1 H), 7.29 (d, J=7Hz, 1 H), 7.24 (d, J=7Hz, 1 H), 7.15 (s, 1H), 7.12 (d, J=7Hz, 1 H), 7.0 (d, J=7Hz, 1 H), 4.55 (d, J=14Hz, 1 H), 4.33 (d, J=14Hz, 1 H), 3.96 (s, 3H), 3.91 (dt, J=3, 12Hz, 1H). 3.79 (t, J=12Hz, 1H). 2.6-3.2 (m, 8H), 1.26 (d, J=7Hz, 6H).

### b) 5-[(3aR,7S,7aS)-3-(3-isopropyl-benzyl)-2,5,5-trioxo-octahydro-1-oxa-5lambda*6*-thia-3-aza-inden-7-ylmethyl]-2-trifluoromethanesulfonyloxy-benzoic acid methyl ester

To a solution of 2-hydroxy-5-[(3aR,7S,7aS)-3-(3-isopropyl-benzyl)-2,5,5-trioxo-octahydro-1-oxa-5lambda*6*-thia-3-aza-inden-7-ylmethyl]-benzoic acid methyl ester (1.66 g, 3.4 mmol) in CH₂Cl₂ (100 mL) is added under Argon NEt₃ (1.66 mL, 11.9 mmol) and trifluoromethanesulfonic acid anhydride (1.13 mL, 6.8 mmol) at -20°C and the reaction mixture is stirred at 0-5 °C for 0.5 h. The reaction mixture is poured onto cold 1 N HCl and is extracted with CH₂Cl₂. The combined organic layers are washed with cold NaHCO₃-solution and water, dried over MgSO₄ and evaporated. The product is obtained after purification by flash-chromatography on silica gel (hexane-EtOAc 3:1 to 1:1) as a white solid: TLC (hexane-EtOAc 3:1) Rf=0.32; ESIMS [M+H+NH3]⁺=637; ¹H-NMR (400 MHz, CDCl₃)_{:} δ 7.86 (s, 1H), 7.1-7.5 (m, 6H), 4.55 (d, J=14Hz, 1H), 4.35 (d, J=14Hz), 4.0 (s, 3H), 3.8 -4.0 (m,2H), 2.7-3.3 (m,8H), 1.27 (d, J=7Hz, 6H).

### c) 3-[(3aR,7S,7aS)-3-(3-Isopropyl-benzyl)-2,5,5-trioxo-octahydro-1-oxa-5lambda*6*-thia-3-aza-inden-7-ylmethyl]-benzoic acid methyl ester

To a solution of 5-[(3aR,7S,7aS)-3-(3-isopropyl-benzyl)-2,5,5-trioxo-octahydro-1-oxa-5lambda*6*-thia-3-aza-inden-7-ylmethyl]-2-trifluoromethanesulfonyloxy-benzoic acid methyl ester (1.88 g, 3.04 mmol) in DMF (50 mL) is added under Argon tributylamine (2.95 mL, 12.1 mmol), 1,3-bis-(phenylphosphino)-propane (0.19 g, 0.455 mmol), (PPh₃)₂PdCl₂ (0.13 g, 0.18 mmol) and formic acid (0.35 mL, 9.1 mmol) and the reaction mixture is heated in a sealed tube at 150°C for 20 min. The reaction mixture is evaporated and the residue is dissolved in EtOAc and washed with 0.1 N HCl, water and brine, dried over MgSO₄ and evaporated. The crude product is crystallized from EtOAc-Et₂O to give the product as colorless crystals: TLC (hexane-EtOAc 1:1) Rf=0.24; ESIMS [M+H+NH₃]⁺=489; ¹H-NMR (400 MHz, CDCl₃)_{:} δ 8.01 (d, J=7Hz, 1 H), 7.82 (s, 1 H), 7.45 (t, J=7Hz, 1 H), 7.4 (d, J=7Hz, 1 H), 7.33 (t, J=7Hz, 1 H), 7.23 (d, J=7Hz, 1 H), 7.15 (s, 1 H), 7.12 (d, J=7Hz, 1 H), 4.57 (d, J=14Hz, 1 H), 4.35 (d, J=14Hz), 3.92 (dt, J=4, 12Hz, 1 H), 3.84 (t, J=12Hz, 1 H), 2.7-3.3 (m, 8H), 1.25 (d, J=7Hz, 6H).

### d) (3aR,7S,7aS)-7-(3-Hydroxymethyl-benzyl)-3-(3-isopropyl-benzyl)-5,5-dioxo-hexa-hydro-1-oxa-5lambda*6*-thia-3-aza-inden-2-one

To a solution of 3-[(3aR,7S,7aS)-3-(3-isopropyl-benzyl)2,5,5-trioxo-octahydro-1-oxa-5lambda*6*-thia-3-aza-inden-7-ylmethyl]-benzoic acid methyl ester (0.212 g, 0.45 mmol) in THF (15 mL) is added under Argon LiBH₄ (0.030 g, 1.35 mmol) at 25°C. After stirring at 25°C for 16 h the reaction mixture is diluted with a 20%-KHSO₄-solution and extracted with EtOAc. Combined layers are washed with K₂CO₃-solution and brine, dried over MgSO₄ and evaporated. The crude product is crystallized from EtOAc-Et₂O to give the product as colorless crystals: TLC (EtOAc-AcOH 200:1) Rf=0.55; ESIMS [M+H+NH₃]⁺=461; ¹H-NMR (400 MHz, CDCl₃): δ 7.1-7.5 (m, 8H), 4.73 (s, 2H), 4.56 (d, J=14Hz, 1H), 4.34 (d, J=14Hz, 1 Hz), 3.92 (dt, J=4, 12Hz, 1 H), 3.83 (t, J=12Hz, 1 H), 2.6-3.4 (m, 9H), 1.26 (d, J=7Hz, 6H).

### e) (3S,4S,5R)-3-(3-Hydroxymethyl-benzyl)-5-(3-isopropyl-benzylamino)-1,1-dioxo-hexa-hydro-1lambda*6*-thiopyran-4-ol hydrochloride

The title compound is prepared in analogous manner as described for example 15e, starting from (3aR,7S,7aS)-7-(3-hydroxymethyl-benzyl)-3-(3-isopropyl-benzyl)-5,5-dioxo-hexahydro-1-oxa-5lambda*6*-thia-3-aza-inden-2-one and Ba(OH)₂ 8H₂O TLC (CH₂Cl₂-MeOH 19.1) Rf=0.31; ESIMS [M+H]⁺=418; ¹H-NMR (400 MHz, CDCl₃)_{:} δ 7.1-7.4 (m, 8H), 4.7 (s, 2H), 4.1 (br, 1H), 3.91 (d, J=12Hz, 1H), 3.78 (d, J=12Hz, 1H), 3.42 (dt, J=14, 4Hz, 1H), 3.31 (dd, J=12, 4Hz, 1 H), 3.12 (m, 2H), 2.94 (m, 2H), 2.7 (m, 3H), 2.42 (m, 1 H), 1.26 (d, J=7Hz, 6H).

### Example 25: (3R,4S,5S)-3-(3-isopropyl-benzylamino)-1,1-dioxo-5-(3-propoxymethylbenzyl)-hexahydro-1lambda*6*-thiopyran-4-ol hydrochloride

To a suspension of NaH (0.016 mg 50% in oil, 0.33 mmol) in DMF (1 mL) is added under Argon (3aR,7S,7aS)-7-(3-hydroxymethyl-benzyl)-3-(3-isopropyl-benzyl)-5,5-dioxo-hexahydro-1-oxa-5lambda*6*-thia-3-aza-inden-2-one (0.048 mg, 0.11 mmol) and n-propyl iodide (0.017 mL, 0.165 mmol) and the reaction mixture is stirred for 1 h at 25°C. The reaction mixture is acidified with 1 N HCl and evaporated to dryness. The crude (3aR,7S,7aS)-3-(3-Isopropylbenzyl)-5,5-dioxo-7-(3-propoxymethyl-benzyl)-hexahydro-1-oxa-5lambda*6*-thia-3-aza-inden-2-one is dissolved in dioxane-water 2:1 (1.5 mL) and after addition of (Ba(OH)2 8H2O (0.174 g, 0.55 mmol) heated in a microwave oven for 5 min at 160°C. The reaction mixture is diluted with EtOAc and filtered. The filtrate is washed with brine, dried over MgSO4 and evaporated. The product is obtained after purification by flash-chromatography on silica gel (hexane-CH2Cl2-MeOH 100:100:5 to 10:100:5) and hydrochloride salt formation as a beige solid: TLC (CH2Cl2-MeOH 19:1) Rf=0.51. ESIMS [M+H]⁺=460. ¹H-NMR (400 MHz, CDCl₃): δ 7.1-7.4 (m, 8H), 4.52 (s, 2H), 4.05 (br s, 1 H), 3.93 (d, J=12Hz, 1 H), 3.79 (d, J=12Hz, 1 H), 3.46 (t, J=7Hz, 2H), 3.42 (dt, J=14, 4Hz, 1 H), 3.29 (dd, J=12, 4Hz, 1 H), 3.16 (dt, J=4, 13 Hz, 1H), 3.12 (t, J=13Hz, 1H), 2.94 (m, 2H), 2.72 (m, 3H), 2.43 (m, 1H), 1.66 (m, 2H), 1.26 (d, J=7Hz, 6H), 0.97 (t, J=7Hz,1H).

### Example 26: (3R,4S,5S)-3-(3-Isopropyl-benzylamino)-5-[3-((S)-2-methoxy-propoxy-methyl)-benzyl]-1,1-dioxo-hexahydro-1lambda*6*-thiopyran-4-ol

### a) (3aR,7S,7aS)-3-(3-Isopropyl-benzyl)-7-[3-((S)-2-methoxy-propoxymethyl)-benzyl]-5,5-dioxo-hexahydro-1-oxa-5lambda*6*-thia-3-aza-inden-2-one

To a solution of (3aR,7S,7aS)-7-(3-hydroxymethyl-benzyl)-3-(3-isopropyl-benzyl)-5,5-dioxo-hexahydro-1-oxa-5lambda*6*-thia-3-aza-inden-2-one (0.08 g, 0.18 mmol) in CH₂Cl₂ (3 mL) is added under Argon a 1.5 M solution of benzotriazole-SOCl₂ in CH₂Cl₂ (0.15 mL). After stirring at 25 °C for 1.5 h, the reaction mixture is filtered and evaporated. The crude (3aR,7S,7aS)-7-(3-chloromethyl-benzyl)-3-(3-isopropyl-benzyl)-5,5-dioxo-hexahydro-1-oxa-5lambda*6*-thia-3-aza-inden-2-one dissolved in DMF (2 mL) is added under Argon to a mixture of (S)-2-methoxy-propan-1-ol (0.064 g, 0.7 mmol) and NaH (0.051 g 50% in oil, 1.05 mmol) in DMF (3 mL) and stirred at 25 °C for 16 h. The reaction mixture is evaporated and the residue is diluted with NaHCO₃-solution and extracted with EtOAc. Combined extracts are washed with brine, dried over MgSO₄ and evaporated. The product is obtained after purification by flash-chromatography on silica gel (hexane-CH₂Cl₂-MeOH 100:100:5 to 10:100:5) as a beige solid: TLC (CH₂Cl₂-MeOH 19:1) Rf=0.59; ESIMS [M+H]⁺=533.

### b) (3R,4S,5S)-3-(3-lsopropyl-benzylamino)-5-[3-((S)-2-methoxy-propoxymethyl)-benzyl]-1,1-dioxo-hexahydro-1lambda*6*-thiopyran-4-ol

The title compound is prepared in analogous manner as described for example 15e starting from (3aR,7S,7aS)-3-(3-isopropyl-benzyl)-7-[3-((S)-2-methoxy-propoxymethyl)-benzyl]-5,5-dioxo-hexahydro-1-oxa-5lambda*6*-thia-3-aza-inden-2-one and Ba(OH)₂ 8H₂O: TLC (CH₂Cl₂-MeOH 19:1) Rf=0.35; ESIMS [M+H]⁺=490.

### Example 27: (3S,4S,5R)-3-(3-Bromo-4-hydroxy-benzyl)-5-(3-ethoxy-benzylamino)-1,1-dioxo-hexahydro-1lambda*6*-thiopyran-4-ol trifluoroacetate

### a) (3R,4S,5S)-3-Amino-5-(3-bromo-4-hydroxy-benzyl)-1,1-dioxo-hexahydro-1lambda*6*-thiopyran-4-ol

To a solution of (3R,4S,5S)-3-amino-5-(3-bromo-4-methoxy-benzyl)-1,1-dioxo-hexahydro-1lambda*6*thiopyran-4-ol (4.73 g, 13 mmol) in CH₂Cl₂ (200 mL) is added under Argon a 1M solution of BBr₃ in CH₂CL₂ (160 mL) at 25°C. The reaction mixture is stirred at 25 °C for 1.5 h and then-slowly poured onto cold K₂CO₃-solution. After stirring at 25°C for 0.5 h, ACN (200 mL), NEt₃ (2.8 mL, 20 mmol) and (Boc)₂O (4.45 g, 20 mmol) is added and stirring is continued for 16 h. The organic solvents are evaporated and the aqueous phase is extracted with EtOAc. Combined extracts are washed with brine, dried over MgSO₄ and evaporated. The solid di-Boc derivative is cleaved with 5-6N HCl in iPrOH (50 mL) and CH₂Cl₂ (20 mL) at 25 °C for 16 h and 6 h at 40-50 °C. After addition of Et₂O the crystallized hydrochloride salt is filtered off and dried: TLC (CH₂Cl₂MeOH-AcOH-H₂O 180:20:2:1) Rf=0.08; ESIMS [M+H] ⁺=350, 352; ¹H-NMR (400 MHz, DMSO-d₆): δ 10.2 (s, 1H), 8.42 (s, 2H), 7.35 (s, 1H), 7.03 (dd, J=7, 1 Hz, 1 H), 6.94 (d, J=7Hz, 1 H), 6.0 (d, J=7Hz, 1 H), 3.3-3.6 (m, 4H), 3.19 (dd, J=12, 14Hz, 1 H), 3.06 (dd, J=12, 2Hz, 1 H), 2.82 (d, J=12Hz, 1 H), 2.45 (dd, J=12, 8Hz, 1 H), 2.14 (m, 1H).

### b) (3S,4S,5R)-3-(3-Bromo-4-hydroxy-benzyl)-5-(3-ethoxy-benzylamino)-1,1-dioxo-hexahydro-1lambda*6*-thiopyran-4-ol

A mixture of the (3R,4S,5S)-3-amino-5-(3-bromo-4-hydroxy-benzyl)-1,1-dioxo-hexahydro-1lambda*6*-thiopyran-4-ol hydrochloride (0.015 g, 0.039 mmol), NaOAc (0.007 g, 0.089 mmol) and 3-ethoxy-benzaldehyde (0.0065 g, 0.04 mmol) is dissolved in CH₂Cl₂-MeOH 2:1 (1 mL) and heated at 50°C for 0.5 h before the addition of NaBH₃CN (0.013 g, 0.2 mmol) at 25°C. The reaction mixture is stirred at 25 °C for 2 h. The reaction is quenched with brine and the product is extracted with EtOAc. The title compound is obtained in pure form after preparative HPLC purification (10% ACN to 70% ACN in 10min, flow rate of 30ml/min., C₁₈ 5um 100x19mm) as its TFA salt: TLC (CH₂Cl₂-MeOH 19:1) Ref=0.31; ESIMS [M+H]⁺=484, 486.

**Examples 28 to 42:** All compounds of the formula I in Table 1 can be prepared using a procedure analogous to that used in Example 27b.

**Table 1**

| | | **m/z** |
|---|---|---|
| **Example** | **Compound of the formula I** | **[M+H]⁺** |
| 28 | (3S,4S,5R)-3-(3-Bromo-4-hydroxy-benzyl)-1,1-dioxo-5-(2-trifluoromethyl-benzylamino)-hexahydro-1lambda*6*-thiopyran-4-ol trifluoro- | 508 and 510 |
| 29 | acetate (3S,4S,5R)-3-(3-Bromo-4-hydroxy-benzyl)-5-(3,5-dimethyl-benzylamino)-1,1-dioxo-hexahydro-1lambda*6*-thiopyran-4-ol trifluoro-acetate | 468 and 470 |
| 30 | (3S,4S,5R)-3-(3-Bromo-4-hydroxy-benzyl)5-(3,5-dimethoxy-benzylamino)-1,1-dioxo-hexahydro-1lambda*6*-thiopyran-4-ol trifluoro-acetate | 500 and 502 |
| 31 | (4S,5R)-3-(3-Bromo-4-hydroxy-benzyl)-5-(3-methoxy-benzylamino)-1,1-dioxo-hexahydro-1lambda*6*-thiopyran-4-ol trifluoroacetate | 470 and 472 |
| 32 | (3S,4S,5R)-3-(3-Bromo-4-hydroxy-benzyl)-5-(3-isopropoxy-benzylamino)-1,1-dioxo-hexahydro-1lambda*6*-thiopyran-4-ol trifluoroacetate | 498 and 500 |
| 33 | (3S,4S,5R)-3-(3-Bromo-4-hydroxy-benzyl)-1,1-dioxo-5-(3-trifluoromethoxy-benzylamino)-hexahydro-1lambda*6*-thiopyran-4-ol trifluoro-acetate | 524 and 526 |
| 34 | (3S,4S,5R)-3-(3-Bromo-4-hydroxy-benzyl)5-(3-chloro-benzylamino)-1,1-dioxo-hexahydro-1lambda*6*-thiopyran-4-ol trifluoroacetate | 474, 476 and 478 |
| 35 | (3S,4S,5R)-3-(3-Bromo-4-hydroxy-benzyl)-5-(3-methyl-benzylamino)-1,1-dioxo-hexahydro-1lambda*6*-thiopyran-4-ol trifluoroacetate | 454 and 456 |
| 36 | (3S,4S,5R)-3-(3-Bromo-4-hydroxy-benzyl)-1,1-dioxo-5-(3-propoxy-benzylamino)-hexahydro-1lambda*6*-thiopyran-4-ol trifluoroacetate | 498 and 500 |
| 37 | 1-(3-{[(3R,4S,5S)-5-(3-Bromo-4-hydroxy-benzyl)-4-hydroxy-1,1-dioxo-hexahydro-1lambda*6*-thiopyran-3-ylamino]-methyl}-phenyl)-ethanone trifluoroacetate | 482 and 484 |
| 38 | (3R,4S,5S)-3-(5-Bromo-2-fluoro-benzylamino)-5-(3-bromo-4-hydroxy-benzyl)-1,1-dioxo-hexahydro-1lambda*6*-thiopyran-4-ol trifluoro- | 535, 537 and 539 |
| 39 | acetate (4S,5R)-3-(3-Bromo-4-hydroxy-benzyl)-5-(5-bromo-2-hydroxy-benzylamino)-1,1-dioxo-hexahydro-1lambda*6*-thiopyran-4-ol trifluoro-acetate | 534, 536 and 538 |
| 40 | (3R,4S,5S)-3-(3-Bromo-4-fluoro-benzylamino)-5-(3-bromo-4-hydroxy-benzyl)-1,1-dioxo-hexahydro-1lambda*6*-thiopyran-4-ol trifluoro-acetate | 535, 537 and 539 |
| 41 | (3S,4S,5R)-3-(3-Bromo-4-hydroxy-benzyl)-5-[(5-ethyl-furan-2-ylmethyl)-amino]-1,1-dioxo-hexahydro-1lambda*6*-thiopyran-4-ol trifluoro-acetate | 458 and 460 |
| 42 | (3S,4S,5R)-3-(3-Bromo-4-hydroxy-benzyl)-5-[(5-bromo-thiophen-2-ylmethyl)-amino]-1,1 dioxo-hexahydro-1lambda*6*-thiopyran-4-ol trifluoroacetate | 523, 525 and 527 |

### Example 43: (S)-4-{2-Hydroxy-5-[(3S,4S,5R)-4-hydroxy-5-(3-isopropyl-benzylamino)-1,1-dioxo-hexahydro-1lambda*6*-thiopyran-3-ylmethyl]-benzyl}-3-propyl-oxazolidin-2-one hydrochloride

### a) (Z)-2-Benzyloxycarbonylamino-3-(5-bromo-2-methoxy-phenyl)-acrylic acid methyl ester

To a suspension of benzyloxycarbonylamino-(dimethoxy-phosphoryl)-acetic acid methyl ester (12.2 g, 36.8 mmol) in CH₂Cl₂ (200 mL) is added DBU (8.4 mL, 55.2 mmol) at 25°C.

After stirring of the reaction mixture for 15 min 5-bromo-2-methoxy-benzaldehyde (8.0 g, 36.8 mmol) is added and the reaction mixture is stirred at 25 °C for 4 h. The solvent is evaporated and the residual oil is dissolved in EtOAc and 0.5N HCl-solution. After extraction with EtOAc, the combined organic layers are washed with 0.1 N HCl, water and brine, dried over MgSO₄ and evaporated. The crude product is recrystallized from EtOAc-Et₂O to give the title compound as white crystals: TLC (hexanes/EtOAc 2:1) Rf=0.52; ESIMS [M+H+NH3]⁺=437, 439.

### b) (S)-2-Benzyloxycarbonylamino-3-(5-bromo-2-methoxy-phenyl)-propionic acid methyl ester

To a solution of (Z)-2-benzyloxycarbonylamino-3-(5-bromo-2-methoxy-phenyl)-acrylic acid methyl ester (5.7 g, 13.5 mmol) in MeOH (200 mL) is added (+)-1,2-bis((2S,5S)-2,5-diethylphospholano)benzene(cyclooctadiene)rhodium(I)tetrafluoroborate (0.047 g, 0.067 mmol) and the resulting reaction mixture is hydrogenated at 5 atm H₂ at 25
°C for 3 days. After evaporation of the solvent, the crude product is crystallized from EtOAc-Et₂O-hexan to give the title compound as white needles: TLC (hexanes/EtOAc 1:1) Rf=0.61; ESIMS [M+H]⁺=422, 424; ¹H-NMR (400 MHz, CDCl₃): δ 6.7-7.3 (m, 8H), 5.72 and 5.35 (d, 1H), 5.12 (m, 2H), 4.54 and 4.45 (m, 1H), 3.76 and 3.70 (s, 3H), 3.34 (s, 3H), 3.08 (dd, J=14, 4Hz, 1 H), 2.94 (dd, J=14, 7Hz, 1H).

### c) [(S)-2-(5-Bromo-2-methoxy-phenyl)-1-hydroxymethyl-ethyl]-carbamic acid benzyl ester

To a solution of (S)-2-benzyloxycarbonylamino-3-(5-bromo-2-methoxy-phenyl)-propionic acid methyl ester (3.89 g, 9.2 mmol) in THF (150 mL) is added LiBH₄ (0.423 g, 18.4 mmol) in portions over a period of 1 h. After stirring the reaction mixture at 25°C for 3 h, MeOH (100 mL) is carefully added and the solution is evaporated. The residue is taken up in cold 0.5 N HCl and extracted with EtOAc. Combined organic layers are washed with brine, dried over MgSO₄ and evaporated. The crude product is crystallized from Et₂O to give the title compound as white crystals: TLC (EtOAc) Rf=0.62; ESIMS [M+H]⁺=394, 396; ¹H-NMR (400 MHz, CDCl₃): δ 7.36 (m, 7H), 6.78 (d, J=7Hz, 1 H), 5.27 (br, 1 H), 5.14 (m, 2H), 3.5-3.9 (m, 6H), 2.88 (m, 2H), 2.53 (br, 1 H).

### d) (S)-4-(5-Bromo-2-methoxy-benzyl)-oxazolidin-2-one

To an oil-free suspension of NaH (0.722 g 50% in oil, 15 mmol) in THF (150 mL) is added a solution of [(S)-2-(5-bromo-2-methoxy-phenyl)-1-hydroxymethyl-ethyl]-carbamic acid benzyl ester (4.53 g, 11.5 mmol) in THF (30 mL). The reaction mixture is stirred at 25°C for 4 h and 4 h at 45-50 °C, then poured onto ice-water and 20% aqueous KHSO₄-solution and extracted with EtOAc. Combined extracts are washed with brine, dried over MgSO₄ and evaporated. The product is obtained after flash-chromatography on silica gel (hexane-EtOAc 3:1 to 1:3) as a colorless oil: TLC (hexane-EtOAc 1:2) Rf=0.28; ESIMS [M+H+NH3]⁺=303, 305; ¹H-NMR (400 MHz, CDCl₃): δ 7.39 (dd, J=1, 7Hz, 1H), 7.26 (d, J=1Hz, 1H), 6.70 (d, J=7Hz, 1H), 5.34 (br, 1 H), 4.49 (m, 1H), 4.18 (m, 2H), 3.85 (s, 3H), 2.94 (dd, J=4,14Hz, 1 H), 2.85 (dd, J=7, 14Hz, 1 H).

### e1) (S)-4-(5-Bromo-2-methoxy-benzyl)-3-propyl-oxazolidin-2-one

To an oil-free suspension of NaH (0.46 g 50% in oil, 9.5 mmol) in DMF (30 mL) is added a solution of (S)-4-(5-bromo-2-methoxy-benzyl)-oxazolidin-2-one (1.8 g, 6.3 mmol) in DMF (10 mL) and the reaction mixture is stirred at 25°C for 0.5 h. n-propyl bromide (0.95 mL, 9.5 mmol) is added and stirring is continued at 25°C for 6 h. The reaction mixture is diluted with water and the product is extracted with EtOAc. Combined extracts are washed with brine, dried over MgSO₄ and evaporated to give the title compound as a colorless oil: TLC (toluene-EtOAc 1:1) Rf=0.52; ESIMS [M+H+NH₃]⁺=345, 347; ¹H-NMR (400 MHz, CDCl₃): δ 7.39 (dd, J=1, 7Hz, 1 H), 7.25 (d, J=1Hz, 1H), 6.79 (d, J=7Hz, 1 H), 4.18 (m, 1 H), 4.04 (m, 2H), 3.85 (s, 3H), 3.53 (m, 1 H), 3.20 (dd, J=4,14Hz, 1 H), 3.12 (m, 1 H), 2.59 (dd, J=9, 14Hz, 1 H), 1.64 (m, 2H), 0.9.8 (t, J=7Hz, 3H).

### e2) 4-Methoxy-3-((S)-2-oxo-3-propyl-oxazolidin-4-ylmethyl)-benzonitrile

To a degassed solution of (S)-4-(5-bromo-2-methoxy-benzyl)-3-propyl-oxazolidin-2-one (1.97 g, 6 mmol) in DMF (20 mL) is added under Argon the Zn(CN)₂ (0.96 g, 7.9 mmol) and Pd(PPh₃)₄ (0.15 g, 0.123 mmol). The reaction mixture is heated in a microwave oven at 160 °C for 30 min. After dilution with EtOAc, the suspension is filtered over Celite and evaporated. The product is obtained after flash-chromatography on silica gel (hexane-EtOAc 2:1 to 1:4) as a colorless oil: TLC (toluene-EtOAc 1:1) Rf=0.35; ESIMS [M+H+NH₃]⁺=292; ¹H-NMR (400 MHz; CDCl₃): δ 7.63 (dd, J=1, 7Hz, 1 H), 7.43 (d, J=1Hz, 1 H), 6.98 (d, J=7Hz, 1 H), 4.0-4.2 (m, 3H), 3.95 (s, 3H), 3.53 (m, 1 H), 3.23 (dd, J=4, 14Hz, 1H), 3.14 (m, 1H), 2.63 (dd, J=9, 14Hz, 1 H), 1.65 (m, 2H), 0.98 (t, J=7Hz, 3H):

### f) 4-Methoxy-3-((S)-2-oxo-3-propyl-oxazolidin-4-ylmethyl)-benzaldehyde

To a solution of 4-methoxy-3-((S)-2-oxo-3-propyl-oxazolidin-4-ylmethyl)-benzonitrile (2.9 g, 10.3 mmol) in 80% formic acid (60 mL) is added under Argon at 60-70 °C PtO₂ (1.2 g) in portions over a period of 24 h. After stirring the reaction mixture at 60-70 °C for 32 h, the catalyst is filtered off over Celite and the colorless filtrate is evaporated. The residual oil is stirred in 1 N H₂SO₄ for 2 h and the product is extracted with EtOAc. Combined layers are washed with NaHCO₃-solution and brine, dried over MgSO₄, and evaporated. The product is obtained after flash-chromatography on silica gel (hexane-EtOAc 2:1 to 1:2) as a colorless oil: TLC (EtOAc) Rf=0.49; ESIMS [M+H+NH₃]⁺=295; ¹H-NMR (400 MHz, CDCl₃): δ 9.9 (s, 1 H), 7.83 (dd, J=1, 7Hz, 1H), 7.69 (d, J=1Hz, 1 H), 7.04 (d, J=7Hz, 1 H), 4.0-4.2 (m, 3H), 3.97 (s, 3H), 3.52 (m, 1 H), 3.25 (dd, J=4, 14Hz, 1 H), 3.14 (m, 1 H), 2.69 (dd, J=9,14Hz, 1 H), 1.65 (m, 2H), 0.98 (t, J=7Hz, 3H).

### g) 4-Hydroxy-5-[1-[4-methoxy-3-((S)-2-oxo-3-propyl-oxazolidin-4-ylmethyl)-phenyl]-meth-(Z)-ylidene]-5,6-dihydro-2H-thiopyran-3-carboxylic acid methyl ester

To a solution of 4-methoxy-3-((S)-2-oxo-3-propyl-oxazolidin-4-ylmethyl)-benzaldehyde (1.96 g, 7.0 mmol) 4-oxo-tetrahydro-thiopyran-3-carboxylic acid methyl ester (1.49 g, 8.4 mmol) and AcOH (0.51 g, 8.4 mmol) in EtOH (8 mL) and water (0.4 mL) is added pyrrolidine (0.41 g, 5.6 mmol) and the resulting reaction mixture is heated at reflux for 3 days. The reaction mixture is evaporated and the residue is taken up in 5% NaHCO₃-solution and the product is extracted with EtOAc. Combined layers are washed with 1N HCl and brine, dried over MgSO₄, and evaporated. The product is obtained after flash-chromatography on silica gel (hexane-EtOAc 6:1 to EtOAc) as a yellow foam: TLC (hexane-EtOAc 1:1) Rf=0.39; ESIMS [M+H+NH₃]⁺=451; ¹H-NMR (400 MHz, CDCl₃): δ 12.7 (s, 1 H), 7.45 (s, 1 H), 7.23 (dd, J=1, 7Hz, 1 H), 7.07 (d, J=1 Hz, 1 H), 6.88 (d, J=7Hz, 1 H), 4.0-4.2 (m, 3H), 3.86 (s, 3H), 3.83 (s, 3H), 3.65 (s, 2H), 3.54 (s, 2H), 3.51 (m, 1 H), 3.22 (dd, J=4, 14Hz, 1 H), 3.11 (m, 1 H), 2.60 (dd, J=9, 14Hz, 1H), 1.64 (m, 2H), 0.98 (t, J=7Hz, 3H).

### h) 4-Hydroxy-5-[1-[4-methoxy-3-((S)-2-oxo-3-propyl-oxazolidin-4-ylmethyl)-phenyl]-meth-(Z)-ylidene]-1,1-dioxo-1,2,5,6-tetrahydro-1lambda*6*-thiopyran-3-carboxylic acid methyl ester

To a solution of 4-hydroxy-5-[1-[4-methoxy-3-((S)-2-oxo-3-propyl-oxazolidin-4-ylmethyl)-phenyl]-meth-(Z)-ylidene]-5,6-dihydro-2H-thiopyran-3-carboxylic acid methyl ester (2.17 g, 5 mmol) in THF (40 mL) is added water (10 mL) and oxone (6.7 g, 10.5 mmol) in portions over a period of 10 min. The reaction mixture is stirred at 25 °C for 1 h. Excess oxone is destroyed with solid Na₂S₂O₅ and the reaction mixture is extracted with EtOAc. Combined organic layers are washed with brine and dried over MgSO₄. The product is obtained after flash-chromatography on silica gel (hexane-EtOAc 2:1 to EtOAc) as a yellow foam: TLC (toluene-EtOAc 1:1) Rf=0.35; ESIMS [M+H+NH₃]=483; ¹H-NMR (400 MHz, CDCl₃): δ 13.1 (s, 1 H), 7.84 (s, 1 H), 7.25 (dd, J=1, 7Hz, 1 H), 7.11 (d, J=1Hz, 1 H), 6.94 (d, J=7Hz, 1 H), 3.8-4.2 (m, 7H), 3.92 (s, 3H), 3.88 (s, 3H), 3.54 (m, 1 H), 3.24 (dd, J=4, 14Hz, 1 H), 3.13 (m, 1 H), 2.64 (dd, J=9, 14Hz, 1 H), 1.67 (m, 2H), 0.98 (t, J=7Hz, 3H).

### i) 4-Hydroxy-5-[4-methoxy-3-((S)-2-oxo-3-propyl-oxazolidin-4-ylmethyl)-benzyl]-1,1-dioxo-1,2,5,6-tetrahydro-1lambda*6*-thiopyran-3-carboxylic acid methyl ester

4-Hydroxy-5-[1-[4-methoxy-3-((S)-2-oxo-3-propyl-oxazolidin-4-ylmethyl)-phenyl]-meth-(Z)-ylidene]-1,1-dioxo-1,2,5,6-tetrahydro-1lambda*6*-thiopyran-3-carboxylic acid methyl ester (1.68 g, 3.6 mmol) is hydrogenated (6 atm H₂) in MeOH (50 mL) with PtO₂ as catalyst (0.1 g) at 50°C for 3 days. The catalyst is filtered off and the filtrate is evaporated to give the product as colorless foam: TLC (toluene/EtOAc 1:1) Rf=0.33; ESIMS [M+H+NH₃]⁺=485.

### j) 4-Hydroxy-5-[4-methoxy-3-((S)-2-oxo-3-propyl-oxazolidin-4-ylmethyl)-benzyl]-1,1-dioxo-hexahydro-1lambda*6*-thiopyran-3-carboxylic acid methyl ester

To a suspension of 4-hydroxy-5-[4-methoxy-3-((S)-2-oxo-3-propyl-oxazolidin-4-ylmethyl)-benzyl]-1,1-dioxo-1,2,5,6-tetrahydro-1lambda*6*-thiopyran-3-carboxylic acid methyl ester (1.42 g, 3 mmol) in iPrOH-AcOH 4:1 (15 mL) is added NaBH₃CN (0.26 g, 3.9 mmol) and the reaction mixture is stirred at 25°C for 2 h. The solvents are evaporated and the residue is redissolved in EtOAc and basified with saturated NaHCO₃-SOlution. After extraction with EtOAc, the combined organic layers are washed with brine and dried over MgSO₄. Evaporation of the solvents gives the product as a white foam as a mixture of diastereoisomers which is used in the next step without further purification: TLC (EtOAc) Rf=0:49, 0.46 and 0.40; ESIMS [M+H+NH₃]⁺=487.

### k) 4-Hydroxy-5-[4-methoxy-3-((S)-2-oxo-3-propyl-oxazolidin-4-ylmethyl)-benzyl]-1,1-dioxo-hexahydro-1lambda*6*-thiopyran-3-carboxylic acid

To a solution 4-hydroxy-5-[4-methoxy-3-((S)-2-oxo-3-propyl-oxazolidin-4-ylmethyl)-benzyl]-1,1-dioxo-hexahydro-1lambda*6*-thiopyran-3-carboxylic acid methyl ester (1.35 g, 2.8 mmol) in dioxane (10 mL) is added 4N NaOH (5 mL) and the reaction mixture is stirred at 25°C for 1 h. The reaction mixture is acidified with 4N HCl at 15-20 °C and extracted with EtOAc Combined organic layers are washed with brine, dried over MgSO₄, filtered and evaporated. The product obtained as a mixture of diastereoisomers is a white foam which is used in the next step without further purification: ESIMS [M-H]⁻=454.

### l) 7-[4-Methoxy-3-((S)-2-oxo-3-propyl-oxazolidin-4-ylmethyl)-benzyl]-5,5-dioxo-hexahydro-1-oxa-5lambda*6*-thia-3-aza-inden-2-one

To a solution of 4-hydroxy-5-[4-methoxy-3-((S)-2-oxo-3-propyl-oxazolidin-4-ylmethyl)-benzyl]-1,1-dioxo-hexahydro-1lambda*6*-thiopyran-3-carboxylic acid (1.2 g, 2.6 mmol) in 1,2-dichlorethane (20 mL) is added NEt₃ (0.51 mL, 3.6 mmol) and DPPA (0.90 g, 3.1 mmol) and the reaction mixture is stirred at 25°C for 4 h, then heated at 60 °C for 1 h followed by 8 h at reflux. The reaction mixture is evaporated and the product is obtained after purification by flash-chromatography on silica-gel (hexane-EtOAc 1:1 to EtOAc) as a yellow foam: TLC (EtOAc) 0.54, 0.43 and 0.20 (mixture of diastereoisomers); ESIMS [M+H+NH₃]⁺=470.

### m) 7-[4-Methoxy-3-((S)-2-oxo-3-propyl-oxazolidin-4-ylmethyl)-benzyl]-2,5,5-trioxo-hexahydro-1-oxa-5lambda*6*-thia-3-aza-indene-3-carboxylic acid tert-butyl ester

To a solution of 7-[4-methoxy-3-((S)-2-oxo-3-propyl-oxazolidin-4-ylmethyl)-benzyl]-5,5-dioxo-hexahydro-1-oxa-5lambda*6*-thia-3-aza-inden-2-one (0.62 g, 1.36 mmol) in ACN (20 mL) is added NEt₃ (0.3 mL, 1.55 mmol), (BOC)₂O (0.35 g, 1.5 mmol) and a catalytic amount of DMAP (0.005 g, 0.04 mmol). The reaction mixture is heated at 45-50 °C for 2 h. The reaction mixture is evaporated and the residual oil is purified by flash-chromatography on silica-gel (hexane-EtOAc 5:1 to EtOAc). The product, a mixture of diastereoisomers, is obtained as a yellow foam: TLC (hexane-EtOAc 1:1) Rf=0.16 and 0.18; ESIMS [M+H+NH₃]⁺=570.

### n) {4-Hydroxy-5-[4-methoxy-3-((S)-2-oxo-3-propyl-oxazolidin-4-ylmethyl)-benzyl]-1,1-dioxo-hexahydro-1lambda*6*-thiopyran-3-yl}-carbamic acid tert-butyl ester

To a solution of 7-[4-methoxy-3-((S)-2-oxo-3-propyl-oxazolidin-4-ylmethyl)-benzyl]-2,5,5-trioxo-hexahydro-1-oxa-5lambda*6*-thia-3-aza-indene-3-carboxylic acid tert-butyl ester (0.74 g, 1.3 mmol) in MeOH (25 mL) is added Cs₂CO₃ (0.22 g, 0.65 mmol) and the reaction mixture is stirred at 25 °C for 16 h. The solvent is evaporated and the product is redissolved in EtOAc and washed with water and brine, dried over MgSO₄ and evaporated. The title compound is obtained as a light yellow foam: TLC (EtOAc) Rf=0.50; ESIMS [M+H]⁺=527.

### o) {(3R*,5S*)-5-[4-Methoxy-3-((S)-2-oxo-3-propyl-oxazolidin-4-ylmethyl)-benzyl]-1,1,4-trioxo-hexahydro-1lambda*6*-thiopyran-3-yl}-carbamic acid tert-butyl ester

To a solution of {4-hydroxy-5-[4-methoxy-3-((S)-2-oxo-3-propyl-oxazolidin-4-ylmethyl)-benzyl]-1,1-dioxo-hexahydro-1lambda*6*-thiopyran-3-yl}-carbamic acid tert-butyl ester (0.53 g, 1 mmol) in CH₂Cl₂ (50 mL) is added Dess-Martin reagent (0.53 g, 1.2 mmol) and the reaction mixture is stirred at 25 °C for 2 h. To the reaction mixture is added saturated NaHCO₃-solution and NaS₂O₃-solution and after stirring for 1 h the product was extracted with CH₂Cl₂. Combined extracts are washed with water, dried over MgSO₄, and evaporated. The crude product is purified by flash-chromatography on silica-gel (hexane-EtOAc 3:1 to EtOAc). The product, a 1:1 mixture of diastereoisomers, is obtained as a yellow foam: TLC (hexane-EtOAc 1:1) Rf=0.49; ESIMS [M+H]⁺=525.

### p) {(3R,4S,5S)-4-Hydroxy-5-[4-methoxy-3-((S)-2-oxo-3-propyl-oxazolidin-4-ylmethyl)-benzyl]-1,1-dioxo-hexahydro-1lambda*6*-thiopyran-3-yl}-carbamic acid tert-butyl ester

### and {(3S,4R,5R)-4-Hydroxy-5-[4-methoxy-3-((S)-2-oxo-3-propyl-oxazolidin-4-ylmethyl)-benzyl]-1,1-dioxo-hexahydro-1lambda*6*-thiopyran-3-yl}-carbamic acid tert-butyl ester

To a solution of {(3R*,5S*)-5-[4-methoxy-3-((S)-2-oxo-3-propyl-oxazolidin-4-ylmethyl)-benzyl]-1,1,4-trioxo-hexahydro-1lambda*6*-thiopyran-3-yl}-carbamic acid tert-butyl ester (0.446 g, 0.85 mmol) in anhydrous THF (20 mL) is added under Argon LiAlH₄ (0.022 g, 0.52 mmol) at 0-5 °C and the reaction mixture is stirred for 30 min. After addition of water (0.03 mL), 4N NaOH (0.05 mL) and water (0.15 mL), the white precipitate is filtered off over Celite and the solvent is evaporated. The diastereoisomers are separated by flash-chromatography on silica-gel (hexane-CH₂Cl₂-MeOH 100:100:5 to 20:100:5) to yield the two minor (Rf=0.41 and 0.24) and the two major diastereoisomers (Rf=0.33 and 0.28) as colorless foams: **{(3R,4S,5S)-diastereoisomer:** TLC (CH₂Cl₂-MeOH 19:1) Rf=0.33; ESIMS [M+H]⁺=527; ¹H-NMR (400 MHz, CDCl₃): δ 7.05 (dd, J=1, 7Hz, 1 H), 6.97 (d, J=1Hz, 1 H), 6.83 (d, J=7Hz, 1 H), 5.22 (br, 1 H), 3.8-4.2 (m, 6H), 3.83 (s, 3H), 3.63 (m, 1 H), 2.4-3.4 (m, 10H), 1.68 (m, 2H), 1.44 (s, 9H), 1.02 (t, J=7Hz, 3H).
**{(3S,4R,5R)-diastereoisomer:** TLC (CH₂Cl₂-MeOH 19:1) Rf=0.28; ESIMS[M+H]=527; ¹H-NMR (400 MHz, CDCl₃) δ 6.95 (m, 2H), 6.94 (d, J=7Hz, 1H), 6.13 (br, 1H), 4.0-4.2 (m, 6H), 3.85 (s, 3H), 2.4-3.5 (m, 11 H), 1.63 (m, 2H), 1.45 (s, 9H), 0.96 (t, J=7Hz, 3H).

### q) (S)-4-[5-((3S,4S,5R)-5-Amino-4-hydroxy-1,1-dioxo-hexahydro-1lambda*6*-thiopyran-3-ylmethyl)-2-methoxy-benzyl]-3-propyl-oxazolidin-2-one

{(3R,4S,5S)-4-hydroxy-5-[4-methoxy-3-((S)-2-oxo-3-propyl-oxazolidin-4-ylmethyl)-benzyl]-1,1-dioxo-hexahydro-1lambda*6*-thiopyran-3-yl}-carbamic acid tert-butyl ester (0.158 g, 0.3 mmol) is dissolved in 4N HCl in dioxane (2 mL) and stirred at 25 °C for 1 h. The solvent is evaporated and the residue is dried to give the hydrochloride salt as a beige solid: TLC (CH₂Cl₂-MeOH-AcOH-H₂O 180:20:2:1) Rf=0.15; ESIMS [M+H]⁺=427; ¹H-NMR (400 MHz, CD₃OD): δ 7.14 (dd, J=1, 7Hz, 1 H), 7.06 (d, J=1Hz, 1 H), 7.01 (d, J=7 Hz, 1 H), 4.1-4.3 (m, 3H), 3.87 (s, 3H), 3.1-3.6 (m, 9H), 3.05 (t, J=12Hz, 1 H), 2.88 (m, 1 H), 2.74 (m, 1 H), 2.59 (dd, J=8, 12Hz, 1 H), 1.63 (m, 2H), 0.96 (t, J=7Hz, 3H).

### r) (S)-4-{5-[(3S,4S,5R)-4-Hydroxy-5-(3-isopropyl-benzylamino)-1,1-dioxo-hexahydro-1lambda*6*-thiopyran-3-ylmethyl]-2-methoxy-benzyl}-3-propyl-oxazolidin-2-one hydrochloride

To a solution of (S)-4-[5-((3S,4S,5R)-5-amino-4-hydroxy-1,1-dioxo-hexahydro-1lambda*6*-thiopyran-3-ylmethyl)-2-methoxy-benzyl]-3-propyl-oxazolidin-2-one (0.127 g, 0.295 mmol) in MeOH- CH₂Cl₂ 1:1 (6 mL) is added sodium acetate (0.03 g, 0.44 mmol) and 3-isopropyl-benzaldehyde (0.049 g, 0.325 mmol). The reaction mixture is stirred at 25 °C for 30 min. before NaBH₃CN (0.029 g, 0.44 mmol) is added followed by stirring at 25°C for 16 h. The reaction mixture is acidified with 1 N HCl, stirred for 15 min, basified with K₂CO₃-solution and extracted with EtOAc. The combined organic layers are washed with brine, dried over MgSO₄ and evaporated. The pure product is obtained after purification by flash-chromatography on silica gel (hexane- CH₂Cl₂-MeOH 100:100:5 to 10:100:5) as a colorless foam: TLC (CH₂Cl₂-MeOH 19:1) Rf=0.30; ESIMS [M+H]⁺=559; ¹H-NMR (400 MHz, CD₃OD): δ 7.48 (s, 1 H), 7.35-7.45 (m, 3H), 7.16 (dd, J=1, 7Hz, 1H), 7.06 (d, J=1Hz, 1H), 6.99 (d, J=7Hz, 1H), 4.35 (d, J=13Hz, 1H), 4.32 (d, J=13Hz, 1H), 4.1-4.3 (m, 3H), 3.88 (s, 3H), 3.05-3.75 (m, 9H), 3.00 (m, 1H), 2.85 (dt, J=14, 4Hz, 1H), 2.72 (dd, J=7, 13Hz, 1H), 2.54 (dd, J=10, 14Hz, 1H), 2.26 (m, 1 H), 1.63 (m, 2H), 1.32 (d, J=7Hz, 6H), 0.94 (t, J=7Hz, 3H).

### s) (S)-4-{2-Hydroxy-5-[(3S,4S,5R)-4-hydroxy-5-(3-isopropyl-benzylamino)-1,1-dioxo-hexahydro-1lambda*6*-thiopyran-3-ylmethyl]-benzyl}-3-propyl-oxazolidin-2-one hydrochloride

To a solution of (S)-4-{5-[(3S,4S,5R)-4-hydroxy-5-(3-isopropyl-benzylamino)-1,1-dioxo-hexahydro-1lambda*6*-thiopyran-3-ylmethyl]-2-methoxy-benzyl}-3-propyl-oxazolidin-2-one hydrochloride (0.100 g, 0.18 mmol) in anhydrous CH₂Cl₂ (5 mL) is added under Argon at 0 °C a 1 M BBr₃-solution in CH₂Cl₂ (1.8 mL). After stirring at 0-5 °C for 4 h, the reaction mixture is poured onto ice-K₂CO₃-solution (5 mL) and stirred for 30 min. The product is extracted with CHCl₃. The combined organic layers are washed with water, dried over MgSO₄ and evaporated. The pure free base is obtained after purification by flash-chromatography on silica gel (hexane- CH₂Cl₂-MeOH-NH₃ 100:100:5:0.5 to 10:100:5:0.5) as a colorless foam. The hydrochloride salt is prepared with 1 N HCl in Et₂O in CH₂Cl₂. After evaporation of the solvents the title compound is obtained after crystallization from MeOH-ACN-Et₂O as white crystals: m.p. 171-174 °C; TLC (CH₂CL₂-MeOH 19:1) Rf=0.12; ESIMS [M+H]⁺=545; ¹H-NMR (400 MHz, CD₃OD): δ 7.44 (s, 1 H), 7.35-7.45 (m, 3H), 6.99 (s, 1 H), 6.96 (dd, J=1, 7Hz, 1 H), 6.81 (d, J=7Hz, 1 H), 4.2-4.4 (m, 5H), 2.8-3.7 (m, 11 H), 2.70 (dd, J=5, 14Hz, 1 H), 2.54 (dd, J=11, 14Hz, 1 H), 2.26 (m, 1H), 1.63 (m, 2H), 1.32 (d, J=7Hz, 6H), 0,94 (t, J=7Hz, 3H).

### Example 44: (3R,4S,5S)-3-(3-Isopropyl-benzylamino)-1,1-dioxo-5-[4-(pyrimidin-4-ylamino)-benzyl]-hexahydro-1lambda*6*-thiopyran-4-ol

### a) (3aR,7S,7aS)-3=(3-Isopropyl-benzyl)-7-(4-methoxy-benzyl)-5,5-dioxo-hexahydro-1-oxa-5lambda*6*-thia-3-aza-inden-2-one

To a solution of (3aR,7S,7aS)-7-(3-bromo-4-methoxy-benzyl)-3-(3-isopropyl-benzyl)-5,5-dioxo-hexahydro-1-oxa-5lambda*6*-thia-3-aza-inden-2-one (1.99 g, 3.8 mmol) in EtOH-THF 1:1 (25 mL) is added 10% Pd-C (0.01g ) as catalyst and the resulting reaction mixture is hydrogenated at 1 atm H₂ at 25°C for 2 h. The catalyst is filtered off and the filtrate is evaporated. The residue is dissolved in EtOAc and washed with NaH₂PO₄-solution and brine, dried over MgSO₄ and evaporated to give the product as a white solid: TLC (toluene/EtOAc 3:1) Rf=0.48; ESIMS [M+H+NH₃]⁺=461; ¹H-NMR (400 MHz, CDCl₃): δ 7.32 (t, J=7Hz, 1 H), 7.23 (d, J=7Hz, 1H), 7.16 (s, 1H), 7.13 (d, J=7Hz,1H), 7.08 (d, J=7Hz, 2H), 6.89 (d, J=7Hz, 2H), 4.58 (d, J=14Hz, 1 H), 4.35 (d, J=14Hz), 3.90 (dt, J=4, 12Hz, 1 H), 3.82 (s, 3H), 3.79 (dd, J=12, 14Hz, 1H), 2.6-3.2 (m, 8H), 1.25 (d, J=7Hz, 6H).

### b) (3aR,7S,7aS)-7-(4-Hydroxy-benzyl)-3-(3-isopropyl-benzyl)-5,5-dioxo-hexahydro-1-oxa-5lambda*6*-thia-3-aza-inden-2-one

To a solution of (3aR,7S,7aS)-3-(3-isopropyl-benzyl)-7-(4-methoxy-benzyl)-5,5-dioxo-hexahydro-1-oxa=5lambda*6*-thia-3-aza-inden-2-one(1.680wg,3.8mmol) in anhydrous CH₂Cl₂ (100 mL) is added under Argon.at 0 °C a solution of BBr₃ (1.9 mL, 19.2 mmol) in CH₂Cl₂ (5 mL). After stirring at 0 °C for 1 h the reaction mixture is poured onto ice-K₂CO₃-solution (25 mL) and stirred for 30 min. The product is extracted with CHCl₃. The combined organic layers are washed with water, dried over MgSO₄ and evaporated. The product is obtained after filtration and drying as a white solid: TLC (hexane-EtOAc 1: 1) Rf=0.24; ESIMS [M+H+NH₃]⁺=447; ¹H-NMR (400 MHz, CDCl₃): δ 7.1-7.4 (m, 4H), 7.04 (d, J=7Hz, 2H), 6.83 (d, J=7Hz, 2H), 4.56 (d, J=14Hz, 1H), 4.35 (d, J=14Hz), 3.89 (dt, J=4, 12Hz, 1H), 3.79 (t, J=12Hz, 1 H), 2.6-3.2 (m, 8H), 1.25 (d, J=7Hz, 6H).

### c) Trifluoro-methanesulfonic acid 4-[(3aR,7S,7aS)-3-(3-isopropyl-benzyl)-2,5,5-trioxo-octahydro-1-oxa-5lambda*6*-thia-3-aza-inden-7-ylmethyl]-phenyl ester

To a solution of (3aR,7S,7aS)-7-(4-hydroxy-benzyl)-3-(3-isopropyl-benzyl)-5,5-dioxo-hexahydro-1-oxa-5lambda*6*-thia-3-aza-inden-2-one (1.89 g, 4.4 mmol) in CH₂Cl₂ (80 mL) is added 2,6-lutidine (0.94 mL, 7.96 mmol) and trifluoromethanesulfonic acid anhydride (1.3 mL, 7.7 mmol) at 0-5 °C and the reaction mixture is stirred at 0-5 °C for 1 h. The reaction mixture is poured onto cold 0.5N HCl and is extracted with CH₂Cl₂. The combined organic layers are washed with water, dried over MgSO₄ and evaporated. The product is obtained after purification by flash-chromatography on silica gel (toluene-EtOAc 4:1) as a white solid: TLC (toluene-EtOAc 3:1) Rf=0.31; ESIMS [M+H+NH₃]⁺=579; ¹H-NMR (400 MHz, CDCl₃): δ 7.1-7.35 (m, 8H), 4.55 (d, J=14Hz, 1 H), 4.35 (d, J=14Hz, 1 Hz), 3.90 (dt, J=4, 12Hz, 1 H), 3.81 (t, J=12Hz, 1 H), 2.7-3.3 (m, 8H), 1.25 (d, J=7Hz, 6H).

### d) (3aR,7S,7aS)-3-(3-Isopropyl-benzyl)-5,5-dioxo-7-[4-(pyrimidin-4-ylamino)-benzyl]-hexahydro-1-oxa-5lambda*6*-thia-3-aza-inden-2-one

To a suspension of trifluoro-methanesulfonic acid 4-[(3aR,7S,7aS)-3-(3-isopropyl-benzyl)-2,5,5-trioxo-octahydro-1-oxa-5lambda*6*-thia-3-aza-inden-7-ylmethyl]-phenyl ester (0.10 g, 0.175 mmol), 4-aminiopyrimidine (0.034 g, 0.35 mmol), CS₂CO₃ (0.087 g, 0.263 mmol) and Xantphos (0.031, 0.053 mmol) in toluene-dioxane 1:1 ( 5 mL) is added under Argon Pd₂(dab)₃ (0.017 mg, 0.018 mmol) and the reaction mixture is heated at 100 °C for 4 h. The reaction mixture is added to a cold NaH₂PO₄-solution and extracted with EtOAc. The combined organic layers are washed with brine, dried over MgSO₄ and evaporated. The product is obtained after purification by flash-chromatography on silica gel (CH₂Cl₂MeOH 95:5) as a white foam: TLC (CH₂Cl₂-MeOH 10:1) Rf=0.42; ESIMS [M+H]⁺=507.

### e) (3R,4S,5S)-3-(3-Isopropyl-benzylamino)-1,1-dioxo-5-[4-(pyrimidin-4-ylamino)-benzyl]-hexahydro-1lambda*6*-thiopyran-4-ol

To a solution of (3aR,7S,7aS)-3-(3-isopropyl-benzyl)-5,5-dioxo-7-[4-(pyrimidin-4-ylamino)-benzyl]-hexahydro-1-oxa-5lambda*6*-thia-3-aza-inden-2-one (0.055 g, 0.1 mmol) in dioxane-water 1:1 (2 mL) is added Ba(OH)₂ 8H₂O (0.167 g, 0.515 mmol) and the resulting suspension was heated in a microwave oven at 160 °C for 10 min. After dilution with EtOAc (30 mL), the reaction mixture is filtered and the filtrate is washed with saturated NaHCO₃-solution and brine, dried over MgSO₄ and evaporated. The pure title compound is obtained after crystallization from ACN as white crystals: m.p. 136-139 °C. TLC (CH₂Cl₂-MeOH 10:1) Rf=0.37; ESIMS [M+H]⁺=481; ¹H-NMR (400 MHz, DMSO-d₆): δ 9.7 (s, 1H), 8.62 (s, 1H), 8.26 (br d, J=6Hz, 1 H), 7.62 (d, J=7Hz, 2H), 7.3-7.5 (m, 4H), 7.18 (d, J=7Hz, 2H), 6.81 (d, J=7Hz, 1H), 6.11 (br s, 1 H), 4.21 (m, 2H), 3.1-3.8 (m, 7H), 2.93 (m, 1 H), 2.74 (m, 1 H), 2.46 (dd, J=12, 14Hz,1H), 2.19 (m, 1H), 1.26 (d, J=7Hz, 6H).

**Examples 45 to 48:** All compounds of the formula I in Table 2 can be prepared using a procedure analogous to that used in Example 44.

**Table 2**

| | | **m/z** |
|---|---|---|
| **Example** | **Compound of the formula I** | **[M+H]⁺** |
| 45 | (3R,4S,5S)-3-(3-tert-Butyl-benzylamino)-1,1-dioxo-5-[4-(pyridin-2-ylamino)-benzyl]-hexahydro-1lambda*6*-thiopyran-4-ol | 494 |
| 46 | (3R,4S,5S)-3-(3-tert-Butyl-benzylamino)-1,1-dioxo-5-[4-(4-phenyl-pyridin-2-ylamino)benzyl]-hexahydro-1lambda*6*-thiopyran-4-ol | 570 |
| 47 | (3S,4S,5R)-3-{4-[6-(4-Fluoro-phenyl)-pyrimidin-4-ylamino]-benzyl}-5-(3-isopropyl-benzylamino)-1,1-dioxo-hexahydro-1lambda*6*-thiopyran-4-ol hydrochloride | 575 |
| 48 | (3R,4S,5S)-3-(3-tert-Butyl-benzylamino)-5-{4-[6-(4-fluoro-phenyl)-pyrimidin-4-ylamino]-benzyl}-1,1-dioxo-hexahydro-1lambda*6*-thiopyran-4-ol | 589 |

### Example 49: (3S,4S,5R)-3-[4-(6-Chloro-benzothiazol-2-ylamino)-benzyl]-5-(3-isopropyl-benzylamino)-1,1-dioxo-hexahydro-1lambda*6*-thiopyran-4-ol trifluoroacetate

The title compound is prepared using a procedure analogous to that used in Example 44, starting from trifluoro-methanesulfonic acid 4-[(3aR,7S,7aS)-3-(3-isopropyl-benzyl)-2,5,5-trioxo-octahydro-1-oxa-5lambda*6*-thia-3-aza-inden-7-ylmethyl]-phenyl ester and 6-chloro-benzothiazol-2-ylamine, as a white solid [HPLC: Rₜ = 1.81 min; ESIMS: [M + H]⁺ = 570, 572].

### Example 50: (3R,4S,5S)-3-(3-Isopropyl-benzylamino)-5-[4-(6-methoxy-benzothiazol-2-ylamino)-benzyl]-1,1-dioxo-hexahydro-1lambda*6*-thlopyran-4-ol trifluoroacetate

The title compound is prepared using a procedure analogous to that used in Example 44, starting from trifluoro-methanesulfonic acid 4-[(3aR,7S,7aS)-3-(3-isopropyl-benzyl)-2,5,5-trioxo-octahydro-1-oxa-5lambda*6*-thia-3-aza-inden-7-ylmethyl]-phenyl ester and 6-methoxy-benzothiazol-2-ylamine, as a white solid [HPLC: Rₜ = 1.68 min; ESIMS: [M + H]⁺ = 566].

### Example 51: (3R,4S,5S)-3-(3-tert-Butyl-benzylamino)-5-[4-(6-chloro-pyrimidin-4-ylamino)-benzyl]-1,1-dioxo-hexahydro-1lambda*6*-thiopyran-4-ol hydrochloride

### a) 3-(4-Bromo-benzyl)-4-oxo-3,4-dihydro-2H-thiopyran-3-carboxylic acid allyl ester

To a solution of 4-oxo-3,4-dihydro-2H-thiopyran-3-carboxylic acid allyl ester (3.5 g, 17.4 mmol) in methylethylketone (80 mL) are added 1-bromo-4-bromomethyl-benzene (5.8 g, 22.7 mmol) and K₂CO₃ (7.4 g, 52.4 mmol). The reaction mixture is stirred for 6 h at 25 °C and then filtered, and the filtrate is evaporated. The residual oil is dissolved in EtOAc, and the solution is washed with saturated NaHCO₃ solution and brine, dried over MgSO₄ and evaporated to yield the title compound after crystallization from Et₂O / hexane as yellow crystals [TLC (hexane / EtOAc 3:1): Rf = 0.35; ESIMS: [M + H]⁺ = 367, 369; ¹H-NMR (400 MHz, CDCl₃): δ = 7.40 (m, 3H), 7.19 (d, J = 7 Hz, 2H), 6.21 (d, J = 10 Hz, 1 H), 5.85 (m, 1 H), 5.28 (dd, J = 12, 16 Hz, 2H), 4.63 (m, 2H), 3.55 (d, J = 12 Hz, 1 H), 3.36 (d, J = 12 Hz, 1 H), 3.22 (d, J = 12 Hz, 1H), 3.03 (d, J = 12 Hz, 1H)].

### b) 3-(4-Bromo-benzyl)-2,3-dihydro-thiopyran-4-one

To a solution of 3-(4-bromo-benzyl)-4-oxo-3,4-dihydro-2H-thiopyran-3-carboxylic acid allyl ester (9.92 g, 27 mmol) in anhydrous THF (400mL) are added under argon dimedone (23.0 g, 162 mmol) and Pd(PPh₃)₄ (0.94 g, 0.81 mmol). The reaction mixture is stirred for 2 h at 25 °C and then evaporated. The product is obtained after purification by flash-chromatography on silica gel (toluene / EtOAc 6:1 to 4:1) and crystallization from hexane / Et₂O as a light yellow solid [TLC (toluene / EtOAc 3:1): Rf = 0.56; ESIMS: [M + H]⁺ = 283, 285; ¹H-NMR (400 MHz, CDCl₃): δ = 7.47 (m, 3H), 7.14 (d, J = 8 Hz, 1 H), 6.21 (d, J = 11 Hz, 1 H), 3.2 (m, 2H), 2.88 (m, 1H), 2.79 (m, 2H)].

### c) (3S*,4R*)-3-(4-Bromo-benzyl)-3,4-dihydro-2H-thiopyran-4-ol

To a solution of 3-(4-bromo-benzyl)-2,3-dihydro-thiopyran-4-one (6.9 g, 24 mmol) in THF / MeOH 1:1 (200 mL) are added CeCl₃ (11.95 g, 48 mmol) and, after stirring of the mixture for 0.5 h at 25 °C, in small portions over a period of 1.5 h NaBH₄ (1.83 g, 48 mmol). After stirring for 1 h, the reaction mixture is diluted with saturated NH₄Cl solution, and the organic solvents are removed under reduced pressure. The product is extracted from the aqueous phase with EtOAc. The combined extracts are washed with water and brine, dried over MgSO₄ and evaporated to yield the title compound as a beige foam [TLC (hexane / EtOAc 1:1): Rf = 0.52; ESIMS: [M - H₂O]⁺ = 267, 269; ¹H-NMR (400 MHz, CDCl₃): δ = 7.44 (d, J = 7 Hz, 2H), 7.15 (d, J = 7 Hz, 2H), 6.32 (d, J = 10 Hz, 1 H), 5.93 (dd, J = 10, 5 Hz, 1 H), 4.5 (m, 1 H), 4.03 (m, 1 H), 2.92 (dd, J = 12, 8 Hz, 1 H), 2.83 (d, J = 12 Hz, 1H), 2.69 (dd, J = 13, 8 Hz, 1 H), 2.54 (d, J = 12 Hz, 1H), 2.08 (m, 1H)].

### d) (35*,4R*)-3-(4-Bromo-benzyl)-1,1-dioxo-1,2,3,4-tetrahydro-1lambda*6*-thiopyran-4-ol

To a solution of (3S*,4R*)-3-(4-bromo-benzyl)-3,4-dihydro-2H-thiopyran-4-ol (10.4 g, 36.6 mmol) in THF (225 mL) are added water (225 mL) and, in portions at 25 - 30 °C, oxone (50.5 g, 80.5 mmol). The reaction mixture is stirred overnight at rt. After the addition of NaOAc (12.5 g, 146 mmol), the excess oxone is destroyed with Na₂S₂O₃ (10 g). The mixture is diluted with water and extracted with EtOAc. The combined extracts are washed with water and brine, dried over MgSO₄ and evaporated. The title compound is obtained in pure form after crystallization from Et₂O as a white solid [TLC (hexane / EtOAc 1:1): Rf = 0.15; ESIMS: [M + H + NH3]⁺ = 334, 346; ¹H-NMR (400 MHz, CDCl₃): δ = 7.51 (d, J = 7 Hz, 2H), 7.18 (d, J
= 7 Hz, 2H), 6.45 (s, 2H), 4.05 (m, 1H), 3.41 (dd, J = 13, 12 Hz,1H), 3.02 (dd, J = 12 Hz, 1H), 2.96 (m; 1H), 2.78 (m, 3H)].

### e) 1-tert-Butyl-3-isocyanatomethyl-benzene

To a solution of bis-(trichloromethyl)-carbonate (12.3 g, 41 mmol) in CH₂Cl₂ (300 mL) is added slowly under argon at 0°C a solution of 3-tert-butyl-benzylamine (6.69 g, 41 mmol) and DIPEA (17.9 mL, 103 mmol) in CH₂Cl₂ (150 mL). The reaction mixture is stirred for 1.5 h at 0 °C, then diluted with CH₂Cl₂ and washed with NaHCO₃ solution. The organic layer is dried over MgSO₄ and evaporated to yield the title compound as a yellow oil [ESIMS: [M + H + NH₃]⁺ = 207; ¹H-NMR (400 MHz, CDCl₃): δ = 7.4 - 7.1 (m, 4H), 4.52 (s, 2H), 1.34 (s, 9H)].

### f) (3aR*,7S*,7aR*)-7-(4-Bromo-benzyl)-3-(3-tert-butyl-benzyl)-5,5-dioxo-hexahydro-1-oxa-5lambda*6*-thia-3-aza-inden-2-one

To a solution of 1-tert-butyl-3-isocyanatomethyl-benzene (8.3 g, 44 mmol) and DBU (0.57 g, 3.7 mmol) in ACN (50 mL) is added under argon (3S*,4R*)-3-(4-bromo-benzyl)-1,1-dioxo-1,2,3,4-tetrahydro-1lambda*6*-thiopyran-4-ol (12.0 g, 37 mmol). The reaction mixture is stirred for 16 h at 60 °C. The precipitated colorless crystals are filtered off, washed with cold ACN and Et₂O and dried [TLC (hexane / EtOAc 1:1): Rf = 0.46; ESIMS: [M + H + NH₃]⁺ = 523, 525; ¹H-NMR (400 MHz, DMSO-d₆): δ = 7.55 (d, J = 7 Hz, 2H), 7.39 (s, 1H), 7.4 - 7.2 (m, 4H), 7.14 (d, J = 7 Hz, 1 H), 4.57 (m, 2H), 4.13 (m, 2H), 3.58 (m, 2H), 3.19 (t, J = 14 Hz, 1H), 3.02 (d, J = 14 Hz, 1H), 2.78 (m, 3H), 1.28 (s, 9H)].

### g) (3S*,4R*,5R*)-3-(4-Bromo-benzyl)-5-(3-tert-butyl-benzylamino)-1,1-dioxo-hexahydro-1lambda*6*-thiopyran-4-ol

To a suspension of (3aR*,7S*,7aR*)-7-(4-bromo-benzyl)-3-(3-tert-butyl-benzyl)-5,5-dioxo-hexahydro-1-oxa-5lambda*6*-thia-3-aza-inden-2-one (11.9 g, 23.4 mmol) in dioxane / water 5:2 (300 mL) is added Ba(OH)₂ x 8 H₂O (38.1 g, 117 mmol). The mixture is stirred under reflux for 36 h and then added to aqueous NaH₂PO₄ solution. The mixture is extracted with EtOAc / THF (3:1). The combined extracts are dried over MgSO₄ and evaporated to yield the title compound after crystallization from EtOAc / Et₂O as a colorless solid [TLC (EtOAc): Rf = 0.54; ESIMS: [M + H]⁺ = 480, 482; ¹H-NMR (400 MHz, DMSO-d₆): δ = 7.49 (d, J = 7 Hz, 2H), 7.28 (s, 1 H), 7.2 (m, 4H), 7.07 (d, J = 7 Hz, 1 H), 5.38 (s, 1 H), 3.74 (m, 3H), 3.18 (m, 1 H), 3.05 (m, 3H), 2.83 (m, 1 H), 2.75 (dd, J = 13, 8 Hz, 1 H), 2.67 (m, 1 H), 2.65 (dd, J = 13, 7 Hz, 1 H), 2.52 (s, 1 H), 1.21 (s, 9H)].

### h) [(3R*,4R*,5S*)-5-(4-Bromo-benzyl)-4-hydroxy-1,1-dioxo-hexahydro-1lambda*6*-thiopyran-3-yl]-(3-tert-butyl-benzyl)-carbamic acid tert-butyl ester

To a suspension of (3S*,4R*,5R*)-3-(4-bromo-benzyl)-5-(3-tert-butyl-benzylamino)-1,1-dioxo-hexahydro-1lambda*6*-thiopyran-4-ol (11.0 g, 22.8 mmol) and NEt₃ (3.6 mL, 25.1 mmol) in ACN / THF 2:1 (200 mL) is added (Boc)₂O (7.6 g, 34.2 mmol). The mixture is stirred for 24 h at at 55 °C. The solvents are evaporated, and the residue is purified by flash-chromatography on silica gel (hexane / EtOAc 5:1 to 3:1) to yield the product as a colorless solid [TLC (hexane / EtOAc 3:1): Rf = 0.24; ESIMS: [M + H - 55]⁺ = 524, 526; ¹H-NMR (400 MHz, CDCl₃): δ = 7.43 (d, J = 7 Hz, 2H), 7.35 (d, J = 7 Hz, 1H), 7.24 (m, 2H), 7.01 (m, 3H), 6.0 (s, 1 H), 4.40 (m, 2H), 4.16 (m, 1H), 3.70 (m, 1 H), 3.57 (m, 1 H), 3.3 (t, J = 13 Hz, 1 H), 2.77 (dd, J = 13, 8 Hz, 1H), 2.68 (m, 2H), 2.57 (dd, J = 13, 7 Hz, 1 H), 2.12 (m, 1 H), 1.53 (s, 9H), 1.33 (s, 9H)].

### i) [(3R*,55*)-5-(4-Bromo-benzyl)-1,1,4-trioxo-hexahydro-1lambda*6*-thiopyran-3-yl]-(3-tert-butyl-benzyl)-carbamic acid tert-butyl ester

To a solution of [(3R*,4R*,5S*)-5-(4-bromo-benzyl)-4-hydroxy-1,1-dioxo-hexahydro-1lambda*6*-thiopyran-3-yl]-(3-tert-butyl-benzyl)-carbamic acid tert-butyl ester (9.2 g, 15.6 mmol) in CH₂Cl₂ (300 mL) is added Dess Martin reagent (10.3 g, 23.5 mmol). The mixture is stirred for 16 h at 25 °C and then added to aqueous NaHCO₃ solution. The excess Dess Martin reagent is destroyed with Na₂S₂O₃. The organic phase is dried over MgSO₄ and evaporated. The residue is purified by flash-chromatography on silica gel (hexane / EtOAc 3: 1 ) to yield the title compound as a colorless amorphous solid [TLC (hexane / EtOAc 3: 1): Rf = 0.33; ESIMS: [M + H - 55]⁺ = 522, 524].

### j) [(3R*,4S*,5S*)-5-(4-Bromo-benzyl)-4-hydroxy-1,1-dioxo-hexahydro-1lambda*6*-thiopyran-3-yl]-(3-tert-butyl-benzyl)-carbamic acid tert-butyl ester

To a solution of Ca(BH₄)₂ · bis-THF complex (1.44 g, 6.6 mmol) in anhydrous THF (100 mL) is added at-50 °C under argon a solution of [(3R*,5S*)-5-(4-bromo-benzyl)-1,1,4-trioxo-hexahydro-1lambda*6*-thiopyran-3-yl]-(3-tert-butyl-benzyl)-carbamic acid tert-butyl ester (3.2 g, 5.5 mmol) in THF (50 mL). The reaction mixture is stirred for 0.5 h at -50 °C and then poured onto an aqueous NaH₂PO₄ solution. The mixture is extracted with EtOAc. The combined organic layers are washed with brine, dried over MgSO₄ and evaporated. The residue is purified by flash-chromatography on silica gel (hexane / EtOAc 3: 1) to yield the title compound as a colorless solid [TLC (hexane / EtOAc 1:1): Rf = 0.57; ESIMS: [M + H - 55]⁺ = 524, 526].

### k) [(3R,4S,5S)-5-(4-Azido-benzyl)-4-hydroxy-1,1-dioxo-hexahydro-1lambda*6*-thio-pyran-3-yl]-(3-tert-butyl-benzyl)-carbamic acid tert-butyl ester

To a solution of [(3R*,4S*,5S*)-5-(4-bromo-benzyl)-4-hydroxy-1,1-dioxo-hexahydro-1lambda*6*-thiopyran-3-yl]-(3-tert-butyl-benzyl)-carbamic acid tert-butyl ester (1.3 g, 2.2 mmol) in EtOH / H₂O 7:3 (45 mL) are added sodium ascorbate (0.044 g, 0.22 mmol), Cul (0.086 g, 0:44 mmol), (1S,2S)-N,N'-dimethyl-cyclohexane-1,2-diamine (0.096 g, 0.66 mmol) and sodium azide (0.44 g, 6.6 mmol). The reaction mixture is heated under reflux for 9 h and then added to cold brine. The mixture is extracted with EtOAc. The combined organic layers are dried (MgSO₄), filtered through a plug of silica gel and evaporated to yield the title compound as a light yellow solid [TLC (hexane / EtOAc 1:1): Rf = 0.54; ESIMS: [M + H - Boc]⁺ = 443].

### l) [(3R,4S,5S)-5-(4-Amino-benzyl)-4-hydroxy-1,1-dioxo-hexahydro-1lambda*6*thiopyran-3-yl]-(3-tert-butyl-benzyl)-carbamic acid tert-butyl ester

A solution of [(3R,4S,5S)-5-(4-azido-benzyl)-4-hydroxy-1,1-dioxo-hexahydro-1lambda*6*-thiopyran-3-yl]-(3-tert-butyl-benzyl)-carbamic acid tert-butyl ester (3.65 g, 6.7 mmol) in MeOH (100 mL) is hydrogenated (1 atm H₂) for 1 h at 25 °C over 10 % Pd / C (0.16 g). The catalyst is filtered off over Celite, and the filtrate is evaporated to yield the racemic title compound. The racemate is separated by preparative chromatography on a Chiralpak AD-H column with hexane / EtOH (1:1) to yield the optically pure (3S,4R,5R)- and (peak 2) (3R,4S,5S)-compounds as colorless solids [TLC (hexane / EtOAc 1:1): Rf = 0.19; ESIMS: [M + H - Boc]⁺ = 417; ¹H-NMR (400 MHz, CDCl₃): δ = 7:4 - 7.2 (m, 3H), 7.08 (d, J = 7 Hz, 1H), 6.87 (d, J = 7 Hz, 2H), 6.61 (d, J = 7 Hz, 2H), 4.65 (m, 1 H), 4.25 (m, 1 H), 3.8 - 2.2 (m, 10H), 1.49 (s, 9H), 1.32 (s, 9H)].

### m) (3R,4S,5S)-3-(3-tert-Butyl-benzylamino)-5-[4-(6-chloro-pyrimidin-4-ylamino)-benzyl]-1,1-dioxo-hexahydro-1lambda*6*-thiopyran-4-ol dihydrochloride

To a solution of [(3R,4S,5S)-5-(4-amino-benzyl)-4-hydroxy-1,1-dioxo-hexahydro-1lambda*6*thiopyran-3-yl]-(3-tert-butyl-benzyl)-carbamic acid tert-butyl ester (0.08 g, 0.15 mmol) and 4,6-dichloro-pyrimidine (0.035 g, 0.23 mmol) in iPrOH (2 mL) is added 1 N HCl in iPrOH (0.1 mL). The mixture is heated in a microwave oven for 1 h at 120 °C, then basified with aqueous NaHCO₃ solution and extracted with EtOAc / THF (3:1). The combined organic layers are washed with brine, dried over MgSO₄ and evaporated. The pure title compound is obtained, in free base form, after purification of the residue by flash-chromatography on silica gel (hexane / EtOAc 1:1 to EtOAc) as a light yellow solid. The free base compound is dissolved in THF and converted into the dihydrochloride salt with 1 N HCl in Et₂O (2 equivalents). After trituration with Et₂O, this salt is obtained as a beige solid [TLC (EtOAc): Rf = 0.30; ESIMS: [M + H]⁺ = 529, 531; ¹H-NMR (400 MHz, CD₃OD): δ = 8.45 (s, 1H), 7.64 (s, 1 H), 7.55 (d, J = 7 Hz, 2H), 7.43 (t, J = 7 Hz, 1 H), 7.35 (d, J = 7 Hz, 1 H), 7.24 (d, J = 7 Hz, 2H), 6.84 (s, 1 H), 4.37 (d, J = 14 Hz, 1 H), 4.32 (d, J = 14 Hz, 1 H), 3.8 - 3.4 (m, 4H), 3.33 (m, 1H), 3.14 (m, 1 H), 2.82 (m, 1 H), 2.53 (dd, J = 14, 12 Hz, 1 H), 2.25 (m, 1 H), 1.36 (s, 9H)].

**Examples 52 to 54:** All compounds of the formula I in Table 3 can be prepared using a procedure analogous to that used in Example 51.

**Table 3**

| **Example** | **Compound of the formula I** | **m/z [M + H]⁺** |
|---|---|---|
| | | **Retention time (HPLC)** |
| 52 | (3R,4S,5S)-3-(3-tert-Butyl-benzylamino)-1,1-dioxo-5-[4-(6-phenyl-pyrimidin-4-ylamino)-benzyl]-hexahydro-1lambda*6*-thiopyran-4-ol hydrochloride | 571 |
| | | 1.60 min |
| 53 | (3R,4S,5S)-3-(3-tert-Butyl-benzylamino)-5-[4-(2-isopropyl-6-methyl-pyrimidin-4-ylamino)-benzyl]-1,1-dioxo-hexahydro-1lambda*6*-thiopyran-4-ol hydrochloride | 551 |
| | | 1.63 min |
| 54 | (3R,4S,5S)-3-(3-tert-Butyl-benzylamino)-5-[4-(6-chloro-2-isopropyl-pyrimidin-4-ylamino)-benzyl]-1,1-dioxo-hexahydro-1lambda*6*-thiopyran-4-ol | 571,573 |
| | | 1.93 min |

### Example 55: (3R,4S,5S)-3-(3-tert-Butyl-benzylamino)-5-[4-(6-methyl-pyridin-2-ylamino)-benzyl]-1,1-dioxo-hexahydro-1lambda*6*-thiopyran-4-ol

To a solution of [(3R,4S,5S)-5-(4-amino-benzyl)-4-hydroxy-1,1-dioxo-hexahydro-1lambda*6*thiopyran-3-yl]-(3-tert-butyl-benzyl)-carbamic acid tert-butyl ester (0.08 g, 0.15 mmol) in dioxane (3 mL) is added under argon NaOtBu (0.023 g, 0.23 mmol). The reaction mixture is heated for 0.5 h at 100 °C and then cooled to rt, followed by the addition of (2'-dicyclohexylphosphanyl-biphenyl-2-yl)-dimethyl-amine (0.012 g, 0.03 mmol), 2-chloro-6-methyl-pyridine (0.04 g, 0.3 mmol) and Pd₂(dba)₃ (0.014 g, 0.015 mmol). The degassed mixture is stirred for 2 h at 100 °C and then added to cold Na₂CO₃ solution. The mixture is extracted with EtOAc, and the combined organic layers are washed with brine, dried over MgSO₄ and evaporated. The residue is purified by flash-chromatography on silica gel (hexane / EtOAc 10:1 to EtOAc) to yield the title compound as a light yellow foam [TLC (EtOAc): Rf = 0.40; HPLC: Rₜ = 1.37 min; ESIMS: [M + H]⁺ = 508; ¹H-NMR (600 MHz, DMSO-d₆): δ = 10.4 (s, 1H), 9.78 (s, 1 H), 9.0 (s, 1H), 7.92 (dd, J = 8, 7 Hz, 1H), 7.64 (s, 1H), 7.43 (m, 1H), 7.37 (m, 2H), 7.3 - 7.2 (m, 4H), 7.0 (d, J = 7 Hz, 1H), 6.9 (d, J = 7 Hz, 1H), 4.26 (m, 2H), 3.87 (dt, J = 14, 4 Hz, 1H), 3.68 (t, J = 10 Hz, 1 H), 3.60 (dd, J = 14, 13 Hz, 1 H), 3.30 (t, J = 14 Hz, 1 H), 3.24 (m, 2H), 2.76 (dt, J = 14, 4 Hz, 1 H), 2.62 (d, J = 7 Hz, 1 H), 2.49 (s, 3H), 2.46 (dd, J = 14, 3 Hz, 1 H), 2.09 (m, 1 H), 1.29 (s, 9H)].

**Examples 56 to 58:** All compounds of the formula I in Table 4 can be prepared using a procedure analogous to that used in Example 55.

**Table 4**

| **Example** | **Compound of the formula I** | **m/z [M + H]⁺** |
|---|---|---|
| | | **Retention time (HPLC)** |
| 56 | (3R,4S,5S)-3-(3-tert-Butyl-benzylamino)-5-{4-[4-(4-fluoro-phenyl)-pyridin-2-ylamino]-benzyl}- | 588 |
| | 1,1-dioxo-hexahydro-1lambda*6*-thiopyran-4-ol dihydrochloride | 1.71 min |
| 57 | (3S,4S,5R)-3-[4-(Biphenyl-3-ylamino)-benzyl]- | 569 |
| | 5-(3-tert-butyl-benzylamino)-1,1-dioxo-hexa-hydro-1lambda*6*-thiopyran4-ol dihydro- | 2.22 min |
| 58 | chloride (3R,4S,5S)-3-(3-tert-Butyl-benzylamino)-1,1-dioxo-5-[4-(5-phenyl-pyridazin-3-ylamino)-ben-zyl]-hexahydro-1lambda*6*-thiopyran-4-ol triflu-oroacetate | 571 |
| | | 1.72 min |

### Example 59: (3R,4S,5S)-3-(3-tert-Butyl-benzylamino)-5-{4-[5-(4-fluoro-phenyl)-isoxazol-3-ylamino]-benzyl}-1,1-dioxo-hexahydro-1lambda*6*-thiopyran-4-ol hydrochloride

### a) (3-tert-Butyl-benzyl)-((3R,4S,5S)-5-{4-[3-(4-fluoro-phenyl)-3-oxo-propionylamino]-benzyl}-4-hydroxy-1,1-dioxo-hexahydro-1lambda*6*-thiopyran-3-yl)-carbamic acid tert-butyl ester

To a solution-of [(3R,4S,5S)-5-(4-amino-benzyl)-4-hydroxy-1,1-dioxo-hexahydro-1lambda*6*thiopyran-3-yl]-(3-tert-butyl-benzyl)-carbamic acid tert-butyl ester (1.0 g, 1.9 mmol) in toluene / DMF 3:1 (20 mL) is added under argon 3-(4-fluoro-phenyl)-3-oxo-propionic acid methyl ester (0.39 g, 19 mmol). The reaction mixture is heated under reflux for 3 h, then cooled, diluted with aqueous NaH₂PO₄ solution and extracted with EtOAc. The combined organic layers are washed with brine, dried over MgSO₄ and evaporated. The residue is purified by flash-chromatography on silica gel (toluene / EtOAc 2: 1) to yield the title compound as a reddish foam [TLC (toluene / EtOAc 1:1): Rf = 0.41; ESIMS: [M + H + NH₃]⁺ = 698; HPLC: Rₜ = 2.38 min].

### b) (3-tert-Butyl-benzyl)-((3R,4S,5S)-5-{4-[(Z)-3-(4-fluoro-phenyl)-1-mercapto-3-oxo-propenylamino]-benzyl}-4-hydroxy-1,1-dioxo-hexahydro-1lambda*6*-thiopyran-3-yl)-carbamic acid tert-butyl ester

To a solution of (3-tert-butyl-benzyl)-((3R,4S,5S)-5-{4-[3-(4-fluoro-phenyl)-3-oxo-propionylamino]-benzyl}-4-hydroxy-1,1-dioxo-hexahydro-1lambda*6*-thiopyran-3-yl)-carbamic acid tert-butyl ester (1.02 g, 1.5 mmol) in CH₂Cl₂ (30 mL) is added Lawesson reagent (1.13 g, 2.7 mmol). The reaction mixture is stirred for 36 h at 25 °C, then diluted with aqueous NaH₂PO₄ solution and extracted with CH₂Cl₂. The combined organic layers are washed with brine, dried over MgSO₄ and evaporated. The residue is purified by flash-chromatography on silica gel (hexane / EtOAc 2:1) to yield the title compound as a yellow solid [TLC (hexane / EtOAc 1:1): Rf = 0.38; ESIMS: [M + H]⁺ = 697].

### c) (3-tert-Butyl-benzyl)-((3R,4S,5S)-5-{4-[(Z)-3-(4-fluoro-phenyl)-1-methylsulfanyl-3-oxo-propenylamino]-benzyl}-4-hydroxy-1,1-dioxo-hexahydro-1lambda*6*-thiopyran-3-yl)-carbamic acid tert-butyl ester

To a solution of (3-tert-butyl-benzyl)-((3R,4S,5S)-5-{4-[(Z)-3-(4-fluoro-phenyl)-1-mercapto-3-oxo-propenylamino]-benzyl}-4-hydroxy-1,1-dioxo-hexahydro-1lambda*6*-thiopyran-3-yl)-carbamic acid tert-butyl ester (0.55 g, 0.78mmol) in THF (7 mL) is added at 0 - 5 °C under argon NaH (0.031 g 60 % in oil, 0.78 mmol). After stirring for 15 min, a solution of methyl iodide (0.113 g, 0.78 mmol) in THF (1 mL) is added. After stirring for 1 h, the mixture is poured onto 10 % aqueous NH₄Cl solution. The mixture is extracted with EtOAc. The combined organic layers are washed with brine, dried over MgSO₄ and evaporated. The residue is purified by flash-chromatography on silica gel (hexane / EtOAc 2:1) to yield the title compound as a yellow solid [TLC (hexane / EtOAc 1:1): Rf = 0.44; ESIMS: [M + H]⁺ = 711; HPLC: Rₜ = 2.66 min].

### d) (3-tert-Butyl-benzyl)-((3R,4S,5S)-5-{4-[5-(4-fluoro-phenyl)-isoxazol-3-ylamino]-benzyl}-4-hydroxy-1,1-dioxo-hexahydro-1lambda*6*-thiopyran-3-yl)-carbamic acid tert-butyl ester

To a solution of (3-tert-butyl-benzyl)-((3R,4S,5S)-5-{4-[(Z)-3-(4-fluoro-phenyl)-1-methylsulfanyl-3-oxo-propenylamino]-benzyl}-4-hydroxy-1,1-dioxo-hexahydro-1lambda*6*-thiopyran-3-yl)-carbamic acid tert-butyl ester (0.078 g, 0.107 mmol) in EtOH (2 mL) are added hydroxylamine hydrochloride (0.015 g, 0.214 mmol) and Na₂CO₃ (0.025 g, 0.235 mmol). The reaction mixture is stirred for 2.5 h at 70 °C, diluted with aqueous NaH₂PO₄ solution and extracted with EtOAc. The combined organic layers are washed with brine, dried over MgSO₄ and evaporated. The residue is purified by flash-chromatography on silica gel (hexane / EtOAc 2:1) to yield the title compound as a yellow solid [TLC (hexane / EtOAc 1:1): Rf = 0.32; ESIMS: [M + H]⁺ = 678; HPLC: Rₜ = 2.59 min].

### e) (3R,4S,5S)-3-(3-tert-Butyl-benzylamino)-5-{4-[5-(4-fluoro-phenyl)-isoxazol-3-ylamino]-benzyl}-1,1-dioxo-hexahydro-1lambda*6*-thiopyran-4-ol hydrochloride

A solution of (3-tert-butyl-benzyl)-((3R,4S,5S)-5-{4-[5-(4-fluoro-phenyl)-isoxazol-3-ylamino]-benzyl}-4-hydroxy-1,1-dioxo-hexahydro-1lambda*6*-thiopyran-3-yl)-carbamic acid tert-butyl ester (0.016 g, 0.018 mmol) in 4N HCl in dioxane (0.5 mL) is stirred for 1 h at 25 °C. The reaction mixture is evaporated and dried to yield the title compound as a light yellow solid [ESIMS: [M + H]⁺ = 578; HPLC: Rₜ = 2.05 min].

### Example 60: (3R,4S,5S)-3-[1-(3-tert-Butyl-phenyl)-cyclopropylamino]-5-[4-(6-chloropyrimidin-4-ylamino)-benzyl]-1,1-dioxo-hexahydro-1lambda*6*-thiopyran-4-ol dihydrochloride

### a) 1-tert-Butyl-3-(1-isocyanato-cyclopropyl)-benzene

To a solution of bis-(trichloromethyl)-carbonate (4.05 g, 13.5 mmol) in CH₂Cl₂ (100 mL) is added over a period of 1 h at 0 - 5 °C under argon a solution of 1-(3-tert-butyl-phenyl)-cyclopropylamine (2.55 g, 13.5 mmol) and DIPEA (5.86 mL, 13.5 mmol) in CH₂Cl₂ (100 mL). After stirring for 1 h at 0 °C, the reaction mixture is washed with cold NaHCO₃ solution, dried over MgSO₄ and evaporated. The crude product, a dark yellow oil, is reacted further without purification [TLC (hexane / EtOAc 3:1); Rf = 0.70; ESIMS: [M + H + NH₃]⁺ = 233].

### b) (3aR*,7S*,7aR*)-7-(4-Bromo-benzyl)-3-[1-(3-tert-butyl-phenyl)-cyclopropyl]-5,5-dioxo-hexahydro-1-oxa-5lambda*6*-thia-3-aza-inden-2-one

To a suspension of 1-tert-butyt-3-(1-isocyanato-cyclopropyl)-benzene (2.62 g, 12.2 mmol) and (3S*,4R*)-3-(4-bromo-benzyl)-1,1-dioxo-1,2,3,4-tetrahydro-1lambda*6*-thiopyran-4-ol (3.17 g, 10 mmol) in ACN (20 mL) is added DBU (0.19 mL, 1.2 mmol). The mixture is stirred for 16 h at 50 °C. After cooling, the crystallized product is filtered off, washed with cold ACN / Et₂O (1: 1) and dried to yield the title compound as white crystals [TLC (hexane / EtOAc 1:1): Rf = 0.67; ESIMS: [M + H + NH₃]⁺ = 549, 551; ¹H-NMR (400 MHz, DMSO-d₆): δ = 7.56 (d, J = 7 Hz, 2H), 7.43 (s, 1 H), 7.3 - 7.2 (m, 5H), 4.38 (m, 1 H), 4.26 (m, 1 H), 3.3 - 2.6 (m, 7H), 1.43 (m, 2H), 1.24 (s, 9H), 1.05 (m, 2H)].

### c) (3S*,4R*,5R*)-3-(4-Bromo-benzyl)-5-[1-(3-tert-butyl-phenyl)-cyclopropylamino]-1,1-dioxo-hexahydro-1lambda*6*-thiopyran-4-ol

The title compound is prepared using a procedure analogous to that used in Example 51g, starting from (3aR*,7S*,7aR*)-7-(4-bromo-benzyl)-3-[1-(3-tert-butyl-phenyl)-cyclopropyl]-5,5-dioxo-hexahydro-1-oxa-5lambda*6*-thia-3-aza-inden-2-one and Ba(OH)₂ x 8 H₂O [TLC (hexane / EtOAc 1:1): Rf = 0.34; ESIMS: [M + H]⁺ = 506, 508; ¹H-NMR (400 MHz, CDCl₃): δ = 7.47 (d, J = 8 Hz, 2H), 7.26 (m, 3H), 7.07 (d, J = 8 Hz, 2H), 7.0 (d, J = 7 Hz, 1 H), 3.62 (s, 1H), 3.2 - 2.5 (m, 8H), 2.27 (m, 1 H), 1.96 (s, 1H), 1.33 (s, 9H), 0.95 (m, 4H)].

### d) (3S*,5R*)-3-(4-Bromo-benzyl)-5-[1-(3-tert-butyl-phenyl)-cyclopropylamino]-1,1-dioxotetrahydro-1lambda*6*-thiopyran-4-one

To a solution of oxalyl chloride (0.92 mL, 9.8 mmol) in CH₂Cl₂ is added at -78 °C under argon a solution of DMSO (1.08 mL, 14.9 mmol) in CH₂Cl₂ (5 mL). After stirring for 10 min at -78 °C, a solution of (3S*,4R*,5R*)-3-(4-bromo-benzyl)-5-[1-(3-tert-butyl-phenyl)-cyclopropylamino]-1,1-dioxo-hexahydro-1lambda*6*-thiopyran-4-ol (1.72 g, 2.7 mmol) in CH₂Cl₂ (10 mL) is added. After stirring for 45 min at -70 °C, 1-ethylpiperidine (3.8 mL, 27 mmol) is added at -70 °C. The reaction mixture is stirred for 30 min at -70 °C and for 1 h at -40 °C and then poured onto cold citric acid solution. The mixture is basified with NaOH and extracted with CH₂Cl₂. The combined organic layers are washed with 5 % K₂CO₃ solution and water, dried over MgSO₄ and evaporated. The resulting crude product is reacted further without purification [TLC (hexane / EtOAc 1:1): Rf = 0.62; ESIMS: [M + H]⁺ = 504, 506].

### e) (3S*,4S*,5R*)-3-(4-Bromo-benzyl)-5-[1-(3-tert-butyl-phenyl)-cyclopropylamino]-1,1-dioxo-hexahydro-1lam bda*6*-thiopyran-4-ol

To a solution of (3S*,5R*)-3-(4-bromo-benzyl)-5-[1-(3-tert-butyl-phenyl)-cyclopropylamino]-1,1-dioxo-tetrahydro-1lambda*6*-thiopyran-4-one (1.36 g, 2.65 mmol) in anhydrous THF is added at -60 °C under argon LiAlH₄ (0.033 g, 0.79 mmol). The reaction mixture is stirred for 0.5 h at -40 °C. After the addition of water (0.04 mL), 4N NaOH (0.05 mL) and water (0.1 mL), the mixture is stirred for 0.5 h and then filtered over Celite. The filtrate is evaporated. The residue is purified by flash-chromatography on silica gel (hexane / EtOAc 2:1 to 1:1) to yield as minor product the (3S*,4R*,5R*)-isomer [TLC (hexane / EtOAc 1:1): Rf = 0.34] and as major product the title compound as a yellow oil [TLC (hexane / EtOAc 1:1): Rf = 0.16; ESIMS: [M + H]⁺ = 506, 508; ¹H-NMR (600 MHz, DMSO-d₆ + TFA): δ = 7.71 (s, 1 H), 7.53 (d, J = 7 Hz, 1 H), 7.46 (d, J = 8 Hz, 2H), 7.43 (d, J = 8 Hz, 1 H), 7.39 (t, J = 8 Hz, 1 H), 7.11 (d, J = 8Hz, 2H), 3.61 (m, 1 H), 3.37 (t, J = 13 Hz, 1 H), 3.33 (t, J = 10 Hz, 1 H), 3.19 (t, J = 13 Hz, 1 H), 3.02 (m, 2H), 2.74 (m, 1 H), 2.51 (m, 1 H), 2.04 (m, 1H), 1.47 (m, 1 H), 1.32 (m, 2H), 1.28 (s, 9H), 1.01 (m, 1H)].

### f) (3S*,4S*,5R*)-3-(4-Azido-benzyl)-5-[1-(3-tert-butyl-phenyl)-cyclopropylamino]-1,1-dioxo-hexahydro-1lambda*6*-thiopyran-4-ol

To a solution of (3S*,4S*,5R*)-3-(4-bromo-benzyl)-5-[1-(3-tert-butyl-phenyl)-cyclopropylamino]-1,1-dioxo-hexahydro-1lambda*6*-thiopyran-4-ol (0.88 g, 1.7 mmol), sodium ascorbate (0.034 g, 0.17 mmol) and Cul (0.066 g, 0.34 mmol) in EtOH / H₂O 7:3 (40 mL) are added (1S,2S)-N,N'-dimethyl-cyclohexane-1,2-diamine (0.074 g, 0.51 mmol) and NaN₃ (0.339 g, 5.1 mmol). The reaction mixture is heated under reflux for 5 h under argon. After the addition of EtOAc, the organic layer is washed with water and brine, dried over MgSO₄ and evaporated.

The residue is purified by flash-chromatography on silica gel (hexane / EtOAc 3:1 to 1:2) to yield the title compound as a light yellow foam [TLC (hexane / EtOAc 1:1): Rf = 0.17; ESIMS: [M + H]⁺ = 469; ¹H-NMR (600 MHz, DMSO-d₆): δ = 7.42 (s, 1H), 7.22 (m, 2H), 7.19 (d, J = 8 Hz, 2H), 7.15 (d, J = 7 Hz, 1 H), 7.04 (d, J = 8 Hz, 2H), 5.13 (d, J = 6 Hz, 1H), 3.15 (s, 1 H), 3.08 (dd, J = 13, 3 Hz, 1 H), 3.05 - 2.95 (m, 2H), 2.94 (t, J = 13 Hz, 1H), 2.89 (t, J = 13 Hz, 1 H), 2.79 (m, 1 H), 2.53 (m, 1 H), 2.42 (dd, J = 13, 10Hz, 1 H), 1.96 (m, 1 H), 1.27 (s, 9H), 1.03 (m, 1H), 0.88 (m, 1H), 0.84 (m, 1H), 0.71 (m, 1H)].

### g) (3S,4S,5R)-3-(4-Amino-benzyl)-5-[1-(3-tert-butyl-phenyl)-cyclopropylamino]-1,1-dioxo-hexahydro-1lambda*6*-thiopyran-4-ol

A solution of (3S*,4S*,5R*)-3-(4-Azido-benzyl)-5-[1-(3-tert-butyl-phenyl)-cyclopropylamino]-1,1-dioxo-hexahydro-1lambda*6*-thiopyran-4-ol in MeOH (20 mL) is hydrogenated (1 atm H₂) at 25 °C over 10 % Pd / C (0.08 g) for 1 h. The catalyst is filtered off over Celite, and the filtrate is evaporated to yield the racemic title compound as a light yellow oil. This racemate is separated by preparative chromatography on a Chiralpak AD-H column with hexane / EtOH (1:1) to yield the optically pure (3R,4R,5S)-isomer and (as peak 2) the optically pure (3S,4S,5R)-isomer as a colorless foam [TLC (hexane / EtOAc 1:2): Rf = 0.20; ESIMS: [M + H]⁺ = 443; ¹H-NMR (400 MHz, CDCl₃): δ = 7.41 (s, 1 H), 7.31 (m, 2H), 7.20 (d, J = 7 Hz, 1H), 6.92 (d, J = 7 Hz, 2H), 6.61 (d, J = 7 Hz, 2H), 3.63 (s, 2H), 3.22 (m, 1H), 3.03 (dd, J = 13, 3 Hz, 1 H), 2.81 (m, 2H), 2.55 (m, 3H), 2.26 (m, 2H), 1.64 (s, 2H), 1.34 (s, 9H), 1.10 (m, 2H), 0.89 (m, 2H)].

### h) (3R,4S,5S)-3-[1-(3-tert-Butyl-phenyl)-cyclopropylamino]-5-[4-(6-chloro-pyrimidin-4-ylamino)-benzyl]-1,1-dioxo-hexahydro-1lambda*6*-thiopyran-4-ol dihydrochloride

To a solution of (3S,4S,5R)-3-(4-amino-benzyl)-5-[1-(3-tert-butyl-phenyl)-cyclopropylamino]-1,1-dioxo-hexahydro-1lambda*6*-thiopyran-4-ol (0.025 g, 0.055 mmol) and 4,6-dichloro-pyrimidine (0.025 g, 0.165 mmol) in iPrOH (2 mL) is added 0.5N HCl in iPrOH (0.1 mL). The reaction mixture is heated in a microwave oven to 120 °C for 1.4 h and then evaporated. The title compound in free base form is obtained after purification of the residue by flash-chromatography on silica gel (hexane / EtOAc / 2N NH₃ in EtOH 50:50:1 to 0:99:1) as a light yellow solid. This free base is dissolved in THF and converted into the dihydrochloride salt with 1N HCl in Et₂O (2 equivalents). After trituration with Et₂O, the dihydrochloride salt is obtained as a white solid [TLC (EtOAc): Rf = 0.52; HPLC: Rₜ = 1.83 min; ESIMS: [M + H]⁺ = 555, 557; ¹H-NMR (600 MHz, DMSO-d₆): δ = 10.04 (s, 1 H), 9.09 (s, 1 H), 9.32 (s, 1 H), 8.48 (s, 1 H), 7.76 (s, 1 H), 7.56 (d, J = 7 Hz, 1 H), 7.52 (d, J = 8 Hz, 2H), 7.42 (d, J = 8 Hz, 1 H), 7.38 (t, J = 8 Hz, 1 H), 7.13 (d, J = 7 Hz, 2H), 6.89 (s, 1H), 5.97 (s, 1 H), 3.59 (m, 1 H), 3.48 (m, 2H), 3.19 (t, J = 14 Hz, 1 H), 3.10 (dd, J = 13, 3 Hz, 1 H), 3.01 (t, J = 13 Hz, 1 H), 2.69 (dt, J = 14, 3 Hz, 1H), 2.45 (dd, J = 14, 10 Hz, 1H), 1.99 (m, 1H), 1.47 (m, 2H), 1.34 (m, 1H), 1.27 (s, 9H), 1.00 (m, 1H)].

### Examnle 61: (3R,4S,5S)-3-[1-(3-tert-Butyl-phenyl)-cyclopropylamino]-5-{4-[6-(4-fluorophenyl)-pyrimidin-4-ylamino]-benzyl}-1,1-dioxo-hexahydro-1lambda*6*-thiopyran-4-ol dihydrochloride

The title compound is prepared using a procedure analogous to that used in Example 60h, starting from (3S,4S,5R)-3-(4-amino-benzyl)-5-[1-(3-tert-butyl-phenyl)-cyclopropylamino]-1,1-dioxo-hexahydro-1lambda*6*-thiopyran-4-*ol and 4-chloro-6-(4-fluoro-phenyl)-pyrimidine, as a white solid [HPLC: Rₜ = 1.68 min; ESIMS: [M + H]⁺ = 615].

## Claims

1. A compound of the formula 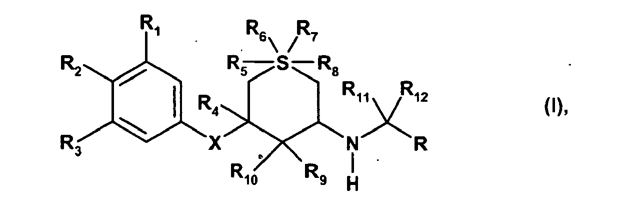 in which
R₁ is hydrogen; halogen; (C₁₋₈)alkyl; hydroxy-(C₁₋₈)alkyl; (C₁₋₈)alkoxy; (C₁₋₈)alkoxy-(C₁₋₈)alkyl; (C₁₋₈)alkoxy-(C₁₋₈)alkoxy-(C₁₋₈)alkyl; (C₆₋₁₀)aryl-(C₁₋₈)alkyl, the aryl moiety of which is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)-alkoxy, hydroxy-(C₁₋₈)alkyl and (C₁₋₈)alkoxy-(C₁₋₈)alkyl; a heteroaryl or heterocycloalkyl group, which group is unsubstituted or mono-, di- or tri-sutistituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; aminocarbonyl, the amino moiety of which is unsubstituted or mono- or di-substituted by substituents selected from the group consisting of (C₁₋₈)alkyl and benzyl, the phenyl moiety of which is unsubstituted or mono-, di-or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl; or (C₁₋₈)alkyl, which is mono-substituted by a pyrrolidinyl or oxazolidinyl group, which group is unsubstituted or mono-, di- or tri-substituted by substituents selected from the group consisting of oxo, (C₁₋₈)alkyl, halogen-(C₁₋₈)alkyl and (C₁₋₈)alkoxy-(C₁₋₈)alkyl;
R₂ is hydrogen; halogen; hydroxy; (C₁₋₈)alkyl; (C₁₋₈)alkoxy; (C₁₋₈)alkylamino; or an arylamino or heteroarylamino group, the aryl or heteroaryl moiety of which is unsubstituted or mono-, di- or tri-substituted by substituents selected from the group consisting of halogen, (C₁₋₈)alkyl, (C₁₋₈)alkoxy, (C₃₋₈)cycloalkyl, (C₃₋₈)cycloalkyl-(C₁₋₈)alkyl and a heteroaryl or aryl group, which heteroaryl or aryl group is unsubstituted or mono-, di- or tri-substituted by substituents selected from the group consisting of hydroxy, halogen, (C₁₋₈)alkyl, halogen-(C₁₋₈)alkyl, (C₁₋₈)alkoxy, halogen-(C₁₋₈)alkoxy, hydroxy-(C₁₋₈)alkyl and (C₁₋₈)alkoxy-(C₁₋₈)alkyl;
R₃ is hydrogen; halogen; (C₁₋₈)alkyl; or benzyl, the phenyl moiety of which is unsubstituted or mono-, di- or tri-substituted by substituents selected from the group consisting of (C₁₋₈)alkyl;
R₄ is hydrogen; or halogen;
R₅ and R₆ together are oxo; or both are absent;
R₇ and R₈ together are oxo; or both are absent;
either R₉ is hydroxy; and
R₁₀ is hydrogen; or hydroxy;
or R₉ and R₁₀ together are oxo;
either R₁₁ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl; and
R₁₂ is hydrogen; (C₁₋₈)alkyl; halogen-(C₁₋₈)alkyl; hydroxy-(C₁₋₈)alkyl; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
or R₁₁ and R₁₂ together are, together with the carbon atom, to which they are attached, (C₃₋₈)cycloalkyl, in which one -CH₂- group can be replaced with -O-;
X is O; or CH₂; and
either R is a group of the formula 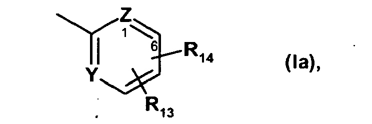 in which
R₁₃ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
R₁₄ is hydrogen; hydroxy; halogen; (C₁₋₈)alkyl; halogen-(C₁₋₈)alkyl; (C₁₋₈)alkoxy; halogen-(C₁₋₈)alkoxy; (C₁₋₈)alkoxy-(C₁₋₈)alkyl; (C₁₋₈)alkylcarbonyl; or (C₃₋₈)cycloalkyl, which is unsubstituted or mono-, di-, tri- or tetra-substituted by substituents selected from the group consisting of halogen and (C₁₋₈)alkyl;
Y is CH; or N; and
Z is CH; or N;
or R is a group of the formula 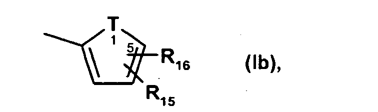 in which
R₁₅ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl;
R₁₆ is hydrogen; halogen; (C₁₋₈)alkyl; halogen-(C₁₋₈)alkyl; (C₁₋₈)alkoxy; (C₁₋₈)alkoxy-(C₁₋₈)alkyl; or (C₃₋₈)cycloalkyl, which is unsubstituted or mono-, di-, tri- or tetra-substituted by substituents selected from the group consisting of halogen and (C₁₋₈)alkyl; and
T is O; or S,
in free base form or in acid addition salt form.

2. A process for the preparation of a compound as defined in claim 1 of the formula I, in free base form or in acid addition salt form, comprising the steps of
a) for the preparation of a compound of the formula I, in which R₁₁ is hydrogen, reaction of a compound of the formula 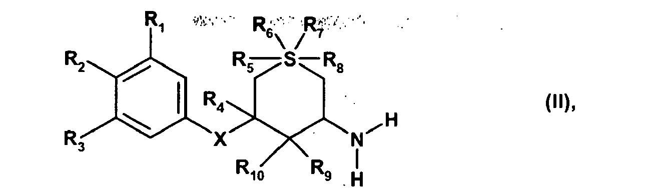 in which R₁, R₂, R₃, R₄, R₅, R₆, R₇, R₈, R₉, R₁₀ and X are as defined for the formula I, with a compound of the formula (O=)C(R₁₂) (III), in which R₁₂ and R are as defined for the formula I, and subsequent subjection of the reaction mixture to a hydrogenating agent, such as NaBH₃CN, or
b) for the preparation of a compound of the formula I, in which R₉ is hydroxy and R₁₀ is hydrogen, reaction of a compound of the formula 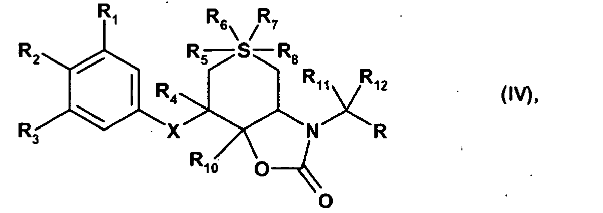 in which R₁, R₂, R₃, R₄, R₅, R₆, R₇, R₈, R₁₀, R₁₁, R₁₂, R and X are as defined for the formula I, with barium hydroxide or
c) for the preparation of a compound of the formula I, in which R₂ is a substituted amino group, conversion of a compound of the formula I, in which R₂ is an unsubstituted amino group, into a compound of the formula I, in which R₂ is a substituted amino group, for example by reaction with a corresponding halide,
in each case optionally followed by reduction, oxidation or functionalisation of the resulting compound and/or by cleavage of protecting groups optionally present, and of recovering the so obtainable compound of the formula I in free base form or in acid addition salt form.

3. A compound as defined in claim 1, in free base form or In pharmaceutically acceptable acid addition salt form, for use as a medicament.

4. A compound as defined in claim 1, in free base form or in pharmaceutically acceptable acid addition salt form, for use in the treatment of neurological or vascular disorders related to beta-amyloid generation and/or aggregation.

5. A pharmaceutical composition comprising a compound as defined in claim 1, in free base form or in pharmaceutically acceptable acid addition salt form, as active ingredient and a pharmaceutical carrier or diluent.

6. The use of a compound as defined in claim 1, in free base form or in pharmaceutical acceptable acid addition salt form, for the manufacture of a medicament for the treatment of neurological or vascular disorders related to beta-amyloid generation and/or aggregation.

7. A combination comprising a therapeutically effective amount of a compound as defined in claim 1, in free base form or in pharmaceutically acceptable acid addition salt form, and a second drug substance, for simultaneous or sequential administration.

## Patentansprüche

1. Verbindungen der Formel 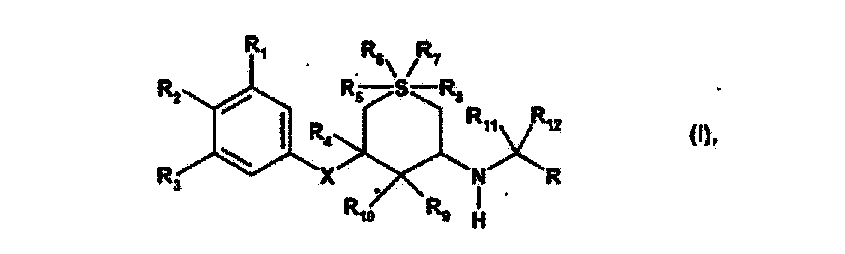 in welcher
R₁ für Wasserstoff, Halogen; (C₁₋₈)-Alkyl; Hydroxy-(C₁₋₈)-Alkyl; (C₁₋₈)-Alkoxy; (C₁₋₈) -Alkoxy-(C₁₋₈)-alkyl; (C₁₋₈)-Alkoxy- (C₁₋₈)-alkoxy- (C₁₋₈)-alkyl; (C₆₋₁₀)-Aryl- (C₁₋₈)-alkyl, dessen Aryleinheit unsubstituiert oder mono-, di- oder trisubstituiert durch Substituenten ausgewählt aus der Gruppe bestehend aus Hydroxy, Halogen, (C₁₋₈)-Alkyl, Halogen-(C₁₋₈)-alkyl; (C₁₋₈)-Alkoxy, Halogen-(C₁₋₈)-alkoxy, Hydroxy- (C₁₋₈)-alkyl und (C₁-₈)-Alkoxy-(C₁₋₈)-alkyl ist; eine Heteroaryl- oder Heterocycloalkylgruppe, wobei diese Gruppe unsubstituiert oder mono-, di-oder trisubstituiert durch Substituenten ausgewählt aus der Gruppe bestehend aus Hydroxy, Halogen, (C₁₋₈)-Alkyl, Halogen-(C₁₋₈)-alkyl, (C₁₋₈)-Alkoxy, Halogen-(C₁₋₈)-alkoxy, Hydroxy-(C₁₋₈)-alkyl und (C₁₋₆)-Alkoxy-(C₁₋₈)-alkyl ist; Aminocarbonyl, dessen Aminoeinheit unsubstituiert oder mono- oder disubstituiert durch Substituenten ausgewählt aus der Gruppe bestehend aus (C₁₋₈)-Alkyl und Benzoyl, dessen Phenyleinheit unsubstituiert oder mono-, di- oder trisubstituiert durch Substituenten ausgewählt aus der Gruppe bestehend aus Hydroxy, Halogen, (C₁₋₈-)Alkyl, Halogen-(C₁₋₈)-alkyl, (C₁₋₈)-Alkoxy, Halogen-(C₁₋₈)-alkoxy, Hydroxy-(C₁₋₈)-alkyl und (C₁₋₈)-Alkoxy-(C₁₋₈)-alkyl ist, ist; oder (C₁₋₈)-Alkyl, das monosubstituiert durch eine Pyrrolidinyl- oder Oxazolidinylgruppe ist, wobei diese Gruppe unsubstituiert oder mono-, di- oder
trisubstituiert durch Substituenten ausgewählt aus der Gruppe bestehend aus Oxo, (C₁₋₈)-Alkyl, Halogen-(C₁₋₈)-alkyl und (C₁₋₈)-Alkoxy-(C₁₋₈)-alkyl ist; steht;
R₂ für Wasserstoff, Halogen, Hydroxy; (C₁₋₈)-Alkyl; (C₁₋₈)-Alkoxy; (C₁₋₈)-Alkylamino; oder eine Arylamino- oder Heteroarylaminogruppe, deren Aryl- bzw. Heteroaryleinheit unsubstituiert oder mono-, di- oder trisubstituiert durch Substituenten ausgewählt aus der Gruppe bestehend aus Halogen, (C₁₋₈)-Alkyl, (C₁₋₈)-Alkoxy, (C₃₋₈)-Cycloalkyl, (C₃₋₈)-Cycloalkyl-(C₁₋₈)-alkyl und einer Heteroaryl-oder Arylgruppe, wobei die Heteroaryl- bzw.
Arylgruppe unsubstituiert oder mono-, di- oder trisubstituiert durch Substituenten ausgewählt aus der Gruppe bestehend aus Hydroxy, Halogen, (C₁₋₈)-Alkyl, Halogen-(C₁₋₈)-alkyl, (C₁₋₈)-Alkoxy, Halogen-(C₁₋₈)-alkoxy, Hydroxy-(C₁₋₈) -alkyl und (C₁₋₈)-Alkoxy-(C₁₋₈)-alkyl ist, ist; steht;
R₃ für Wasserstoff; Halogen; (C₁₋₈)-Alkyl; oder Benzyl, dessen Phenyleinheit unsubstituiert oder mono-, di- oder trisubstituiert durch Substituenten ausgewählt aus der Gruppe bestehend aus (C₁₋₈)-Alkyl ist; steht;
R₄ für Wasserstoff; oder Halogen; steht;
R₅ und R₆ zusammen für Oxo stehen; oder beide abwesend sind;
R₇ und R₈ zusammen für Oxo stehen; oder beide abwesend sind.;
entweder R₉ für Hydroxy steht; und
R₁₀ für Wasserstoff; oder Hydroxy; steht;
oder R₉ und R₁₀ zusammen für Oxo stehen;
entweder R₁₁ für Wasserstoff; (C₁₋₈)-Alkyl; Halogen-(C₁₋₈)-alkyl; Hydroxy-(C₁₋₈)-alkyl; (C₁₋₈)-Alkoxy-(C₁-₈)-alkyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist; steht; und
R₁₂ für Wasserstoff; (C₁₋₈)-Alkyl; Halogen-(C₁₋₈)-alkyl; Hydroxy-(C₁₋₈)-alkyl; (C₁₋₈)-Alkoxy-(C₁₋₈)-alkyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist; steht;
oder R₁₁ und R₁₂ zusammen, zusammen mit dem Kohlenstoffatom, an das sie gebunden sind, für (C₃₋₈)-Cycloalkyl, in welchem eine -CH₂-Gruppe durch -O- ersetzt sein kann, stehen;
X für O; oder CH₂; steht; und entweder R für eine Gruppe der Formel 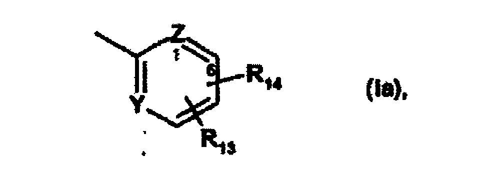 steht, in welcher
R₁₃ für Wasserstoff; Halogen; (C₁₋₈)-Alkyl; Halogen-(C₁₋₈)-alkyl; (C₁₋₈)-Alkoxy; (C₁₋₈)-Alkoxy- (C₁₋₈)-alkyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist, steht;
R₁₄ für Wasserstoff; Hydroxy; Halogen; (C₁₋₈)-Alkyl; Halogen-(C₁₋₈)-alkyl; (C₁₋₈)-Alkoxy; Halogen-(C₁₋₈)-alkoxy; (C₁₋₈)-Alkoxy- (C₁₋₈) -alkyl; (C₁₋₈)-Alkylcarbonyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist, steht;
Y für CH; oder N steht; und
Z für CH; oder N steht;
oder R für eine Gruppe der Formel 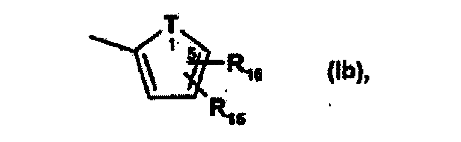 steht, in welcher
R₁₅ für Wasserstoff; Halogen; (C₁₋₈)-Alkyl; Halogen-(C₁₋₈)-alkyl; (C₁₋₈)-Alkoxy; (C₁₋₈)-Alkoxy-(C₁₋₈)-alkyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist, steht;
R₁₆ für Wasserstoff; Halogen; (C₁₋₈)-Alkyl; Halogen-(C₁₋₈)-alkyl; (C₁₋₈)-Alkoxy; (C₁₋₈)-Alkoxy-(C₁₋₈)-alkyl; oder (C₃₋₈)-Cycloalkyl, welches unsubstituiert oder mono-, di- oder tri- oder tetrasubstituiert durch Substituenten ausgewählt aus der Gruppe bestehend aus Halogen und (C₁₋₈)-Alkyl ist, steht; und
T für O; oder S steht,
in Form der freien Base oder in Form eines Säureadditionssalzes.

2. Verfahren zur Herstellung einer Verbindung der Formel I nach Anspruch 1 in Form der freien Base oder in Form eines Säureadditionssalzes, bei dem man
a) zur Herstellung einer Verbindung der Formel I in welcher R₁₁ für Wasserstoff steht, eine Verbindung der Formel 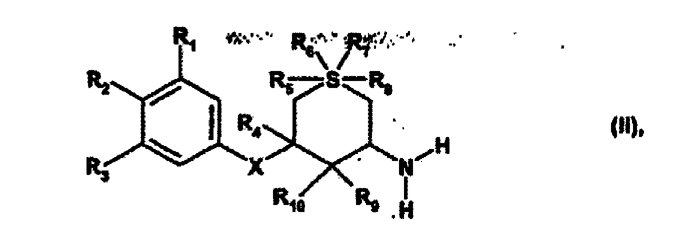 in welcher R₁, R₂, R₃, R₄, R₅, R₆, R₇, R₈, R₉, R₁₀ und X wie für die Formel I definiert sind, mit einer Verbindung der Formel (O=)C(R₁₂)R (III), in welcher R₁₂ und R wie für die Formel I definiert sind, umsetzt, und die Reaktionsmischung anschließend einem Hydriermittel wie NaBH₃CN aussetzt, oder#
b) zur Herstellung einer Verbindung der Formel I, in welcher R₉ für Hydroxy steht und R₁₀ für Wasserstoff steht, eine Verbindung der Formel 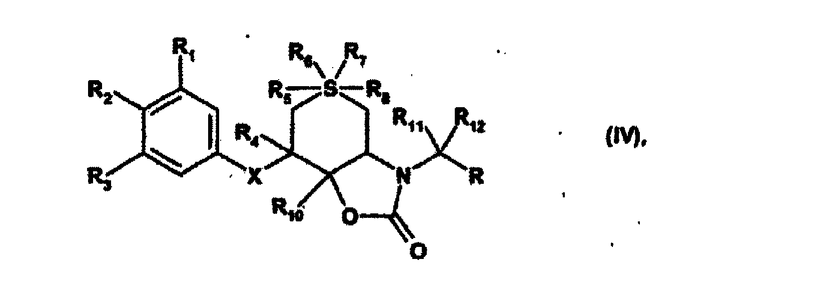 in welcher R₁, R₂, R₃, R₄, R₅, R₆, R₇, R₈, R₁₀, R₁₁, R₁₂, R und X wie für die Formel I definiert sind, mit Bariumhydroxid umsetzt oder
c) zur Herstellung einer Verbindung der Formel I, in welcher R₂ für eine substituierte Aminogruppe steht, eine Verbindung der Formel I, in welcher R₂ für eine unsubstituierte Aminogruppe steht, zum Beispiel durch Umsetzung mit einem entsprechenden Halogenid in eine Verbindung der Formel I, in welcher R₂ für eine substituierte Aminogruppe steht, umwandelt,
wobei sich jeweils gegebenenfalls eine Reduktion, Oxidation oder Funktionalisierung der erhaltenen Verbindung und/oder eine Abspaltung von gegebenenfalls vorhandenen Schutzgruppen und die Isolierung der auf diese Weise erhaltenen Verbindung der Formel I in Form der freien Base oder in Form eines Säureadditionssalzes anschließt.

3. Verbindungen nach Anspruch 1 in Form der freien Base oder in Form eines pharmazeutisch unbedenklichen Säureadditionssalzes zur Verwendung als Medikament.

4. Verbindungen nach Anspruch 1 in Form der freien Base oder in Form eines pharmazeutisch unbedenklichen Säureadditionssalzes zur Verwendung bei der Behandlung von neurologische oder vaskulären Erkrankungen, die mit der beta-Amyloid-Bildung und/oder -Aggregation in Zusammenhang stehen.

5. pharmazeutische Zusammensetzung, enthaltend eine Verbindung nach Anspruch 1 in Form der freien Base oder in Form eines pharmazeutisch unbedenklichen Säureadditionssalzes als Wirkstoff und einen pharmazeutischen Träger oder ein pharmazeutisches Verdünnungsmittel.

6. Verwendung einer Verbindung nach Anspruch 1 in Form der freien Base oder in Form eines pharmazeutisch unbedenklichen Säureadditionssalzes zur Herstellung eines Medikaments zur Behandlung von neurologischen oder vaskulären Erkrankungen, die mit der beta-Amyloid-Bildung und/oder -Aggregation in Zusammenhang stehen.

7. Kombination, enthaltend eine therapeutisch wirksame Menge einer Verbindung nach Anspruch 1 in Form der freien Base oder in Form eines pharmazeutisch unbedenklichen Säureadditionssalzes und eine zweite Arzneimittelsubstanz, zur gleichzeitigen oder aufeinander folgenden Verabreichung.

## Revendications

1. Composé de formule 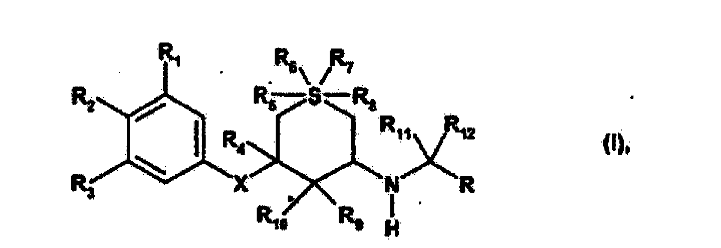 dans laquelle
R₁ est hydrogène ; halogène ; (C₁₋₈)alkyle ;
hydroxy-(C₁₋₈)alkyle ; (C₁₋₈)alcoxy ; (C₁₋₈)alcoxy-(C₁₋₈)alkyle ; (C₁₋₈)alcoxy-(C₁₋₈)alcoxy-(C₁₋₈)alkyle ; (C₆₋₁₀)aryl-(C₁₋₈)alkyle, dont le motif aryle est non substitué ou mono-, di- ou tri-substitué par des substituants choisis dans le groupe constitué d'hydroxy, halogène, (C₁-₈)alkyle, halogène-(C₁₋₈)alkyle, (C₁₋₈)alcoxy, halogène-(C₁₋₈)alcoxy, hydroxy-(C₁₋₈)alkyle et (C₁₋₈)alcoxy-(C₁₋₈)alkyle ; un groupement hétéroaryle ou hétérocycloalkyle, lequel groupement est non substitué ou mono-, di- ou tri-substitué par des substituants choisis dans le groupe constitué d'hydroxy, halogène, (C₁₋₈)alkyle, halogène-(C₁₋₈)alkyle, (C₁₋₈)alcoxy, halogène-(C₁₋₈)alcoxy, hydroxy-(C₁₋₈)alkyle et (C₁₋₈)alcoxy-(C₁₋₈)alkyle ; aminocarbonyle, dont le motif amino est non substitué ou mono- ou di-substitué
par des substituants choisis dans le groupe constitué de (C₁₋₈)alkyle et benzyl, dont le motif phényle est non substitué ou mono-, di- ou tri-substitué par des substituants choisis dans le groupe constitué d'hydroxy, halogène, (C₁₋₈)alkyle, halogène-(C₁₋₈)alkyle, (C₁₋₈)alcoxy, halogène-(C₁₋₈)alcoxy, hydroxy-(C₁₋₈)alkyle et (C₁₋₈)alcoxy-(C₁₋₈)alkyle ; ou (C₁₋₈)alkyle, qui est mono-substitué par un groupement pyrrolidinyle ou oxazolidinyle, lequel groupement est non substitué ou mono-, di- ou tri-substitué par des substituants choisis dans le groupe constitué d'oxo, (C₁₋₈)alkyle, halogène-(C₁₋₈)alkyle et (C₁₋₈)alcoxy-(C₁₋₈)alkyle ;
R₂ est hydrogène ; halogène ; hydroxy ; (C₁₋₈)alkyle ; (C₁₋₈)alcoxy ; (C₁₋₈)alkylamino ; ou un groupement arylamino ou hétéroarylamino, dont le motif aryle ou hétéroaryle est non substitué ou mono-, di- ou tri-substitué par des substituants choisis dans le groupe constitué d'halogène, (C₁₋₈)alkyle, (C₁₋₈)alcoxy, (C₃₋₈)cycloalkyle, (C₃₋₈)cycloalkyl-(C₁₋₈)alkyle et un groupement hétéroaryle ou aryle, lequel groupement hétéroaryle ou aryle est non substitué ou mono-, di- ou tri-substitué par des substituants choisis dans le groupe constitué d'hydroxy, halogène, (C₁₋₈)alkyle, halogène- (C₁₋₈)alkyle, (C₁₋₈)alcoxy, halogène-(C₁₋₈)alcoxy, hydroxy-(C₁₋₈)alkyle et (C₁₋₈)alcoxy-(C₁₋₈)alkyle ;
R₃ est hydrogène ; halogène ; (C₁₋₈)alkyle ; ou benzyle, dont le motif phényle est non substitué ou mono-, di- ou tri-substitué par des substituants choisis dans le groupe constitué de (C₁₋₈)alkyle ;
R₄ est hydrogène ; ou halogène ;
R₅ et R₆ ensemble sont oxo ; ou sont tous deux absents ;
R₇ et R₈ ensemble sont oxo ; ou sont tous deux absents ;
soit R₉ est hydroxy ; et
R₁₀ est hydrogène ; ou hydroxy ;
soit R₉ et R₁₀ ensemble sont oxo ;
soit R₁₁ est hydrogène ; (C₁₋₈)alkyle ; halogène-(C₁₋₈)alkyle ; hydroxy-(C₁₋₈)alkyle ; (C₁₋₈)alcoxy-(C₁₋₈)alkyle ; ou (C₃₋₈)cycloalkyle, qui est non substitué ou mono-, di-,tri- ou tétra-substitué par des substituants choisis dans le groupe constitué d'halogène et (C₁₋₈)alkyle ; et
R₁₂ est hydrogène ; (C₁₋₈)alkyle ; halogène-(C₁₋₈)alkyle ; hydroxy-(C₁₋₈)alkyle ; (C₁₋₈)alcoxy-(C₁₋₈)alkyle ; ou (C₃₋₈)cycloalkyle, qui est non substitué ou mono-, di-, tri- ou tétra-substitué par des substituants choisis dans le groupe constitué d'halogène ou (C₁₋₈)alkyle
soit R₁₁ et R₁₂ ensemble sont, ensemble avec l'atome de carbone auquel ils sont liés, (C₃₋₈)cycloalkyle, dans lequel un groupement -CH₂- peut être remplacé par -O- ;
X est O ; ou CH₂ ; et
soit R est un groupement de formule 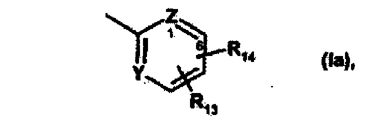 dans laquelle
R₁₃ est hydrogène ; halogène ; (C₁₋₈)alkyle ;
halogène-(C₁₋₈)alkyle ; (C₁₋₈)alcoxy; (C₁₋₈)alcoxy-(C₁₋₈)alkyle ; ou (C₃₋₈)cycloalkyle, qui est non substitué ou mono-, di-, tri- ou tétra-substitué par des substituants choisis dans le groupe constitué d'halogène et de (C₁₋₈)alkyle ;
R₁₄ est hydrogène ; hydroxy ; halogène ; (C₁₋₈)alkyle ; halogène-(C₁₋₈)alkyle ; (C₁₋₈)alcoxy ; halogène- (C₁₋₈)alcoxy (C₁₋₈)alcoxy- (C₁₋₈) alkyle ; (C₁₋₈)alkylcarbonyle ; ou (C₃₋₈)cycloalkyle, qui est non substitué ou mono-, di-, tri- ou tétra-substitué par des substituants choisis dans le groupe constitué d'halogène et (C₁₋₈)alkyle;
Y est CH ; ou N ; et
Z est CH ; ou N ;
soit R est un groupement de formule 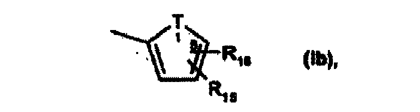 dans laquelle
R₁₅ est hydrogène ; halogène ; (C₁₋₈)alkyle ;
halogène-(C₁₋₈)alkyle ; (C₁₋₈)alcoxy; (C₁₋₈)alcoxy-(C₁₋₈)alkyle ; ou (C₃₋₈)cycloalkyle, qui est non substitué ou mono-, di-, tri- ou tétra-substitué par des substituants choisis dans le groupe constitué d' halogène et de (C₁₋₈)alkyle ;
R₁₆ est hydrogène ; halogène ; (C₁₋₈)alkyle ;
halogène-(C₁₋₈)alkyle ; (C₁₋₈)alcoxy; (C₁₋₈)alcoxy-(C₁₋₈)alkyle ; ou (C₃₋₈)cycloalkyle, qui est non substitué ou mono-, di-, tri- ou tétra-substitué par des substituants choisis dans le groupe constitué d'halogène et de (C₁₋₈)alkyle ; et
T est O ; ou S,
sous forme de base libre ou sous forme de sel d'addition d'acide.

2. Procédé de préparation d'un composé tel que défini dans la revendication 1 de formule I, sous forme de base libre ou sous forme de sel d'addition d'acide, comprenant les étapes consistant à
a) pour la préparation d'un composé de formule I, dans laquelle R₁₁ est hydrogène, la réaction d'un composé de formule 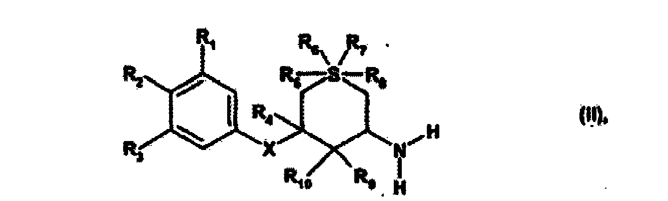 dans laquelle R₁, R₂, R₃, R₄, R₅, R₆, R₇, R₈, R₉, R₁₀ et X sont tels que définis pour la formule I avec un composé de formule (O=)C(R₁₂)R (III), dans laquelle R₁₂ et R sont tels que définis pour la formule I, puis on soumet le mélange réactionnel à un agent d'hydrogénation, tel que le NaBH₃CN, ou
b) pour la préparation d'un composé de formule I dans laquelle R₉ est hydroxy et R₁₀ est hydrogène, la réaction d'un composé de formule 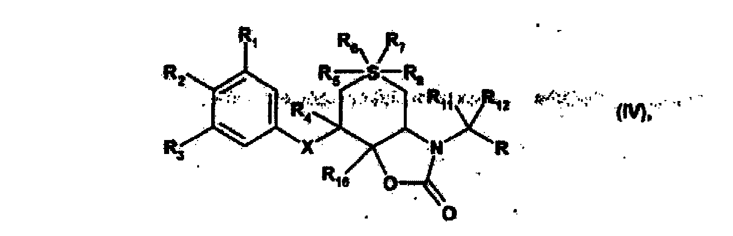 dans laquelle R₁, R₂, R₃, R₄, R₅, R₆, R₇, R₈, R₁₀, R₁₁, R₁₂, R et X sont tels que définis pour la formule I, avec de l'hydroxyde de barium ou
c) pour la préparation d'un composé de formule I dans laquelle R₂ est un groupement amino substitué, la transformation d'un composé de formule I, dans laquelle R₂ est un groupement amino non substitué, en un composé de formule I, dans laquelle R₂ est un groupement amino substitué, par exemple par réaction avec un halogénure correspondant,
dans chaque cas, éventuellement suivie de la réduction, de l'oxydation ou de la fonctionnalisation du composé résultant et/ou du clivage de groupements protecteurs éventuellement présents, et de la récupération du composé susceptible d'être ainsi obtenu de formule I sous forme de base libre ou sous forme de sel.

3. composé tel que défini dans la revendication 1, sous forme de base libre ou sous forme de sel d'addition d'acide pharmaceutiquement acceptable, destiné à être utilisé comme médicament.

4. Composé tel que défini dans la revendication 1, sous forme de base libre ou sous forme de sel d'addition d'acide pharmaceutiquement acceptable, destiné à être utilisé dans le traitement de troubles neurologiques ou vasculaires liés à la génération et/ou à l'agrégation de la bêta-amyloïde.

5. Composition pharmaceutique comprenant un composé tel que défini dans la revendication 1, sous forme de base libre ou sous forme de sel d'addition d'acide pharmaceutiquement acceptable, en tant que principe actif, et un support ou un diluant pharmaceutique.

6. Utilisation d'un composé tel que défini dans la revendication 1, sous forme de base libre ou sous forme de sel d'addition d'acide pharmaceutiquement acceptable, pour la fabrication d'un médicament destiné au traitement de troubles neurologiques ou vasculaires liés à la génération et/ou à l'agrégation de la bêta-amyloïde.

7. Combinaison, comprenant une quantité thérapeutiquement efficace d'un composé tel que défini dans la revendication 1, sous forme de base libre ou sous forme de sel d'addition d'acide pharmaceutiquement acceptable, et une deuxième substance médicamenteuse, destinée à une administration simultanée ou séquentielle.
